# EUROPEAN PATENT SPECIFICATION

(11) **EP 4 110 396 B1**
(45) Date of publication and mention of the grant of the patent: **28.10.2026**
(21) Application number: 21709500.9
(22) Date of filing: 19.02.2021
(51) Int. Cl.: A61K 45/06, A61P 9/10, A61K 31/195, A61K 31/223, A61K 31/437, A61K 31/496, A61K 31/4965

(54) **THERAPEUTIC COMBINATION FOR THE TREATMENT OF BRAIN ISCHEMIA AND SAID THERAPEUTIC COMBINATION FOR USE IN THE TREATMENT OF BRAIN ISCHEMIA**
THERAPEUTISCHE KOMBINATION FÜR DIE BEHANDLUNG VON GEHIRNISCHÄMIE UND DIESE THERAPEUTISCHE KOMBINATION ZUR VERWENDUNG BEI DER BEHANDLUNG VON GEHIRNISCHÄMIE
COMBINAISON THÉRAPEUTIQUE POUR LE TRAITEMENT DE L'ISCHÉMIE CÉRÉBRALE ET LADITE COMBINAISON THÉRAPEUTIQUE DESTINÉE À ÊTRE UTILISÉE DANS LE TRAITEMENT DE L'ISCHÉMIE CÉRÉBRALE

(30) Priority: 26.02.2020 NL 2025001
(43) Date of publication of application: 04.01.2023
(73) Proprietor: Universiteit Maastricht, 6211 LK Maastricht (NL); Academisch ziekenhuis Maastricht, 6229 HX Maastricht (NL)
(72) Inventor: SCHMIDT, Harald Horst Heinz Wilhelm, 52070 Aachen (DE); CASAS GUIJARRO, Ana Isabel, 6224 JG Maastricht (NL)
(74) Representative: Nederlandsch Octrooibureau
(86) International application number: PCT/NL2021/050118
(87) International publication number: WO 2021/172982

(56) References cited:
- WO-A1-99/12539
- A DASGUPTA ET AL: "Soluble Guanylate Cyclase: A New Therapeutic Target for Pulmonary Arterial Hypertension and Chronic Thromboembolic Pulmonary Hypertension", CLINICAL PHARMACOLOGY AND THERAPEUTICS, vol. 97, no. 1, 28 November 2014 (2014-11-28), US, pages 88 - 102, XP055297653, ISSN: 0009-9236, DOI: 10.1002/cpt.10
- GEORGE F. LASKER ET AL: "The sGC activator BAY 60-2770 has potent erectile activity in the rat", AMERICAN JOURNAL OF PHYSIOLOGY HEART AND CIRCULATORY PHYSIOLOGY, vol. 304, no. 12, 15 June 2013 (2013-06-15), US, pages H1670 - H1679, XP055752489, ISSN: 0363-6135, DOI: 10.1152/ajpheart.00062.2013
- YULIYA SHARKOVSKA ET AL: "Nitric oxide-independent stimulation of soluble guanylate cyclase reduces organ damage in experimental low-renin and high-renin models :", JOURNAL OF HYPERTENSION., vol. 28, no. 8, 1 August 2010 (2010-08-01), GB, pages 1666 - 1675, XP055752356, ISSN: 0263-6352, DOI: 10.1097/HJH.0b013e32833b558c
- EZEAMUZIE C I ET AL: "Reactive oxygen species mediate phorbol ester-stimulated cAMP response in human eosinophils", EUROPEAN JOURNAL OF PHARMACOLOGY, ELSEVIER SCIENCE, NL, vol. 543, no. 1-3, 14 August 2006 (2006-08-14), pages 174 - 180, XP028029044, ISSN: 0014-2999, [retrieved on 20060814], DOI: 10.1016/J.EJPHAR.2006.05.035
- YONEYAMA M ET AL: "Endogenous reactive oxygen species are essential for proliferation of neural stem/progenitor cells", NEUROCHEMISTRY INTERNATIONAL, ELSEVIER, AMSTERDAM, NL, vol. 56, no. 6-7, 1 May 2010 (2010-05-01), pages 740 - 746, XP027020043, ISSN: 0197-0186, [retrieved on 20091201]
- MATTHIAS OELZE ET AL: "Nebivolol Inhibits Superoxide Formation by NADPH Oxidase and Endothelial Dysfunction in Angiotensin II-Treated Rats", HYPERTENSION, vol. 48, no. 4, 1 October 2006 (2006-10-01), US, pages 677 - 684, XP055752064, ISSN: 0194-911X, DOI: 10.1161/01.HYP.0000239207.82326.29
- JACEK ZIELONKA ET AL: "High-throughput Assays for Superoxide and Hydrogen Peroxide", JOURNAL OF BIOLOGICAL CHEMISTRY, vol. 289, no. 23, 24 April 2014 (2014-04-24), pages 16176 - 16189, XP055189462, ISSN: 0021-9258, DOI: 10.1074/jbc.M114.548693
- G J MCBEAN ET AL: "Redox-based therapeutics in neurodegenerative disease : Redox therapeutics for neurodegeneration", BRITISH JOURNAL OF PHARMACOLOGY, vol. 174, no. 12, 1 June 2017 (2017-06-01), UK, pages 1750 - 1770, XP055751352, ISSN: 0007-1188, DOI: 10.1111/bph.13551
- SEREN M ET AL: "Collaborative Therapy with Nebivalol and l-NAME for Spinal Cord Ischemia/Reperfusion Injury", ANNALS OF VASCULAR SURGERY, ELSEVIER, AMSTERDAM, NL, vol. 22, no. 3, 1 May 2008 (2008-05-01), pages 425 - 431, XP022640951, ISSN: 0890-5096, [retrieved on 20080506], DOI: 10.1016/J.AVSG.2007.12.024
- J.-P. STASCH ET AL: "Soluble Guanylate Cyclase as an Emerging Therapeutic Target in Cardiopulmonary Disease", CIRCULATION, vol. 123, no. 20, 23 May 2011 (2011-05-23), US, pages 2263 - 2273, XP055440845, ISSN: 0009-7322, DOI: 10.1161/CIRCULATIONAHA.110.981738
- CAMILA S. ESTANCIAL ET AL: "Pharmacological characterisation of the relaxation induced by the soluble guanylate cyclase activator, BAY 60-2770 in rabbit corpus cavernosum", BJU INTERNATIONAL, vol. 116, no. 4, 1 October 2015 (2015-10-01), GB, pages 657 - 664, XP055752353, ISSN: 1464-4096, DOI: 10.1111/bju.13105
- RITCHIE REBECCA H ET AL: "The opposing roles of NO and oxidative stress in cardiovascular disease", PHARMACOLOGICAL RESEARCH, ACADEMIC PRESS, LONDON, GB, vol. 116, 15 December 2016 (2016-12-15), pages 57 - 69, XP029915609, ISSN: 1043-6618, DOI: 10.1016/J.PHRS.2016.12.017
- MELANIE E ARMITAGE ET AL: "Translating the oxidative stress hypothesis into the clinic: NOX versus NOS", JOURNAL OF MOLECULAR MEDICINE, SPRINGER, BERLIN, DE, vol. 87, no. 11, 16 October 2009 (2009-10-16), pages 1071 - 1076, XP019750580, ISSN: 1432-1440, DOI: 10.1007/S00109-009-0544-2
- WILLMOT M ET AL: "Nitric oxide synthase inhibitors in experimental ischemic stroke and their effects on infarct size and cerebral blood flow: A systematic review", FREE RADICAL BIOLOGY & MEDICINE, ELSEVIER INC, US, vol. 39, no. 3, 1 August 2005 (2005-08-01), pages 412 - 425, XP027830015, ISSN: 0891-5849, [retrieved on 20050801]
- SEBASTIAN ALTENHÖFER ET AL: "Evolution of NADPH Oxidase Inhibitors: Selectivity and Mechanisms for Target Engagement", ANTIOXIDANTS AND REDOX SIGNALING, vol. 23, no. 5, 10 August 2015 (2015-08-10), US, pages 406 - 427, XP055464131, ISSN: 1523-0864, DOI: 10.1089/ars.2013.5814
- ZHANG YIXUAN ET AL: "NADPH oxidases and oxidase crosstalk in cardiovascular diseases: novel therapeutic targets", NATURE REVIEWS CARDIOLOGY, NATURE PUBLISHING GROUP, GB, vol. 17, no. 3, 7 October 2019 (2019-10-07), pages 170 - 194, XP037024683, ISSN: 1759-5002, [retrieved on 20191007], DOI: 10.1038/S41569-019-0260-8

## Description

### TECHNICAL FIELD

The present invention relates to a therapeutic combination comprising two or three of at least one NADPH oxidase inhibitor, at least one nitric oxide synthase inhibitor and at least one soluble guanylate cyclase agonist. More specifically, the invention relates to said therapeutic combination for use in the prevention or treatment of brain ischemia or for use in the prevention or treatment of ischemia-reperfusion injury. An aspect of the invention relates to a kit comprising: the therapeutic combination according to the invention and optionally instructions for use. The invention also relates to a pharmaceutical composition comprising two or three of at least one NADPH oxidase inhibitor, at least one nitric oxide synthase inhibitor and at least one soluble guanylate cyclase agonist, and to said pharmaceutical composition for use in the prevention or treatment of brain ischemia or for use in the prevention or treatment of ischemia-reperfusion injury.

### BACKGROUND

When a blood clot obstructs a cerebral vessel, it will partially or completely obstruct the blood flow downstream of the affected site. Collateral vessels can often continue to supply blood to the afflicted area but their capacity is limited. Unless the clot is very small and/or disintegrates spontaneously (making the event a transient ischemic attack) the result is an ischemic stroke.

Traditionally, the medical community has viewed stroke as an unpredictable and untreatable event after which only supportive treatment and rehabilitation is possible. While the introduction of thrombolysis by tissue plasminogen activator (alteplase) in the late 1990s and widespread use of thrombectomy (mechanical recanalization) have put an end to this therapeutic nihilism, substantially reducing mortality in acute ischemic stroke, the condition remains in the top ranks of those with unmet medical need.

Both methods can restore downstream reperfusion but neither is universally applicable. Alteplase has a narrow time window for its administration (approx. 4.5 hours after symptom onset), it comes with a large number of caveats and exclusion criteria that are mostly related to bleeding, and being a recombinantly produced protein it is very expensive (the one-time treatment dose costs several thousand dollars). Thrombectomy, an intervention which requires a high degree of experience to perform, is limited to proximal large artery occlusions in the anterior circulation in patients who can be treated within 24 hours of the time last known to be well. Less than 20 percent of patients with acute ischemic stroke meet these criteria.

Neither reperfusion method addresses the reperfusion injury that will invariably occur upon restoration of blood supply. This oxidative process, driven by reactive oxygen species (ROS), often accounts for the majority of the overall stroke damage by causing additional lipid peroxidation and damage to nucleic acids¹. The combination of primary ischemic tissue damage (caused by failure of cellular energy resources, compromization of membrane phospholipids, and onset of the apoptotic cascade) followed by the formation of free radicals created during abrupt reoxygenation is a pervasive detrimental feature of interventional post-ischemic recanalization that is observed not only when ischemia is successfully resolved in the brain but also after myocardial infarction, acute kidney injury, and retinal ischemia². However, it is especially deleterious in the brain, where neuroinflammation triggered by glial cells and hemorrhagic transformation resulting from the damage of the vascular bed (especially after thrombolysis) constitute further clinical complications. Finally, glucose becomes toxic in ischemic stroke³, depriving the ischemic brain tissue of the only energy source that the brain can utilize.

Several clinical trials have failed to show suitability of antioxidants to prevent reperfusion injury in ischemic stroke. The reasons are unclear; the antioxidants might not have reached the ischemic site of the brain in sufficient concentrations; and on the other hand indiscriminately administering high doses of antioxidants has to be avoided since ROS also perform essential physiological functions, e.g. in cellular signaling⁴.

Decades of translational stroke research have yielded just one registered drug, a thrombolytic, and no neuroprotective therapy. Two systematic errors contributed to this. First, mainly single drug targets are pursued The "one disease-one target" approach overlooks complex disease patho-mechanisms and possible underlying comorbidities, while focusing on a symptom-rather than a mechanism-based therapy; second, preclinical stroke models ignore real-world comorbidities such as diabetes. Diabetic patients are, however, excluded from thrombolysis because of a high risk of fatal hemorrhagic transformation leaving them without any therapeutic option. Every year, 15 million people are diagnosed with stroke worldwide, of which 5 million die and another 5 million remain permanently disabled (source: The Internet Stroke Center). Although classical studies revealed several potential targets for stroke therapy, all translational attempts failed, leaving 85% of stroke patients without access to any pharmacotherapy.

Thus, together with non-precise clinical trial design, the drug discovery field has recently become considerably ineffective where indeed, stroke therapy remains a dramatic case of translational failure. There exists an urgent and unserved medical need for pharmacological interventions that prevent and/or treat brain ischemia and prevent and/or treat ischemia-reperfusion injury in the brain and in other organs.

NADPH oxidase inhibitors, nitric oxide synthase inhibitors and soluble guanylate cyclase agonists are known and their three individual mechanisms of action are previously described as being possibly beneficial in treatment of stroke; see WO99/12539; Stasch et al. 2011, Circulation, vol. 123, nr. 20, pages 2263-73; Ritchie R et al. 2016, Pharm. Res. vol. 116, pages 57-69; and Altenhöfer S et al. 2015, Antiox. Redox. Sign., vol. 23, nr. 5, pages 406-427.

### SUMMARY OF THE INVENTION

To overcome this roadblock in the art, the inventors designed a network-based approach focused on the interactome, a comprehensive map including all biologically relevant molecular interactions. The inventors introduce *in-silico* multi-target discovery based on genes associated to ischemic stroke, diabetes and their comorbidities. Proteins associated with specific diseases are not randomly spread but tend to interact forming connected subgraphs, the so-called disease modules. Within the diseasome, genes related to reactive oxygen species (ROS) and cyclic GMP (cGMP), amongst others, are located. Genetic evidence pointed cGMP as the underlying mechanism of a validated disease cluster including highly prevalent diseases, i.e. diabetes and stroke. Moreover, several ROS sources have been identified as critical players of stroke patho-mechanism and its comorbidities. Some relate to reactive oxygen species (ROS) interfering with cyclic GMP (cGMP) as possible patho-mechanism. Only one of these, however, contained both ROS (NOX4-5) and cGMP (NOS1-3, GUCYA1, GUCYB1) related genes. The inventors extended these findings to all known ROS and cGMP related clinical drug targets and conducted an interactome-based first neighbor analysis. They identified a disease module for target prediction in stroke therapy by linking six mechanistically related stroke targets within a causal network approach.

Indeed, the inventors' approach as explained in example 5 used a local seed-protein based method extended to all known ROS-cGMP related clinical drug targets followed by a first neighbor protein network analysis, thus identifying the first stroke-based disease module. PPI networks have been broadly used to understand complex disease mechanisms, but they still remain as a small representation of all molecular interaction networks. Thus, the inventors conducted a protein-metabolite network in conjunction with PPI networks, as previously described for NOX4 (11). The inventors' approach could be therefore translated to a wide range of complex diseases for further *de novo* identification of mechanism-related patho-phenotypes leading to target identification and future therapeutic options.

Also in example 5, the inventors importantly validated their therapeutic prediction both *in vivo* and *in vitro,* including a translational human blood-brain barrier and a stroke-diabetes comorbidity models, by a co-treatment of NOX and NOS inhibitors in combination with an sGC activator, while surprisingly identifying the cause of hemorrhagic transformation, the most detrimental diabetic-dependent event in stroke patients. In a WT mouse (devoid of human NOX5) model of ischemic stroke with diabetes as comorbidity multi-target network pharmacology inhibiting NOS and NOX and activating GUCY was neuroprotective in a highly synergistic manner: infarct size was decreased, blood-brain barrier stabilized, neuro-motor function improved and survival increased. Moreover, in a knock-in mouse expressing also NOX5, the inventors, surprisingly observed diabetes-associated post-stroke hemorrhagic transformation, which was entirely prevented by network pharmacology. Thus, a multi-target ROS-cGMP module both explains post-stroke neurodegeneration and diabetic hemorrhagic transformation, effectively targeted by highly synergistic network pharmacology. Importantly, maximal therapeutic efficacy was achieved while combining subthreshold concentrations or doses ineffective on their own. Hence, the inventors conclude that translational implementation of this therapeutic approach will ensure (i) maximal reduction of potential side effects, (ii) subsequent mechanism-based ultra-synergistic therapy, and ultimately (iii) a significant reduction of the number needed to treat.

All of the above has been explained in example 5 and associated Figures.

The current inventors now surprisingly established that it is now possible due to their invention to inhibit only those enzymatic sources of ROS that have been identified to be clinically most relevant in ischemic stroke, and based on their invention the inventors established that it has now become possible to prevent or even reverse the damage induced by oxidative stress in this setting. It was found by the inventors that NADPH oxidase type 4 (NOX4) plays a key role by being activated via NADPH to form hydrogen peroxide inducing blood-brain-barrier disruption and augmenting infarct size. Mice deficient in NOX4, but not those deficient for NOX1 or NOX2, were partially protected from oxidative stress, blood-brain-barrier leakage, and neuronal apoptosis, after both transient and permanent cerebral ischemia⁵. The inventors also confirmed that the deleterious effects of nitric oxide synthase (NOS), a signaling enzyme which however in stroke produces neurotoxic quantities of NO, can be partially prevented by NOS inhibitors (NOSi)⁶.

The inventors have been able to show that, surprisingly, combinations of some or all of (a) a NOX inhibitor (NOXi), preferably a NOX4-selective inhibitor; (b) a NOS inhibitor (NOSi); and (c) an agonist of soluble guanylate cyclase (sGC), all of which can be administered at concentrations that are minimally effective when administered individually, protect against neuronal damage in animal models of ischemic stroke better than any of the individual compounds. Even more surprisingly, they have shown that such a combination also allows for administration of glucose while avoiding its typical and long-known paradoxical toxicity in post-ischemic conditions. The inventors show that cells can be subjected to ischemic conditions, thereafter provided with glucose, whereas cell viability improves when some or all of (a) a NOX inhibitor (NOXi), preferably a NOX4-selective inhibitor; (b) a NOS inhibitor (NOSi); and (c) an agonist of soluble guanylate cyclase (sGC) is/are added to the cells after the ischemic conditions, compared to the cell viability obtained with the cells subjected first to ischemic conditions and then provided with glucose, in the absence of the NOXi, NOSi and/or sGC agonist (sGCa).

An aspect of the invention relates to a therapeutic combination for use as a medicament, comprising:
- a first therapeutic composition comprising at least one of: at least one NADPH oxidase inhibitor, at least one nitric oxide synthase inhibitor and at least one soluble guanylate cyclase agonist;
- a second therapeutic composition comprising at least one of: at least one NADPH oxidase inhibitor, at least one nitric oxide synthase inhibitor and at least one soluble guanylate cyclase agonist, wherein the second therapeutic composition is different from the first therapeutic composition; and optionally
- a third therapeutic composition comprising at least one of: at least one NADPH oxidase inhibitor, at least one nitric oxide synthase inhibitor and at least one soluble guanylate cyclase agonist, wherein the third therapeutic composition is different from the first therapeutic composition and is different from the second therapeutic composition;
wherein optionally the first therapeutic composition, the second therapeutic composition and/or the third therapeutic composition, when present, further comprise(s) a pharmaceutically acceptable diluent and/or a pharmaceutically acceptable excipient and, wherein, the NADPH oxidase inhibitor is selected from setanaxib, GKT136901, GKT137831, GLX7013114, VAS2870, perphenazine, fluphenazine, perazine and thioridazine, and wherein the nitric oxide synthase inhibitor is selected from NG-nitro-L-arginine methyl ester, L-NAME, NG-monomethyl-L-arginine, 2-iminobiotin, ronopterin, S-methyl-1-thiocitrulline and propylthiouracil.

Preferred is the therapeutic combination comprising:
a first therapeutic composition comprising at least one of: at least one NADPH oxidase inhibitor and at least one nitric oxide synthase inhibitor;
a second therapeutic composition comprising at least one soluble guanylate cyclase agonist; and optionally
a third therapeutic composition comprising at least one of: at least one NADPH oxidase inhibitor and at least one nitric oxide synthase inhibitor, wherein the third therapeutic composition is different from the first therapeutic composition;

wherein optionally the first therapeutic composition, the second therapeutic composition and/or the third therapeutic composition, when present, further comprise(s) a pharmaceutically acceptable diluent and/or a pharmaceutically acceptable excipient,
wherein, the NADPH oxidase inhibitor is selected from any one or more of NADPH oxidase inhibitors setanaxib, GKT136901, GKT137831, GLX7013114, VAS2870, perphenazine, fluphenazine, perazine and thioridazine, and
wherein the nitric oxide synthase inhibitor is selected from any one or more of nitric oxide synthase inhibitors NG-nitro-L-arginine methyl ester, L-NAME, NG-monomethyl-L-arginine, 2-iminobiotin, ronopterin, S-methyl-1-thiocitrulline and propylthiouracil.

An embodiment is the therapeutic combination of the invention, wherein
a. the first therapeutic composition is provided as a first unit dose comprising:
   i. a first NADPH oxidase inhibitor;
   ii. optionally a second NADPH oxidase inhibitor;
b. the second therapeutic composition is provided as a second unit dose comprising:
   i. a first nitric oxide synthase inhibitor;
   ii. optionally a second nitric oxide synthase inhibitor; and, when present,
c. the third therapeutic composition is provided as a third unit dose comprising:
   i. a first soluble guanylate cyclase agonist; and
   ii. optionally a second soluble guanylate cyclase agonist; or wherein

a. the first therapeutic composition is provided as a first unit dose comprising:
   i. a first soluble guanylate cyclase agonist;
   ii. optionally a second soluble guanylate cyclase agonist;
b. the second therapeutic composition is provided as a second unit dose comprising:
   i. a first nitric oxide synthase inhibitor;
   ii. optionally a second nitric oxide synthase inhibitor; and, when present,
c. the third therapeutic composition is provided as a third unit dose comprising:
   i. a first NADPH oxidase inhibitor; and
   ii. optionally a second NADPH oxidase inhibitor; or wherein

a. the first therapeutic composition is provided as a first unit dose comprising:
   i. a first soluble guanylate cyclase agonist;
   ii. optionally a second soluble guanylate cyclase agonist;
b. the second therapeutic composition is provided as a second unit dose comprising:
   i. a first NADPH oxidase inhibitor;
   ii. optionally a second NADPH oxidase inhibitor; and, when present,
c. the third therapeutic composition is provided as a third unit dose comprising:
   i. a first nitric oxide synthase inhibitor; and
   ii. optionally a second nitric oxide synthase inhibitor;
   wherein preferably the therapeutic combination comprises or consists of the first, second and third therapeutic compositions.

Typically, the therapeutic combination of the invention is a therapeutic combination, wherein
a. the first therapeutic composition is provided as a first unit dose comprising:
   i. a first NADPH oxidase inhibitor;
   ii. optionally a second NADPH oxidase inhibitor;
b. the second therapeutic composition is provided as a second unit dose comprising:
   i. a first soluble guanylate cyclase agonist; and
   ii. optionally a second soluble guanylate cyclase agonist
      ; and, when present,
c. the third therapeutic composition is provided as a third unit dose comprising:
   i. a first nitric oxide synthase inhibitor;
   ii. optionally a second nitric oxide synthase inhibitor
      ; or wherein

a. the first therapeutic composition is provided as a first unit dose comprising:
   i. a first nitric oxide synthase inhibitor;
   ii. optionally a second nitric oxide synthase inhibitor
b. the second therapeutic composition is provided as a second unit dose comprising:
   i. a first soluble guanylate cyclase agonist;
   ii. optionally a second soluble guanylate cyclase agonist;
      ; and, when present,
c. the third therapeutic composition is provided as a third unit dose comprising:
   i. a first NADPH oxidase inhibitor; and
   ii. optionally a second NADPH oxidase inhibitor;
wherein preferably the therapeutic combination comprises or consists of the first, second and third therapeutic compositions.

An aspect of the invention relates to a therapeutic combination according to the invention for use in the prevention or treatment of any one or more of brain ischemia, cerebral infarction, ischemic stroke and ischemia-reperfusion injury.

An aspect of the invention relates to a kit comprising: the therapeutic combination according to the invention or the therapeutic combination for use according to the invention; and optionally instructions for use, preferably the therapeutic combination according to the invention.

An embodiment is the kit according to the invention, comprising:
the first therapeutic composition provided as one or more unit doses for oral administration, each unit dose comprising 0.1 mg to 1000 mg, preferably 1 mg to 500 mg, most preferably 5 mg to 250 mg of the first therapeutic composition;
the second therapeutic composition provided as one or more unit doses for oral administration, each unit dose comprising 0.1 mg to 1000 mg, preferably 1 mg to 500 mg, most preferably 5 mg to 250 mg of the second therapeutic composition;
the third therapeutic composition, when present in the therapeutic combination, provided as one or more unit doses for oral administration, each unit dose comprising 0.1 mg to 1000 mg, preferably 1 mg to 500 mg, most preferably 5 mg to 250 mg of the third therapeutic composition. In the kit, the first therapeutic composition comprises at least one of: at least one NADPH oxidase inhibitor, at least one nitric oxide synthase inhibitor and at least one soluble guanylate cyclase agonist; the second therapeutic composition comprises at least one of: at least one NADPH oxidase inhibitor, at least one nitric oxide synthase inhibitor and at least one soluble guanylate cyclase agonist, wherein the second therapeutic composition is different from the first therapeutic composition; and when present, the third therapeutic composition comprises at least one of: at least one NADPH oxidase inhibitor, at least one nitric oxide synthase inhibitor and at least one soluble guanylate cyclase agonist, wherein the third therapeutic composition is different from the first therapeutic composition and is different from the second therapeutic composition, and optionally the first therapeutic composition, the second therapeutic composition and/or the third therapeutic composition, when present, further comprise(s) a pharmaceutically acceptable diluent and/or a pharmaceutically acceptable excipient.

An aspect of the invention relates to a pharmaceutical composition for use as a medicament, comprising:
two or three of: at least one NADPH oxidase inhibitor, at least one nitric oxide synthase inhibitor and at least one soluble guanylate cyclase agonist or a pharmaceutically acceptable salt thereof; and optionally a pharmaceutically acceptable diluent and optionally a pharmaceutically acceptable excipient. An aspect of the invention relates to a pharmaceutical composition comprising: at least one NADPH oxidase inhibitor or at least one nitric oxide synthase inhibitor and at least one soluble guanylate cyclase agonist or a pharmaceutically acceptable salt thereof; or
at least one NADPH oxidase inhibitor, at least one nitric oxide synthase inhibitor and at least one soluble guanylate cyclase agonist or a pharmaceutically acceptable salt thereof and optionally
a pharmaceutically acceptable diluent and optionally a pharmaceutically acceptable excipient, wherein, the NADPH oxidase inhibitor is selected from setanaxib, GKT136901, GKT137831, GLX7013114, VAS2870, perphenazine, fluphenazine, perazine and thioridazine, and wherein the nitric oxide synthase inhibitor is selected from NG-nitro-L-arginine methyl ester, L-NAME, NG-monomethyl-L-arginine, 2-iminobiotin, ronopterin, S-methyl-1-thiocitrulline and propylthiouracil. It is part of the invention that the pharmaceutical composition can comprise for example two NADPH oxidase inhibitors and one nitric oxide synthase inhibitor, or vice versa; two nitric oxide synthase inhibitors and one soluble guanylate cyclase agonist, or vice versa; two soluble guanylate cyclase agonists and one NADPH oxidase inhibitor, or vice versa; one or two one NADPH oxidase inhibitor(s), one or two nitric oxide synthase inhibitor(s) and one or two soluble guanylate cyclase agonist(s). It is also part of the invention that any one or more of three NADPH oxidase inhibitors, nitric oxide synthase inhibitors and soluble guanylate cyclase agonists can be comprised by the pharmaceutical composition.

An embodiment is the pharmaceutical composition according to the invention, wherein the sole active pharmaceutical ingredients in the pharmaceutical composition are at least one NADPH oxidase inhibitor and at least one nitric oxide synthase inhibitor, preferably a single NADPH oxidase inhibitor and a single nitric oxide synthase inhibitor.

An embodiment is the pharmaceutical composition according to the invention, wherein the sole active pharmaceutical ingredients in the pharmaceutical composition are at least one NADPH oxidase inhibitor and at least one soluble guanylate cyclase agonist, preferably a single NADPH oxidase inhibitor and a single soluble guanylate cyclase agonist.

An embodiment is the pharmaceutical composition according to the invention, wherein the sole active pharmaceutical ingredients in the pharmaceutical composition are at least one nitric oxide synthase inhibitor and at least one soluble guanylate cyclase agonist, preferably a single nitric oxide synthase inhibitor and a single soluble guanylate cyclase agonist.

An embodiment is the pharmaceutical composition according to the invention, wherein the pharmaceutical composition comprises the combination of at least one NADPH oxidase inhibitor, at least one nitric oxide synthase inhibitor and at least one soluble guanylate cyclase agonist, preferably the combination of a single NADPH oxidase inhibitor, a single nitric oxide synthase inhibitor and a single soluble guanylate cyclase agonist, or wherein the sole active pharmaceutical ingredients in the composition are the combination of at least one NADPH oxidase inhibitor, at least one nitric oxide synthase inhibitor and at least one soluble guanylate cyclase agonist, preferably the combination of a single NADPH oxidase inhibitor, a single nitric oxide synthase inhibitor and a single soluble guanylate cyclase agonist.

An embodiment relates to the pharmaceutical composition according to the invention for use in the prevention or treatment of brain ischemia.

An embodiment relates to the composition comprising two or three of: at least one NADPH oxidase inhibitor, at least one nitric oxide synthase inhibitor and at least one soluble guanylate cyclase agonist.

An embodiment relates to the composition for use in the prevention or treatment of brain ischemia, cerebral infarction, ischemic stroke and/or ischemia-reperfusion injury.

### BRIEF DESCRIPTION OF THE DRAWINGS

The present invention is based on a novel method to limit reperfusion injury after recanalization of brain regions that have been ischemic for a limited time. It is especially valuable in the context of those cases of brain ischemia where recanalization is effected by thrombolysis or thrombectomy. Without wanting to be limited by mechanistic assumptions or being bound by any theory, the inventors hypothesize that the surprisingly strong protective effect of the double/triple combination therapy can be explained by the fact that by separately inhibiting NADPH oxidase and nitric oxide synthase two distinct pathways of free radical formation are blocked, preventing blood-brain barrier damage and subsequent loss of brain tissue; while on the other hand activators and stimulators of soluble guanylate cyclase (sGC) provide a protective mechanism by functionally reactivating the sGC apo-enzyme that might have suffered loss of its heme cofactor by remaining free radicals.

The detailed description of embodiments of the invention will be better understood when read in conjunction with the appended drawings.

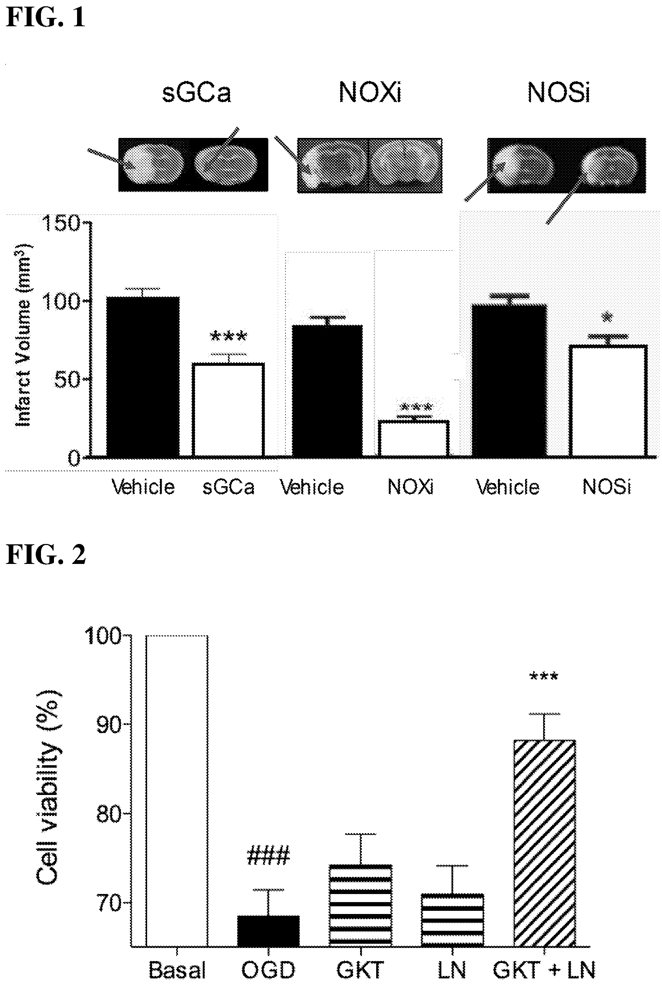

**Figure 1****:** Soluble guanylate cyclase activators (sGCa), NADPH oxidase inhibitors (NOXi) and nitric oxide synthase inhibitors (NOSi) individually achieve statistically significant reductions of infarct volume in wildtype mice subjected to transient occlusion of the middle cerebral artery (tMCAO) when compared with mice treated with vehicle. *P < 0.05, ***P < 0.001 compared to vehicle.
**Figure 2****:** NOXi/NOSi combinations protect mouse hippocampal brain slices against cell death precipitated by 2-hour re-oxygenation after 15 minutes of combined oxygen and glucose deprivation (OGD). GKT = NOXi (GKT-136901, 0.1 µM); LN = NOSi (N omega-Nitro-L-arginine methyl ester, L-NAME; 0.3 µM); GKT+LN = combination at identical respective concentrations. ### P < 0.001 compared to basal conditions; *** P < 0.001 compared to OGD conditions.

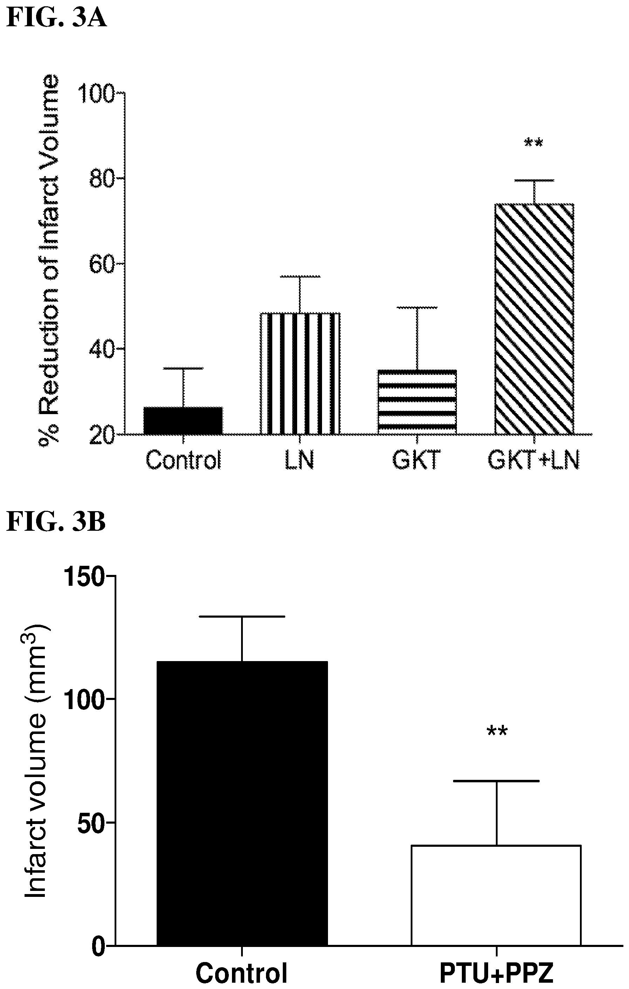

**Figure 3****:** NOXi/NOSi combinations significantly reduce infarct volume in mice after 1-hour tMCAO when administered 1 hour post-ischemia. (A) LN = NOSi (N omega-Nitro-L-arginine methyl ester, L-NAME; 3 mg/kg); GKT = NOXi (GKT-136901, 10 mg/kg); GKT+LN = combination at identical respective doses. ** P < 0.01 compared to controls subjected to tMCAO without subsequent treatment. (B) PTU+PPZ = NOXi perphenazine (PPZ; 1.5 mg/kg) with NOSi propylthiouracil (PTU; 4.5 mg/kg). ** P < 0.01 compared to controls subjected to tMCAO without subsequent treatment.

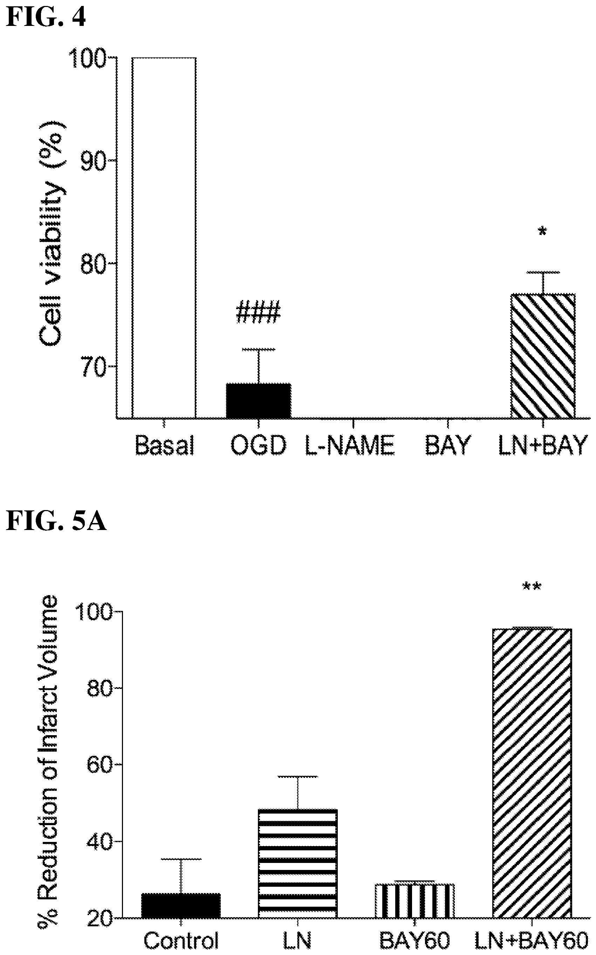

**Figure 4****:** NOSi/sGCa combinations protect rat hippocampal brain slices against cell death precipitated by 2-hour re-oxygenation after 15 minutes of combined oxygen and glucose deprivation (OGD). LN = NOSi (N omega-Nitro-L-arginine methyl ester, L-NAME; 0.3 µM); BAY = sGC activator (BAY 60-2770, 0.01 µM); LN+BAY = combination at identical respective doses. ### P < 0.001 compared to basal conditions, * P < 0.05 compared to OGD conditions.

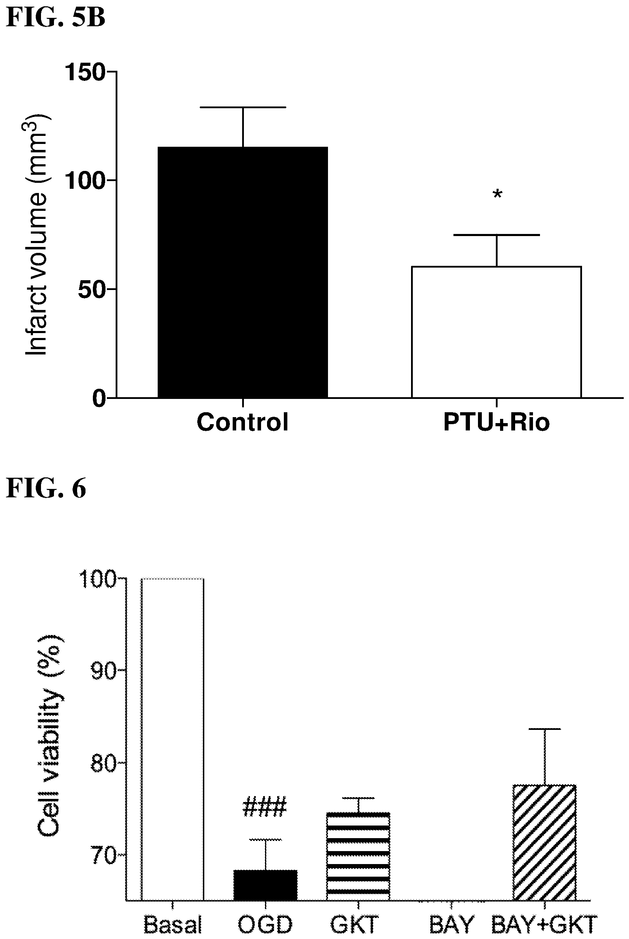

**Figure 5****:** NOSi/sGCa combinations significantly reduce infarct volume in mice after 1-hour tMCAO when administered 1 hour post-ischemia.
   (A) LN = NOSi (N omega-Nitro-L-arginine methyl ester, L-NAME; 3 mg/kg); BAY = sGC activator (BAY 60-2770, 0.5 µg/kg); LN+BAY = combination at identical respective doses. ** P < 0.01 compared to controls subjected to tMCAO without subsequent treatment.
   (B) PTU+Rio = NOSi propylthiouracil (PTU; 4.5 mg/kg) with sGC stimulator riociguat (Rio; 0.05 mg/kg). * P < 0.05 compared to controls subjected to tMCAO without subsequent treatment.
**Figure 6****:** NOXi/sGCa combinations increase protection of rat hippocampal brain slices against cell death precipitated by 2-hour re-oxygenation after 15 minutes of combined oxygen and glucose deprivation (OGD). GKT = NOXi (GKT-136901, 0.1 µM); BAY = sGC activator (BAY 60-2770, 0.01 µM); BAY+GKT = combination at identical respective doses. ### P < 0.001 compared to basal conditions.

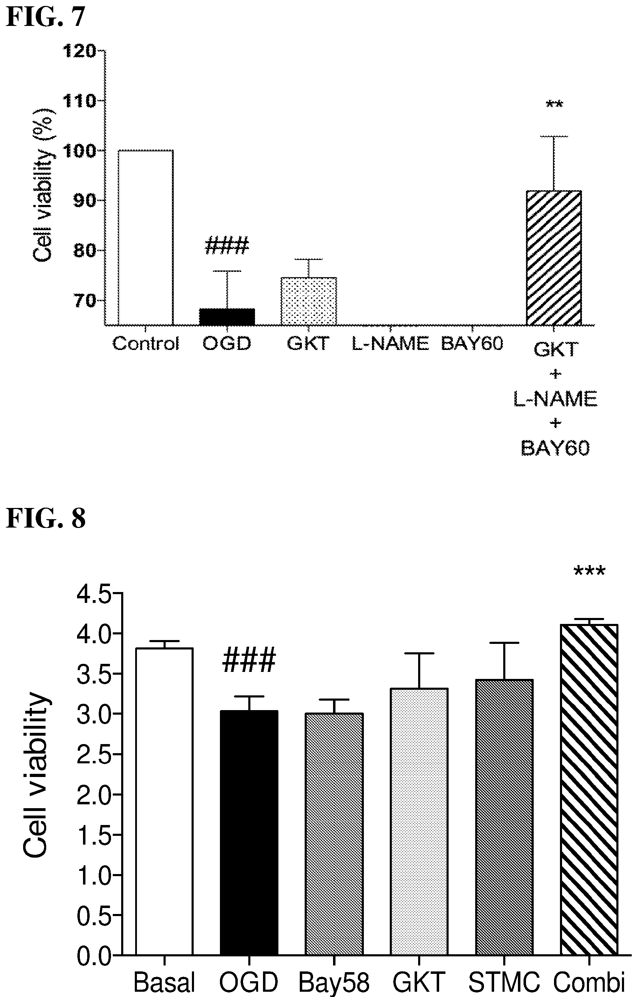

**Figure 7****:** NOXi/NOSi/sGCa triple combinations protect rat hippocampal slices from cell death precipitated by 2-hour re-oxygenation after 15 minutes of combined oxygen and glucose deprivation (OGD). GKT = NOXi (GKT-136901, 0.1 µM); L-NAME = NOSi (N omega-Nitro-L-arginine methyl ester, 0.3 µM); BAY60 = sGC activator (BAY 60-2770), 0.01 µM; GKT+L-NAME+BAY60 = combination at identical respective concentrations. ### P < 0.001 compared to basal conditions; ** P < 0.01 compared to OGD conditions.
**Figure 8****:** NOXi/NOSi/sGCa triple combination administered after oxygen and glucose deprivation (OGD) restores viability of human brain microvascular endothelial cells at 24 hours, and is superior to any individual compound. Bay58 = sGC activator (cinaciguat, i.e. BAY58-2667, 0.01 µM); GKT = NOXi (GKT-136901, 0.1 µM); NOSi (S-methyl-1-thiocitrulline, STMC, 0.3 µM); Combi = combination at identical respective concentrations. ### P < 0.001 compared to basal conditions; *** P < 0.001 compared to OGD conditions.

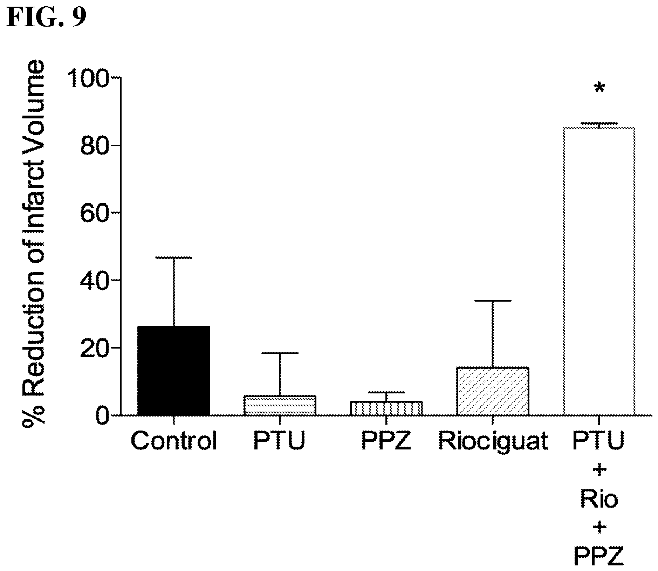

**Figure 9****:** NOXi/NOSi/sGCa triple combinations dramatically reduce infarct volume in mice after 1-hour tMCAO when administered 1 hour post-ischemia while individual compounds are without effect. PTU = NOSi (propylthiouracil, 3 mg/kg); PPZ = NOXi (perphenazine, 1 mg/kg); Rio = sGC stimulator (riociguat, 0.004 mg/kg); PTU+Rio+PPZ = combination at identical respective doses. * P < 0.01 compared to untreated post-ischemic controls.

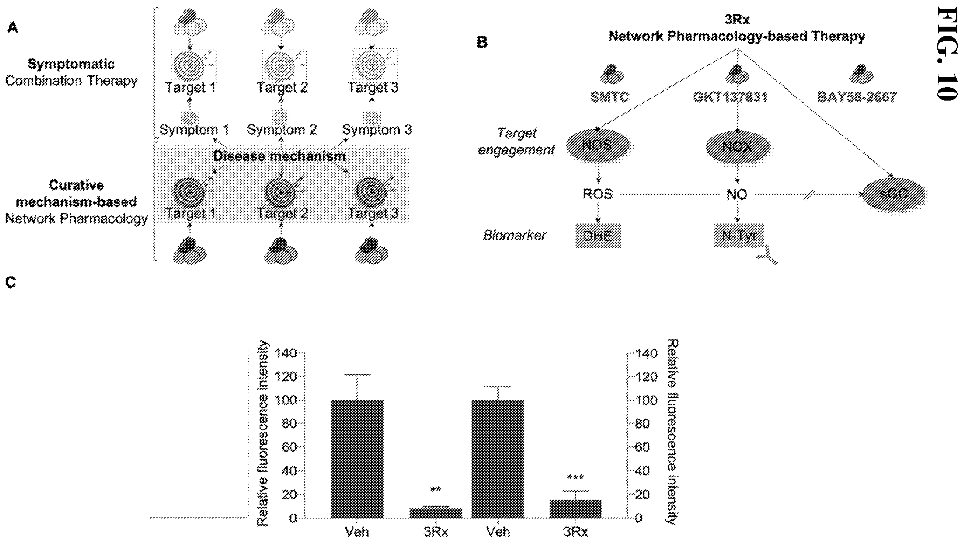

**Figure 10****: Target engagement through ROS/RNS detection. (A)** Classic combination therapy compared to a network pharmacology-based approach. While symptomatic therapy focuses on multiple mechanistically unrelated drugs, often targeting a symptom rather than a mechanism, the inventors propose a curative mechanism-based therapeutic strategy to restore the physiological ROS-cGMP signaling. **(B)** Network pharmacology based triple therapy (3Rx) focused on both NO synthase (NOS) and NADPH oxidase (NOX) inhibition together with sGC activation. ROS formation was assessed using the DHE staining, while N-Tyr was used as a nitration biomarker. **(C)** Treated (3Rx) diabetic mice showed decreased ROS formation compared to non-treated mice (Veh) (***p<0.01, n=*4)*.* Representative staining pictures are shown on the left side of the figure. Nitration levels (N-Tyr) cells were significantly reduced in treated animals (3Rx) in comparison with non-treated mice (Veh) (***p<0.001, n=*4)*.*

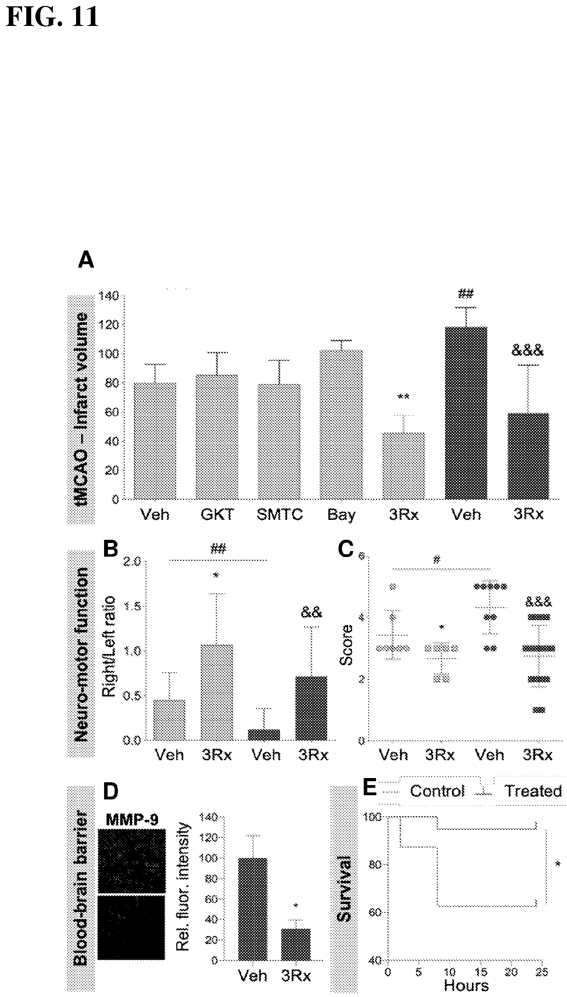

**Figure 11****: Network pharmacology therapy reduced infarct size, stabilized blood-brain barrier, and increases survival in a stroke model with diabetes as a comorbidity.** Diabetes was induced by streptozotocin (STZ) administration in adult mice (6-9 weeks), which were later (12-24 weeks) subjected to 45 min transient occlusion of the middle cerebral artery (tMCAO) followed by 24h reperfusion. Treatment was injected i.p. 1h post-reperfusion. **(A)** 24h post-stroke infarct size was reduced in non-diabetic mice treated with the combination therapy (3Rx), *i.e*. GKT137831 (10 mg/kg), SMTC (1 mg/kg) and BAY58-2667 (0.03 mg/kg), while no effect was detected in animals treated with single-compound subthreshold doses of the same drugs (***p<0.01, n=*7)*.* Moreover, 45 min tMCAO in diabetic mice increased infarct volume in comparison with non-diabetic animals (^{##}*p<0.01, n=*7) being completely prevented by the triple therapy (*^{&&&}*p*<0.001, n=*10)*.* **(B)** Of clinical relevance, neurological outcome (elevated body swing test) was improved in diabetic (*^{&&}p<0.01, n=*10) and non-diabetic (**p<0.05, n=*6) mice under treatment (3Rx) compared to non-treated mice while diabetes significantly aggravated neuromotor functioning in basal (Veh) conditions (*^{##}p<0.01, n=*10)*.* **(C)** Similarly, the Bederson score (23) showed a significant improvement in neuromotor functioning both in comorbid (**p<0.05, n=*6) and non-comorbid (*^{&&&}p<0.001, n=*10) conditions while worsening the outcome in diabetic animals (^{#}*p<0.05, n=*10)*.* **(D)** Blood-brain barrier disruption was indirectly assessed through measuring MMP-9 level. The triple therapy preserved BBB from leakage 1-day after reperfusion (**p<0.05, n=*4)*.* **(E)** Triple therapy increased survival in the acute reperfusion phase (first 24h) compared to non-treated mice.

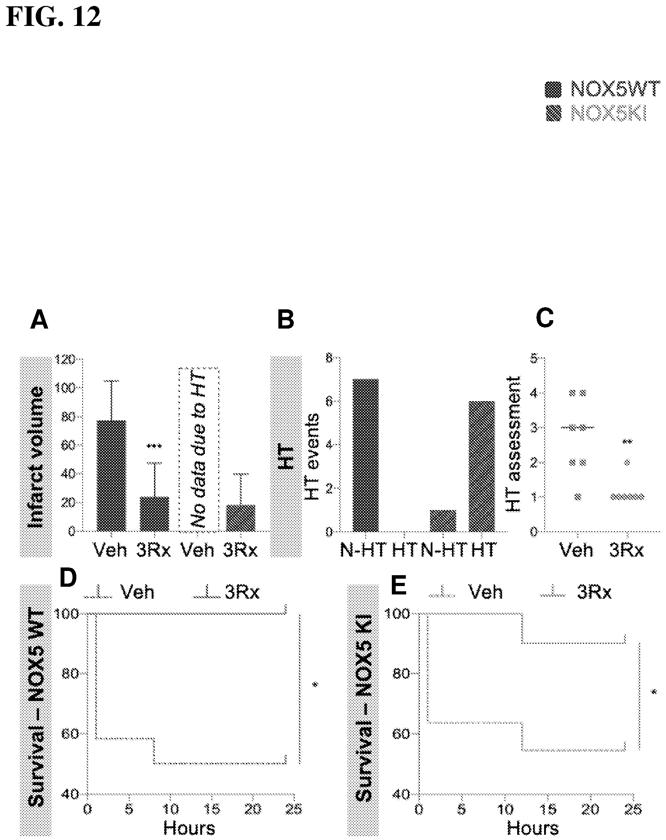

**Figure 12****: Identification of NOX5 as the cause of diabetes-associated hemorrhagic transformation.** The humanized NOX5KI mice were generated as described in [R. Chen, B. Ovbiagele, W. Feng, Diabetes and Stroke: Epidemiology, Pathophysiology, Pharmaceuticals and Outcomes, Am. J. Med. Sci. 351, 380-386 (2016)]. Then, diabetes was induced by streptozotocin administration to NOX5KIIWT adult mice (6-9 weeks), which were later subjected to 45 min transient occlusion of the middle cerebral artery (tMCAO) followed by 24 h reperfusion. Treatment was injected i.p. 1 h post-reperfusion. **(A)** Infarct size was reduced in NOX5WT mice post-treatment (3Rx) 24 h after reperfusion (****p<0.01, n=*8)*.* However, no apparent infarct volume was detected in NOX5KI due to the development of hemorrhagic transformation (HT). Infarct assessment was again possible in NOX5KI treated mice due to the full prevention of HT. **(B)** No HT events occurred in non-treated NOX5WT mice while all NOX5KI animals (n=6) but 1 developed HT within the first 24h post-reperfusion under basal conditions (Veh). **(C)** Macroscopic evaluation of HT in NOX5KI mice showed a significant reduction in cerebral HT after the 3Rx treatment compared to non-treated (Veh) animals (**p<0.05, n=*7)*.* **(D)** In line with previous findings, the triple therapy increased survival in NOX5WT mice (**p<0.05, n=*6) and **(E)** NOX5KI animals within the acute phase post-stroke (**p<0.05, n=*7*).*

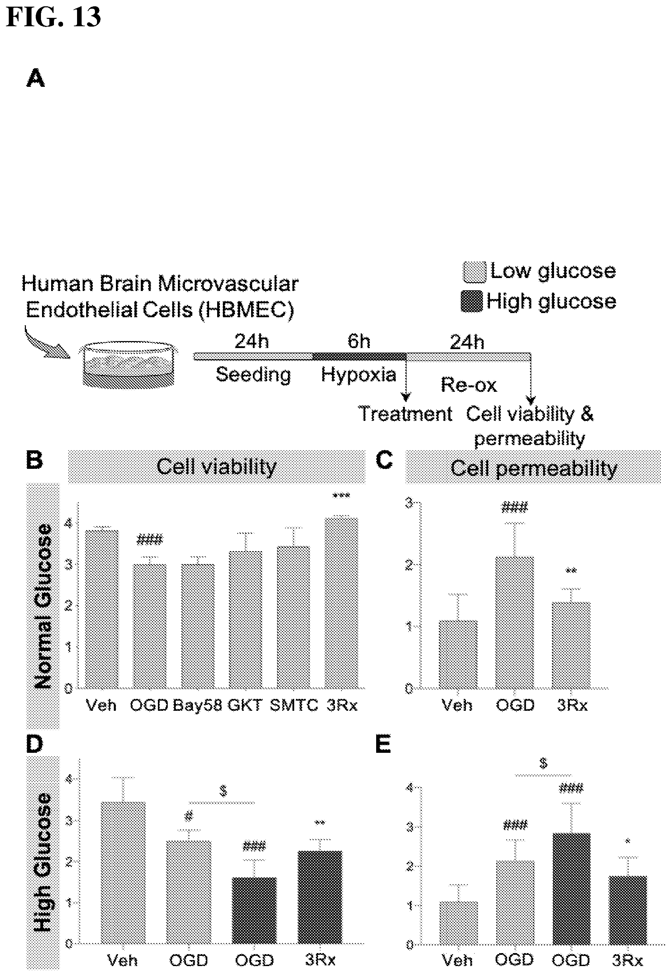

**Figure 13****: Therapeutic translation to an *in vitro* human blood-brain barrier model.** Human Brain Microvascular Endothelial Cells (HBMECs) were incubated under physiological conditions until the optimal cell confluence was reached. Then, HBMECs were subjected to 6h hypoxia (Hyp) in the presence of normal or hyperglycemic conditions followed by 24 h re-oxygenation. To mimic the clinical situation, therapy was added just after re-oxygenation. 1-day later, both cell viability and permeability were assessed. **(A)** Under normal glucose conditions, cell viability significantly increased in cells treated with the combination therapy (3Rx) (****p<0.001, n=*10) while no reduction was detected after single-drug treatments, *i.e*. GKT137831 (0,3 µM), SMTC (0,1 µM), and BAY 58-2667 (0,03 µM) compared to non-treated cells (*^{###}p<0.001, n=*6)*.* **(B)** Similarly, cell permeability was reduced under the triple therapy (***p<0.01, n=6)* in comparison with non-treated cells (*^{###}p<0.001, n=*6)*.* **(C)** To translate previous findings to a human in vitro set-up, cells were subjected to hypoxia under hyperglycemic conditions. The presence of high glucose (25mM) significantly reduced cell viability compared to non-hyperglycemic cells (*^{$}p<0.05, n=5).* This glucose-dependent detrimental effect was fully prevented by the triple therapy (***p<0.01, n=*10, (*^{#}p<0.05, n=*6*, ^{###}p<0.001, n=*6)*.* **(D)** Likewise, cell permeability worsening in hyperglycemic cells compared to normal glycemic cells was completely prevented by the 3Rx therapy (**p<0.05, n=7, ^{$}p<0.05, n=5, ^{###}p<0.001, n=*6)*.*

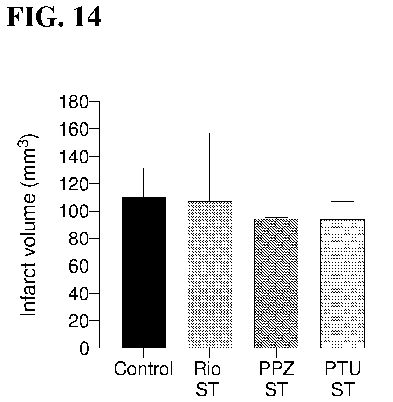

**Figure 14****:** Subthreshold doses (ST) of Rio, PPZ, and PTU showed no reduction of infarct volume in mice after 1-hour tMCAO when administered 1 hour post-ischemia. PTU = NOSi (propylthiouracil, 3 mg/kg); PPZ = NOXi (perphenazine, 1 mg/kg); Rio = sGC stimulator (riociguat, 0.004 mg/kg).

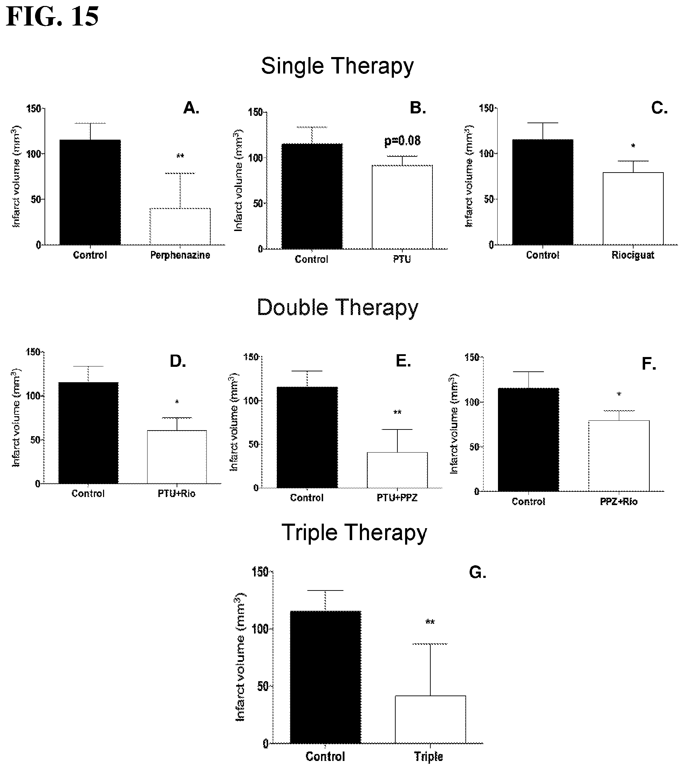

**Figure 15A****-G:** The use of market-authorized drugs to selectively inhibit NOX4/5, NOS, and stimulate sGC leads to significantly smaller infarct size in wild-type mice post-tMCAO. Single therapy: Mice treated with perphenazine (3 mg/kg, i.p.) **(****Figure 15A****)** or riociguat (0.01 mg/kg, i.p.) **(****Figure 15C****)** 1h post-reperfusion had significantly smaller infarct size 24 after ischemia while PTU (9 mg/kg) treatment **(****Figure 15B****)** tends to reduce (n=5, n=4, n=3 respectively; *p<0.05, **p<0.01 compared to non-treated animals). Double therapy: Three different combinations, *i.e*. (PTU + riociguat **(****Figure 15D****);** 4.5mg/kg + 0.05 mg/kg; i.p.), (perphenazine + riociguat (Figure 15F); 1.5 mg/kg + 0.005mg/kg; i.p.), and (PTU + perphenazine **(****Figure 15E****);** 4.5 mg/kg + 1.5 mg/kg; i.p.) were tested in mice 24 h post-tMCAO. All three test groups showed significantly smaller infarct sizes as compared with the control groups (n=3, n=3, n=3 respectively; *p<0.05, **p<0.01 compared to non-treated animals). Triple combination **(****Figure 15G****):** Based on the network pharmacology approach, the drug doses were lowered due to the expected synergistic effect; perphenazine (1 mg/kg, i.p.), PTU (3 mg/kg, i.p.), and riociguat (0.004 mg/kg, i.p.). The synergistic therapy approach significantly reduced infarct size 24 h after stroke reaching the maximal synergistic effect (n=3; **p<0.01 compared to non-treated animals). **The selection criteria for the used doses of the single compounds were: Compound 1 (Perphenazine):** This compound has been previously used as a sedative drug in animal models with doses between 4 mg/kg of body weight and 2 mg/kg [D. B. Carter, M. J. Kennett, C. L. Franklin, Use of perphenazine to control cannibalism in DBA/1 mice. Comp. Med. (2002)]. Therefore, it was decided to use a maximal dose similar to such previous studies, *i.e*. 3 mg/kg. **Compound 2 (Riociguat):** The sGC activator riociguat has been used as a treatment against skin fibrosis in animal models treated with 0.1-3 mg/kg [C. Dees, et al., Stimulators of soluble guanylate cyclase (sGC) inhibit experimental skin fibrosis of different aetiologies. Ann. Rheum. Dis. (2015) https:/doi.org/10.1136/annrheumdis-2014-206809]. Moreover, a similar dosage (100 nM riociguat) aims to modulate platelet function post-treatment [C. Reiss, et al., The sGC stimulator riociguat inhibits platelet function in washed platelets but not in whole blood. Br. J. Pharmacol. (2015) https:/doi.org/10.1111/bph.13286]. Thus, it was decided to use the lowest effective dose, i.e. 0.1 mg/kg to avoid possible hypotensive side-effects [N. Rai, et al., Effect of Riociguat and Sildenafil on Right Heart Remodeling and Function in Pressure Overload Induced Model of Pulmonary Arterial Banding. Biomed Res. Int. (2018) https:/doi.org/10.1155/2018/3293584]. **Compound 3 (Propylthiouracil, PTU):** PTU has been broadly used as an anti-thyroid drug. Most common dosages used preclinically are between 9-12 mg/kg [M. K. Mallela, et al., Evaluation of developmental toxicity of propylthiouracil and methimazole. Birth Defects Res. Part B - Dev. Reprod. Toxicol. (2014) https:/doi.org/10.1002/bdrb.21113; G. Bagnato, et al., Propylthiouracil prevents cutaneous and pulmonary fibrosis in the reactive oxygen species murine model of systemic sclerosis. Arthritis Res. Ther. (2013) https:/doi.org/10.1186/ar4300]. Hence, it was decided to use the lowest previously tested dosage, *i.e*. 9 mg/kg. **Double therapy:** Figure 15D and Figure 15E are the same as Figure 5B and Figure 3B, respectively. **Triple therapy:** the data displayed in Figure 15G is the same data as displayed in Figure 9 though in a different presentation.

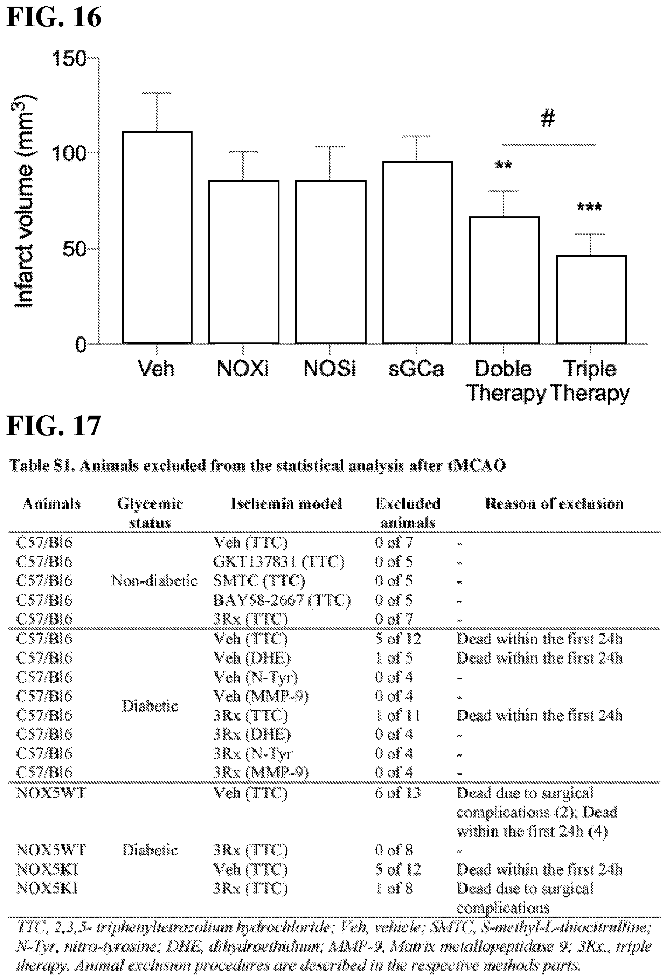

**Figure 16****:** Network pharmacology based therapeutic strategy. Comparison between double and triple combinatory therapy post-stroke using infarct size as the main read-out parameter. Both double (NOXi + NOSi) and triple (NOXi + NOSi + sGCa) therapy significantly reduced infarct volume compared to non-treated animals (**p<0,01; ***p<0,001; n=7). Escalating from a double to a triple therapy potentiates the synergistic effect of the treatment significantly reducing infarct size in comparison with the double therapy (#p<0,05).
**Figure 17****:** Details regarding study design and animal exclusion: Animals excluded from the statistical analysis after tMCAO (Table S1).

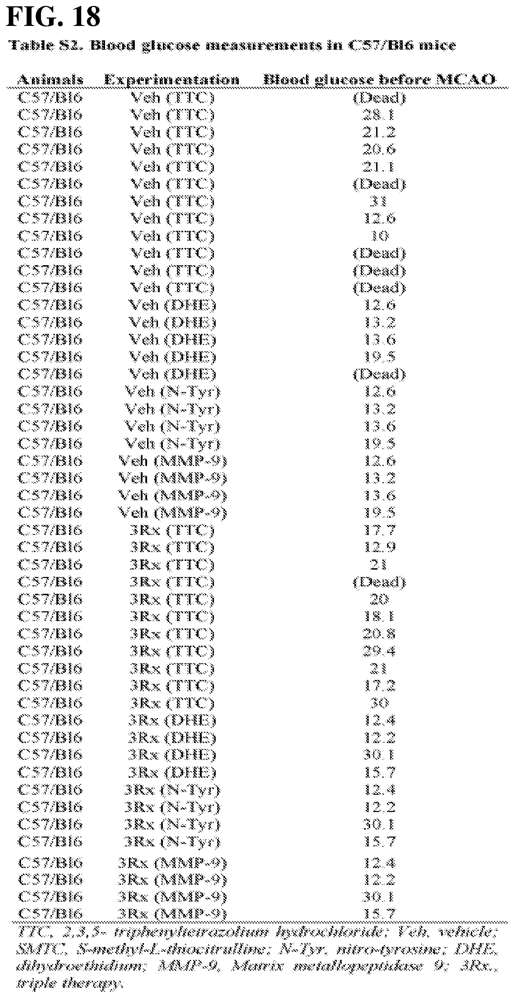

**Figure 18****:** Results of daily monitoring of diabetic mice for any signs of welfare discomfort, and blood glucose levels (Table S2).

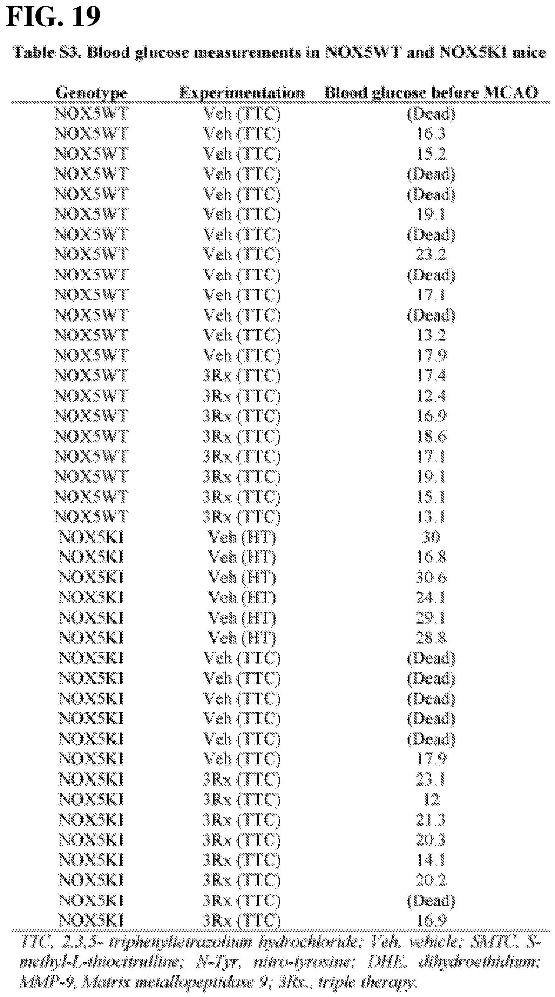

**Figure 19****:** Results of daily monitoring of diabetic mice for any signs of welfare discomfort, and blood glucose levels (Table S3).

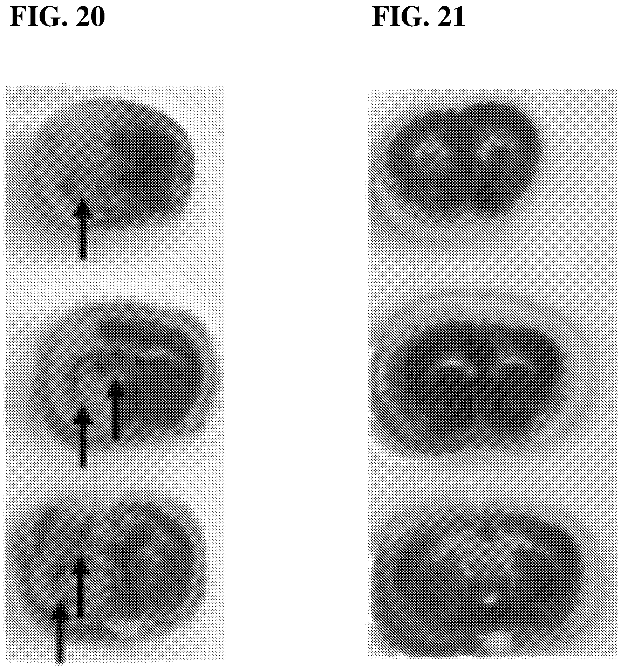

**Figure 20****:** Representative TTC staining pictures of hemorrhagic transformation in NOX5KI mice.
**Figure 21****:** Mechanical validation of the tMCAO model. NOX5KI mice (n=3) were subjected to the complete MCAO surgical procedure although no long-term occlusion was performed. The silicon coated filament was located and rapidly removed from the middle cerebral artery. No vascular disruption was observed.

### DEFINITIONS

As used herein, the terms "treatment," "treating," and the like refer to obtaining a desired pharmacologic and/or physiologic effect with respect to brain ischemia. The effect may be prophylactic in terms of completely or partially preventing brain ischemia or a symptom thereof and/or may be therapeutic in terms of a partial or complete cure for said medical condition and/or adverse effect attributable to the condition. For the purpose of this invention, beneficial or desired clinical results of treatment include, but are not limited to: remission (*i.e*., apparent resolution of the brain ischemia, whether final or temporary); reduction of the extent of brain ischemia; stabilization of the state of said condition; delay or slowing of progression of brain ischemia; alleviation of adverse symptoms of brain ischemia and its subsequent conditions, especially post-ischemic reperfusion injury following restitution of blood supply to the brain volume affected by ischemia, *i.e.*, one or more symptoms, which may be subjective or objective, that are seen or experienced in the untreated state of brain ischemia or reperfusion injury disappear or approach values seen in, or are experienced by, a healthy patient or individual. "Treatment" according to the invention can also include prolonging survival as compared to expected survival if not receiving medical treatment.

The term "therapeutically acceptable amount" or "therapeutically effective dose" interchangeably refer to an amount of one or more active pharmaceutical ingredients that is sufficient to achieve the desired result of treatment as described above. In some embodiments, a therapeutically acceptable amount does not induce or cause undesirable side effects; in other embodiments such undesirable side effects are induced or caused by treatment according to the invention but their intensity and/or duration is deemed acceptable considering the therapeutic objective.

As used herein, the terms "about", "approximately," "substantially," and "significantly" will be understood by persons of ordinary skill in the art and will vary to some extent on the context in which they are used. If there are uses of the term which are not clear to persons of ordinary skill in the art given the context in which it is used, "about" and "approximately" will mean plus or minus <10% of the particular term and "substantially" and "significantly" will mean plus or minus > 10% of the particular term.

"NADPH oxidase" or "NOX" shall mean the family of hydrogen peroxide-forming enzymes also known as NAD(P)H:oxygen oxidoreductases or nicotinamide adenine dinucleotide phosphate oxidases (EC 1.6.3.1). "NADPH oxidase 4" or "NOX4" shall mean the enzyme known as subtype 4 of said enzyme group. "NADPH oxidase inhibitor" or "NOXi" shall mean any compound that reduces the activity of members of this enzyme family.

"Nitric oxide synthase" or "NOS" shall mean the family of enzymes (EC 1.14.13.39) catalyzing the production of nitric oxide (NO) from L-arginine. "Nitric oxide synthase inhibitor" or "NOSi" shall mean any compound that reduces the activity of members of this enzyme family.

"Soluble guanylate cyclase" or "sGC" shall mean the enzyme with the systematic name GTP diphosphate-lyase (cyclizing; 3',5'-cyclic-GMP-forming) (EC 4.6.1.2) which is physiologically activated by nitric oxide. "Soluble guanylate cyclase activator" or "sGCa" shall mean any compound that restores enzymatic activity of the soluble guanylate cyclase apoenzyme, i.e., the protein from which the heme cofactor has been removed, thereby losing its enzymatic activity. For the purpose of the invention this shall also include compounds commonly categorized as "soluble guanylate cyclase stimulators" which the inventors have recently shown to be equally effective on the holoenzyme and the apoenzyme of sGC.

The term "modulator" has its regular scientific meaning and here refers to a compound which is an agonist, *i.e*. a compound capable of binding to a binding site on a protein, and capable of activating said protein upon binding to the binding site, such that a biological response is established due to the activation of the protein.

As used herein, the term "at least additive effect" will be understood by the person skilled in the art as that the effect observed is at least the sum of the separate effects. As an example, if cell viability is at 30% without treatment and compound A restores cell viability to 40%, while compound B restores the cell viability to 45%, then the sum of the effects of A and B is 25% (40-30 + 45-30). If the combination A+B then achieves a cell viability of 55%, which is 25% higher than untreated cells, this is at least an additive effect of the combination in view of the sum of the effects of the separate compounds.

As used herein, the term "synergistic effect" will be understood by the person skilled in the art as that the effect observed is greater than the sum of the separate effects. As an example, if cell viability is at 30% without treatment and compound A restores cell viability to 40%, while compound B restores the cell viability to 45%, then the sum of the effects of A and B is 25% (40-30 + 45-30). If the combination A+B then achieves a cell viability of 65%, which is 35% higher than untreated cells, this is greater than the sum of the separate effects of A and B combined. This is therefore a synergistic effect of the combination of the compounds in view of the sum of the effects of the separate compounds.

The present invention will be described with respect to particular embodiments but the invention is not limited thereto but only by the claims.

Furthermore, the terms first, second, third and the like in the description and in the claims, are used for distinguishing between similar elements and not necessarily for describing a sequential or chronological order. The terms are interchangeable under appropriate circumstances and the embodiments of the invention can operate in other sequences than described or illustrated herein.

Furthermore, the various embodiments, although referred to as "preferred" or "e.g." or "for example" or "in particular" are to be construed as exemplary manners in which the invention may be implemented.

The term "comprising", used in the claims, should not be interpreted as being restricted to the elements or steps listed thereafter; it does not exclude other elements or steps. It needs to be interpreted as specifying the presence of the stated features, integers, steps or components as referred to, but does not preclude the presence or addition of one or more other features, integers, steps or components, or groups thereof. Thus, the scope of the expression "a composition comprising compounds A and B" should not be limited to a composition consisting only of compounds A and B, rather with respect to the present invention, the only enumerated compounds of the composition are compound A and compound B, and further the claim should be interpreted as including equivalents of those compounds.

In addition, reference to an element by the indefinite article "a" or "an" does not exclude the possibility that more than one of the element are present, unless the context clearly requires that there is one and only one of the elements. The indefinite article "a" or "an" thus usually means "at least one".

"N omega-nitro-L-arginine methyl ester" refers to NG-nitro-L-arginine methyl ester, and vice versa.

### ABBREVIATIONS USED

The abbreviation "ELISA" as used herein has its regular scientific meaning throughout the text and stands for "enzyme-linked immunosorbent assay".

The abbreviation "HBMEC" as used herein has its regular scientific meaning throughout the text and stands for "human brain microvascular endothelial cell".

The abbreviation "L-NAME" as used herein has its regular scientific meaning throughout the text and stands for the "inactive prodrug of N omega-nitro-L-arginine methyl ester".

The abbreviation "MCA" as used herein has its regular scientific meaning throughout the text and stands for "middle cerebral artery".

The abbreviation "MCAO" as used herein has its regular scientific meaning throughout the text and stands for "middle cerebral artery occlusion".

The abbreviation "MTT" as used herein has its regular scientific meaning throughout the text and stands for "3-(4,5-dimethylthiazol-2-yl)-2,5-diphenyltetrazolium bromide".

The abbreviation "NADPH" as used herein has its regular scientific meaning throughout the text and stands for "nicotinamide adenine dinucleotide phosphate".

The abbreviation "NOSi" as used herein has its regular scientific meaning throughout the text and stands for "nitric oxide synthase inhibitor".

The abbreviation "NOXi" as used herein has its regular scientific meaning throughout the text and stands for "NADPH oxidase inhibitor".

The abbreviation "OGD" as used herein has its regular scientific meaning throughout the text and stands for "oxygen and glucose deprivation".

The abbreviation "sGC" as used herein has its regular scientific meaning throughout the text and stands for "soluble guanylate cyclase".

The abbreviation "sGCa" as used herein has its regular scientific meaning throughout the text and stands for "soluble guanylate cyclase activator".

The abbreviation "sGCs" as used herein has its regular scientific meaning throughout the text and stands for "soluble guanylate cyclase stimulator".

### DETAILED DESCRIPTION OF THE INVENTION

Herein after, the present invention is described in further detail and is exemplified in the embodiments and exemplifying embodiments of the examples in the Example section. Also described are typical non-limiting embodiments of the invention. Those knowledgeable in the field will readily identify specific additional applications that are not explicitly identified here but are nevertheless considered covered by the invention.

In certain embodiments, it is a first goal of the invention to provide improved therapy options for patients suffering from brain ischemia, cerebral infarction, ischemic stroke and/or ischemia-reperfusion injury. This first goal is at least partially achieved by providing a therapeutic combination for use as a medicament, comprising:
- a first therapeutic composition comprising at least one of: at least one NADPH oxidase inhibitor, at least one nitric oxide synthase inhibitor and at least one soluble guanylate cyclase agonist;
- a second therapeutic composition comprising at least one of: at least one NADPH oxidase inhibitor, at least one nitric oxide synthase inhibitor and at least one soluble guanylate cyclase agonist, wherein the second therapeutic composition is different from the first therapeutic composition; and optionally
- a third therapeutic composition comprising at least one of: at least one NADPH oxidase inhibitor, at least one nitric oxide synthase inhibitor and at least one soluble guanylate cyclase agonist, wherein the third therapeutic composition is different from the first therapeutic composition and is different from the second therapeutic composition;
wherein optionally the first therapeutic composition, the second therapeutic composition and/or the third therapeutic composition, when present, further comprise(s) a pharmaceutically acceptable diluent and/or a pharmaceutically acceptable excipient, and wherein, the NADPH oxidase inhibitor is selected from setanaxib, GKT136901, GKT137831, GLX7013114, VAS2870, perphenazine, fluphenazine, perazine and thioridazine, and wherein the nitric oxide synthase inhibitor is selected NG-nitro-L-arginine methyl ester, L-NAME, NG-monomethyl-L-arginine, 2-iminobiotin, ronopterin, S-methyl-1-thiocitrulline and propylthiouracil.

Preferred is the therapeutic combination comprising:
- a first therapeutic composition comprising at least one of: at least one NADPH oxidase inhibitor and at least one nitric oxide synthase inhibitor;
- a second therapeutic composition comprising at least one soluble guanylate cyclase agonist; and optionally
- a third therapeutic composition comprising at least one of: at least one NADPH oxidase inhibitor and at least one nitric oxide synthase inhibitor, wherein the third therapeutic composition is different from the first therapeutic composition;

wherein optionally the first therapeutic composition, the second therapeutic composition and/or the third therapeutic composition, when present, further comprise(s) a pharmaceutically acceptable diluent and/or a pharmaceutically acceptable excipient,
wherein, the NADPH oxidase inhibitor is selected from setanaxib, GKT136901, GKT137831, GLX7013114, VAS2870, perphenazine, fluphenazine, perazine and thioridazine, and
wherein the nitric oxide synthase inhibitor is selected from NG-nitro-L-arginine methyl ester, L-NAME, NG-monomethyl-L-arginine, 2-iminobiotin, ronopterin, S-methyl-1-thiocitrulline and propylthiouracil.

An embodiment is the therapeutic combination according to the invention, wherein the first therapeutic composition comprises at least one NADPH oxidase inhibitor, preferably one NADPH oxidase inhibitor, and the second therapeutic composition comprises at least one nitric oxide synthase inhibitor, preferably one nitric oxide synthase inhibitor, and wherein the third therapeutic composition, when present, comprises at least one soluble guanylate cyclase agonist, preferably one soluble guanylate cyclase agonist.

An embodiment is the therapeutic combination according to the invention, wherein the first therapeutic composition comprises at least one NADPH oxidase inhibitor, preferably one NADPH oxidase inhibitor, and the second therapeutic composition comprises at least one soluble guanylate cyclase agonist, preferably one soluble guanylate cyclase agonist, and wherein the third therapeutic composition, when present, comprises at least one nitric oxide synthase inhibitor, preferably one nitric oxide synthase inhibitor. Preferred is the therapeutic combination of the invention, wherein the first therapeutic composition comprises at least one NADPH oxidase inhibitor, preferably one NADPH oxidase inhibitor, and wherein the third therapeutic composition, when present, comprises at least one nitric oxide synthase inhibitor, preferably one nitric oxide synthase inhibitor.

An embodiment is the therapeutic combination according to the invention, wherein the first therapeutic composition comprises at least one nitric oxide synthase inhibitor, preferably one nitric oxide synthase inhibitor, and the second therapeutic composition comprises at least one soluble guanylate cyclase agonist, preferably one soluble guanylate cyclase agonist, and wherein the third therapeutic composition, when present, comprises at least one NADPH oxidase inhibitor, preferably one NADPH oxidase inhibitor. Preferred is the therapeutic combination of the invention, wherein the first therapeutic composition comprises at least one nitric oxide synthase inhibitor, preferably one nitric oxide synthase inhibitor and wherein the third therapeutic composition, when present, comprises at least one NADPH oxidase inhibitor, preferably one NADPH oxidase inhibitor.

An embodiment is the therapeutic combination according to the invention, wherein the first therapeutic composition comprises at least one NADPH oxidase inhibitor, preferably one NADPH oxidase inhibitor, the second therapeutic composition comprises at least one nitric oxide synthase inhibitor, preferably one nitric oxide synthase inhibitor, and the third therapeutic composition comprises at least one soluble guanylate cyclase agonist, preferably one soluble guanylate cyclase agonist; preferably, the first therapeutic composition comprises one NADPH oxidase inhibitor, the second therapeutic composition comprises one nitric oxide synthase inhibitor, and the third therapeutic composition comprises one soluble guanylate cyclase agonist.

An embodiment is the therapeutic combination according to the invention, wherein, when present, the at least one NADPH oxidase inhibitor, when present, the at least one nitric oxide synthase inhibitor and, when present, the at least one soluble guanylate cyclase agonist in the first therapeutic composition, in the second therapeutic composition and, when present, in the third therapeutic composition, are the sole pharmaceutically active ingredients in said first, second and third therapeutic compositions; preferably, the first therapeutic composition comprises a single NADPH oxidase inhibitor as the sole pharmaceutically active ingredient, and/or the second therapeutic composition comprises a single nitric oxide synthase inhibitor as the sole pharmaceutically active ingredient, and/or, when present, the third therapeutic composition comprises a single soluble guanylate cyclase agonist as the sole pharmaceutically active ingredient, more preferably, the first therapeutic composition comprises a single NADPH oxidase inhibitor as the sole pharmaceutically active ingredient, and the second therapeutic composition comprises a single nitric oxide synthase inhibitor as the sole pharmaceutically active ingredient, and, when present, the third therapeutic composition comprises a single soluble guanylate cyclase agonist as the sole pharmaceutically active ingredient, most preferably, the therapeutic combination comprises the first, second and third therapeutic compositions. Preferred is the therapeutic combination of the invention, wherein, when present, the at least one NADPH oxidase inhibitor, and when present, the at least one nitric oxide synthase inhibitor in the first and optional third therapeutic composition, and the soluble guanylate cyclase agonist in the second therapeutic composition , are the sole pharmaceutically active ingredients in said first, second and optional third therapeutic compositions; preferably, the therapeutic combination comprises the first, second and third therapeutic compositions.

An embodiment is the therapeutic combination according to the invention, wherein the first pharmaceutical composition comprises one NADPH oxidase inhibitor as the sole active pharmaceutical ingredient and the second pharmaceutical composition comprises one nitric oxide synthase inhibitor as the sole active pharmaceutical ingredient and the third pharmaceutical composition, when present, comprises one soluble guanylate cyclase agonist as the sole active pharmaceutical ingredient, or wherein the first pharmaceutical composition comprises one soluble guanylate cyclase agonist as the sole active pharmaceutical ingredient and the second pharmaceutical composition comprises one nitric oxide synthase inhibitor as the sole active pharmaceutical ingredient, or wherein the first pharmaceutical composition comprises one soluble guanylate cyclase agonist as the sole active pharmaceutical ingredient and the second pharmaceutical composition comprises one NADPH oxidase inhibitor as the sole active pharmaceutical ingredient; preferably, the therapeutic combination comprises the first, second and third therapeutic compositions. Preferred is the therapeutic combination of the invention, wherein the first pharmaceutical composition comprises one NADPH oxidase inhibitor as the sole active pharmaceutical ingredient and the second pharmaceutical composition comprises one soluble guanylate cyclase agonist as the sole active pharmaceutical ingredient and the third pharmaceutical composition, when present, comprises one nitric oxide synthase inhibitor as the sole active pharmaceutical ingredient, or wherein the first pharmaceutical composition comprises one nitric oxide synthase inhibitor as the sole active pharmaceutical ingredient and the second pharmaceutical composition comprises one soluble guanylate cyclase agonist as the sole active pharmaceutical ingredient, or wherein the first pharmaceutical composition comprises one NADPH oxidase inhibitor as the sole active pharmaceutical ingredient and the second pharmaceutical composition comprises one soluble guanylate cyclase agonist as the sole active pharmaceutical ingredient; preferably, the therapeutic combination comprises the first, second and third therapeutic compositions.

An embodiment is the therapeutic combination according to the invention, wherein the therapeutic combination consists of the first, second and third pharmaceutical compositions, wherein the first pharmaceutical composition comprises an NADPH oxidase inhibitor as the sole active pharmaceutical ingredient; wherein the second pharmaceutical composition comprises a nitric oxide synthase inhibitor as the sole active pharmaceutical ingredient; and wherein the third pharmaceutical composition comprises a soluble guanylate cyclase agonist as the sole active pharmaceutical ingredient. Preferred is the therapeutic combination of the invention, wherein the therapeutic combination consists of the first, second and third pharmaceutical compositions, wherein the first pharmaceutical composition comprises an NADPH oxidase inhibitor as the sole active pharmaceutical ingredient; wherein the second pharmaceutical composition comprises a soluble guanylate cyclase agonist as the sole active pharmaceutical ingredient; and wherein the third pharmaceutical composition comprises a nitric oxide synthase inhibitor as the sole active pharmaceutical ingredient.

An embodiment is the therapeutic combination according to the invention, wherein the therapeutic combination consists of the first pharmaceutical composition and the second pharmaceutical composition.

An embodiment is the therapeutic combination according to the invention, wherein the first therapeutic composition comprises an NADPH oxidase inhibitor as the sole active pharmaceutical ingredient and the second therapeutic composition comprises a nitric oxide synthase inhibitor as the sole active pharmaceutical ingredient.

An embodiment is the therapeutic combination according to the invention, wherein the first therapeutic composition comprises an NADPH oxidase inhibitor as the sole active pharmaceutical ingredient and the second therapeutic composition comprises a soluble guanylate cyclase agonist as the sole active pharmaceutical ingredient.

An embodiment is the therapeutic combination according to the invention, wherein the first therapeutic composition comprises a nitric oxide synthase inhibitor as the sole active ingredient and the second therapeutic composition comprises a soluble guanylate cyclase agonist as the sole active pharmaceutical ingredient.

According to the invention, the NADPH oxidase inhibitor is selected from setanaxib, GKT136901, GKT137831, GLX7013114, VAS2870, perphenazine, fluphenazine, perazine and thioridazine, or the NADPH oxidase inhibitor is one of NADPH oxidase inhibitors setanaxib, GKT136901, GKT137831, GLX7013114, VAS2870, perphenazine, fluphenazine, perazine and thioridazine.

An embodiment is the therapeutic combination according to the invention, wherein the NADPH oxidase inhibitor comprises or is selected from GKT137831, GKT136901 and perphenazine or wherein the NADPH oxidase inhibitor is GKT137831, GKT136901 or perphenazine.

According to the invention, the nitric oxide synthase inhibitor is selected from NG-nitro-L-arginine methyl ester, L-NAME, NG-monomethyl-L-arginine, 2-iminobiotin, ronopterin, S-methyl-1-thiocitrulline and propylthiouracil, or the nitric oxide synthase inhibitor is one of nitric oxide synthase inhibitors NG-nitro-L-arginine methyl ester, L-NAME, NG-monomethyl-L-arginine, 2-iminobiotin, ronopterin, S-methyl-1-thiocitrulline and propylthiouracil.

An embodiment is the therapeutic combination according to the invention, wherein the nitric oxide synthase inhibitor, comprises or is selected from L-NAME, S-methyl-1-thiocitrulline and propylthiouracil, or wherein the nitric oxide synthase inhibitor is one of L-NAME, S-methyl-1-thiocitrulline and propylthiouracil.

An embodiment is the therapeutic combination according to the invention, wherein the soluble guanylate cyclase agonist, when present, is a soluble guanylate cyclase activator or a soluble guanylate cyclase stimulator.

An embodiment is the therapeutic combination according to the invention, wherein, when present, the soluble guanylate cyclase agonist comprises or is selected from any one or more of soluble guanylate cyclase agonists cinaciguat, BAY60-2770, BAY41-2272, ataciguat, BI 703704, BI 684067, S-3448, BR-11257, MGV-354, TY-55002, riociguat, vericiguat, nelociguat, olinciguat, BAY41-2772, BAY60-4552, BAY63-2521, IWP-953, A-350619, CF-1571, CFM-1571, lificiguat, etriciguat and praliciguat, or wherein, when present, the soluble guanylate cyclase agonist is one of soluble guanylate cyclase agonists cinaciguat, BAY60-2770, BAY41-2272, ataciguat, BI 703704, BI 684067, S-3448, BR-11257, MGV-354, TY-55002, riociguat, vericiguat, nelociguat, olinciguat, BAY41-2772, BAY60-4552, BAY63-2521, IWP-953, A-350619, CF-1571, CFM-1571, lificiguat, etriciguat and praliciguat.

An embodiment is the therapeutic combination according to the invention, wherein, when present, the soluble guanylate cyclase agonist comprises or is selected from any one or more of cinaciguat, BAY60-2770 and riociguat, or wherein, when present, the soluble guanylate cyclase agonist is one of cinaciguat, BAY60-2770 and riociguat. Preferred is the therapeutic combination of the invention, wherein the soluble guanylate cyclase agonist comprises or is selected from any one or more of cinaciguat, BAY60-2770 and riociguat.

An embodiment is the therapeutic combination according to the invention, wherein the NADPH oxidase inhibitor is GKT137831, GKT136901 or perphenazine, and wherein the nitric oxide synthase inhibitor is L-NAME or propylthiouracil; preferably, the NADPH oxidase inhibitor is GKT136901 and the nitric oxide synthase inhibitor is L-NAME.

An embodiment is the therapeutic combination according to the invention, wherein the NADPH oxidase inhibitor is perphenazine, and wherein the nitric oxide synthase inhibitor is propylthiouracil.

An embodiment is the therapeutic combination according to the invention, wherein the NADPH oxidase inhibitor is GKT136901, and wherein the soluble guanylate cyclase agonist is BAY60-2770.

An embodiment is the therapeutic combination according to the invention, wherein the nitric oxide synthase inhibitor is L-NAME or propylthiouracil, and wherein the soluble guanylate cyclase agonist is BAY60-2770 or riociguat, and preferably, the nitric oxide synthase inhibitor is L-NAME and the soluble guanylate cyclase agonist is BAY60-2770.

An embodiment is the therapeutic combination according to the invention, wherein the nitric oxide synthase inhibitor is propylthiouracil, and wherein the soluble guanylate cyclase agonist is riociguat.

An embodiment is the therapeutic combination according to the invention, wherein the NADPH oxidase inhibitor is one of GKT137831, GKT136901 and perphenazine, wherein the nitric oxide synthase inhibitor is one of L-NAME, propylthiouracil and S-methyl-1-thiocitrulline, and wherein the soluble guanylate cyclase agonist is one of BAY60-2770, riociguat and BAY58-2667 (cinaciguat); preferably, the NADPH oxidase inhibitor is GKT136901, the nitric oxide synthase inhibitor is L-NAME and the soluble guanylate cyclase agonist is BAY60-2770, or preferably the NADPH oxidase inhibitor is perphenazine, the nitric oxide synthase inhibitor is propylthiouracil and the soluble guanylate cyclase agonist is riociguat, or preferably the NADPH oxidase inhibitor is GKT136901, the nitric oxide synthase inhibitor is S-methyl-1-thiocitrulline and the soluble guanylate cyclase agonist is BAY58-2667 (cinaciguat).

An embodiment is the therapeutic combination according to the invention, wherein
a. the first therapeutic composition is provided as a first unit dose comprising:
   i. a first NADPH oxidase inhibitor;
   ii. optionally a second NADPH oxidase inhibitor;
b. the second therapeutic composition is provided as a second unit dose comprising:
   i. a first nitric oxide synthase inhibitor;
   ii. optionally a second nitric oxide synthase inhibitor; and, when present,
c. the third therapeutic composition is provided as a third unit dose comprising:
   i. a first soluble guanylate cyclase agonist; and
   ii. optionally a second soluble guanylate cyclase agonist; or wherein

a. the first therapeutic composition is provided as a first unit dose comprising:
   i. a first soluble guanylate cyclase agonist;
   ii. optionally a second soluble guanylate cyclase agonist;
b. the second therapeutic composition is provided as a second unit dose comprising:
   i. a first nitric oxide synthase inhibitor;
   ii. optionally a second nitric oxide synthase inhibitor; and, when present,
c. the third therapeutic composition is provided as a third unit dose comprising:
   i. a first NADPH oxidase inhibitor; and
   ii. optionally a second NADPH oxidase inhibitor; or wherein

a. the first therapeutic composition is provided as a first unit dose comprising:
   i. a first soluble guanylate cyclase agonist;
   ii. optionally a second soluble guanylate cyclase agonist;
b. the second therapeutic composition is provided as a second unit dose comprising:
   i. a first NADPH oxidase inhibitor;
   ii. optionally a second NADPH oxidase inhibitor; and, when present,
c. the third therapeutic composition is provided as a third unit dose comprising:
   i. a first nitric oxide synthase inhibitor; and
   ii. optionally a second nitric oxide synthase inhibitor;
   wherein preferably the therapeutic combination comprises or consists of the first, second and third therapeutic compositions.

Preferred is the therapeutic combination of the invention, wherein
a. the first therapeutic composition is provided as a first unit dose comprising:
   i. a first NADPH oxidase inhibitor;
   ii. optionally a second NADPH oxidase inhibitor;
b. the second therapeutic composition is provided as a second unit dose comprising:
   i. a first soluble guanylate cyclase agonist; and
   ii. optionally a second soluble guanylate cyclase agonist
      ; and, when present,
c. the third therapeutic composition is provided as a third unit dose comprising:
   i. a first nitric oxide synthase inhibitor;
   ii. optionally a second nitric oxide synthase inhibitor
      ; or wherein

a. the first therapeutic composition is provided as a first unit dose comprising:
   i. a first nitric oxide synthase inhibitor;
   ii. optionally a second nitric oxide synthase inhibitor
b. the second therapeutic composition is provided as a second unit dose comprising:
   i. a first soluble guanylate cyclase agonist;
   ii. optionally a second soluble guanylate cyclase agonist;
      ; and, when present,
c. the third therapeutic composition is provided as a third unit dose comprising:
   i. a first NADPH oxidase inhibitor; and
   ii. optionally a second NADPH oxidase inhibitor; wherein preferably the therapeutic combination comprises or consists of the first, second and third therapeutic compositions.

An embodiment of the invention relates to the therapeutic combination according to the invention, for use in the prevention or treatment of brain ischemia.

An embodiment of the invention relates to the therapeutic combination according to the invention, for use in the prevention or treatment of cerebral infarction.

An embodiment of the invention relates to the therapeutic combination according to the invention, for use in the prevention or treatment of ischemic stroke.

An embodiment of the invention relates to the therapeutic combination according to the invention, for use in the prevention or treatment of ischemia-reperfusion injury.

An embodiment of the invention relates to the therapeutic combination according to the invention for use in the prevention or treatment of any one or more of brain ischemia, cerebral infarction, ischemic stroke and ischemia-reperfusion injury.

In an embodiment of the invention, the cause of brain ischemia is cerebral infarction, *i.e.,* ischemic stroke caused by thrombosis or embolism in the brain or cerebral venous sinus thrombosis. In an embodiment, the ischemic stroke is cryptogenic (of initially or permanently undetermined cause).

In an embodiment of the invention, treatment according to the invention limits or prevents hemorrhagic transformation of the ischemic stroke.

In an embodiment of the invention the cause of brain ischemia is intracerebral arteritis as can occur in patients with autoimmune diseases causing systemic arteriovenous vasculitis (*e.g*., Behcet's disease).

In an embodiment of the invention, the cause of brain ischemia is an extra-cerebral interruption of the blood supply to one or both hemispheres of the brain, *e.g*. resulting from stenosis of the carotid arteries.

In an embodiment of the invention, the brain ischemia is a diffuse whole brain ischemia resulting from asphyxia (*e.g*., drowning, perinatal asphyxia, or assault).

In an embodiment of the invention the brain ischemia is treated by administering an NADPH oxidase inhibitor (NOXi) and a nitric oxide synthase inhibitor (NOSi) to the ischemic or post-ischemic brain. The inhibitors can be administered sequentially, the individual compounds being administered within an interval ranging from one minute to one hour); or can be administered in a combined formulation.

The inventors have found that the combination of a NOXi and NOSi has at least an additive effect and even a synergistic effect, preferably a synergistic effect, when looking at the sum of the effect of the two compounds separately.

In particular, the combination of the NOXi GKT136901 in combination with the NOSi L-NAME seems to have a synergistic effect in the treatment or prevention of brain ischemia and/or ischemic stroke. This is illustrated by Example 1 here below.

The combination of the NOXi perphenazine and the NOSi propylthiouracil has shown a significant effect on infarct volume after a period of ischemia and reperfusion. When this combination was used, the size of an infarct was significantly reduced when compared to an untreated infarct. This is illustrated in Example 2 here below.

Thus, according to the invention the combination of a NOXi and NOSi is applicable and useful in an improved treatment of any one or more of brain ischemia, cerebral infarct, ischemic stroke and ischemia-reperfusion injury.

In an embodiment of the invention the brain ischemia is treated by administering a nitric oxide synthase inhibitor (NOSi) and a soluble guanylate cyclase agonist, such as a soluble guanylate cyclase activator (sGCa) or a soluble guanylate cyclase stimulator (sGCs), to the ischemic or post-ischemic brain. The compounds can be administered sequentially, the compounds being administered within an interval ranging from one minute to one hour apart; or can be administered in a combined formulation.

The inventors have found that the combination of a NOSi and sGCa or sGCs has at least an additive effect, preferably a synergistic effect, when looking at the sum of the effect of the two compounds separately.

In particular, the combination of the NOSi L-NAME and the sGCa BAY60-2770 seems to have a synergistic effect in the treatment or prevention of brain ischemia and/or ischemic stroke. This is illustrated by Example 1 here below.

Additionally the combination of L-NAME and BAY60-2770 also seems to have a synergistic effect in the reduction of infarct volume after a period of ischemia and reperfusion. By using a combination of this NOSi and sGCa a reduction in infarct volume was achieved that was greater than the reduction in volume achieved by the reductions of the separate L-NAME and BAY60-2770 combined. This is illustrated by Example 2 here below.

The combination of the NOSi propylthiouracil and the sGCs riociguat seems to have a significant effect on infarct volume after a period of ischemia and reperfusion. When this combination was used, the size of an infarct was significantly reduced when compared to an untreated infarct. This is illustrated in Example 2 here below.

Thus, the combination of a NOSi and sGCa or sGCs is applicable and useful in an improved treatment of any one or more of brain ischemia, cerebral infarct, ischemic stroke and ischemia-reperfusion injury, according to the invention.

In an embodiment of the invention the brain ischemia is treated by administering an NAPH oxidase inhibitor (NOXi) and a soluble guanylate cyclase agonist, such as a soluble guanylate cyclase activator (sGCa) or a soluble guanylate cyclase stimulator (sGCs), to the ischemic or post-ischemic brain. The compounds can be administered sequentially, the compounds being administered within an interval ranging from one minute to one hour apart; or can be administered in a combined formulation.

The inventors have found that the combination of an NOXi and an sGCa or an sGCs has at least an additive effect, preferably a synergistic effect, when the sum of the achieved effect of the two compounds separately is considered.

The combination of the NOXi GKT136901 and the sGCa BAY60-2770 has at least an additive effect, and even a synergistic effect in the treatment or prevention of brain ischemia and/or ischemic stroke. This is illustrated by Example 1 here below.

Thus, the combination of an NOXi and an sGCa or an sGCs is applicable and useful in an improved treatment of any one or more of brain ischemia, cerebral infarct, ischemic stroke and ischemia-reperfusion injury, according to the invention.

In an embodiment the brain ischemia is treated by administering an NAPH oxidase inhibitor (NOXi), a nitric oxide synthase inhibitor (NOSi), and a soluble guanylate cyclase agonist, such as a soluble guanylate cyclase activator (sGCa) or a soluble guanylate cyclase stimulator (sGCs), to the ischemic or post-ischemic brain. The compounds can be administered sequentially, the compounds being administered within an interval ranging from one minute to one hour apart; or can be administered in a combined formulation containing all three compounds, or can be administered as a combined formulation containing two of the compounds preceded or followed by the third individual agent.

The inventors have found that the combination of an NOXi, NOSi and sGCa or sGCs has at least an additive effect, preferably a synergistic effect on cell viability when improvement of cell viability is concerned, and when the effect on cell viability with the combination of the three compounds is compared with the sum of the effects of the three compounds when tested separately for an effect on cell viability.

In particular, the combination of the NOXi GKT136901, the NOSi L-NAME and the sGCa BAY60-2770 has a synergistic effect in the treatment of brain ischemia and/or ischemic stroke. This is illustrated by Example 1 here below.

Additionally, the combination of the NOXi GKT136901, the NOSi S-metyl-1-thiocitrulline and the sGCa cinaciguat (BAY58-2667) has a synergistic effect in the treatment or prevention of brain ischemia and/or ischemic stroke. This is illustrated by Example 3 here below.

Furthermore, the combination of the NOXi perphenazine, the NOSi propylthiouracil and the sGCs riociguat has a synergistic effect in the reduction of infarct volume after a period of ischemia and reperfusion. By using the combination of these NOXi, NOSi and sGCs, a reduction in infarct volume was achieved that was greater than the combined total reduction in volume achieved when separate subjects were each treated with one of perphenazine, proylthirouracil and riociguat. This is illustrated by Example 2 here below.

Thus, the combination of an NOXi, NOSi and sGCa or sGCs is applicable and useful in an improved treatment of brain ischemia, cerebral infarct, ischemic stroke and ischemia-reperfusion injury, according to the invention.

In an embodiment of the invention the compounds, or any combination thereof, are administered intravenously. In another embodiment the compounds, or any combination thereof, are administered perorally. In yet another embodiment the compounds are administered through different routes; for example, one is administered intravenously while one or two others are administered orally; or vice versa.

In an embodiment of the invention the compounds, or any combination thereof, are administered prior to the initiation of recanalization of the obstructed vessel supplying blood to the ischemic brain region. In another embodiment the compounds, or any combination thereof, are administered together with the initiation of recanalization.

The inventors found that in particular the NOXi GKT136901 showed potent activity in reducing the infarct volume in a brain after a period of ischemia and reperfusion. This is illustrated in Example 2 here below.

The inventors found that in particular the NOSi L-NAME showed potent activity in reducing the infarct volume in a brain after a period of ischemia and reperfusion. This is illustrated in Example 2 here below.

The soluble guanylate cyclase agonist that is suitable for use in the invention can be any pharmacologically acceptable compound with this type of activity. In an embodiment of the invention the soluble guanylate cyclase agonist is a soluble guanylate cyclase activator (sGCa). Non-limiting examples of sGCa that may be used in the invention include cinaciguat (BAY58-2667), BAY60-2770, BAY41-2272, ataciguat (HMR 1766), BI 703704, BI 684067, S-3448, BR-11257, MGV-354, TY-55002, and the compounds claimed in international patent applications WO/2001/19355, WO/2001/19776, WO/2001/19778, WO/2001/19780, WO/2002/070462, WO/2002/070510, and WO/2009/032249.

In another embodiment the soluble guanylate cyclase agonist is a soluble guanylate cyclase stimulator (sGCs). Non-limiting examples of SGCs that may be used in the invention include riociguat (BAY65-2521), vericiguat (BAY1021189 / MK-1242-001), nelociguat (desmethyl riociguat), olinciguat (IW-1701), BAY41-2772, BAY60-4552, BAY63-2521, IWP-953, A-350619, CF-1571, CFM-1571, lificiguat (YC-1), etriciguat, praliciguat (IW-1973), the compounds disclosed in international patent applications WO/2000/06568, WO/2000/06569, WO/2002/42301, WO/2003/095451, WO/2011/147809, WO/2012/004258, WO/2012/028647 und WO/2012/059549, and WO/2014/144100; and the compounds reported by Li et al., Eur J Med Chem 2019; 173: 107-116 (doi: 10.1016/j.ejmech.2019.04.014).

The inventors found that in particular the sGCa BAY60-2770 showed potent activity in reducing the infarct volume in a brain after a period of ischemia and reperfusion. This is illustrated in Example 2 here below.

An embodiment is the therapeutic combination for use according to the invention, wherein at least one, preferably at least two, more preferably all of the first, second and, when present, third therapeutic compositions is administered to a patient in need thereof, preferably administered orally.

An embodiment is the therapeutic combination for use according to the invention, wherein at least one, preferably at least two, more preferably all of the first, second and, when present, third therapeutic compositions is administered parentally to a patient in need thereof.

An embodiment is the therapeutic combination for use according to the invention, wherein the patient in need thereof suffers from an obstructed blood vessel in an ischemic brain region of the patient, and wherein the therapeutic combination is administered to said patient in need thereof prior to initiation of recanalization of the obstructed blood vessel in the ischemic brain region or at the start of the initiation of recanalization of the obstructed blood vessel in the ischemic brain region, preferably an effective dose of the therapeutic combination is administered to said patient in need thereof.

An embodiment is the therapeutic combination for use according to the invention, wherein at least two of the first, second and, when present, third therapeutic compositions are administered sequentially to a patient in need thereof within a time frame of 1 minute to 1 hour, preferably all three of said first, second and third therapeutic compositions are administered sequentially.

An embodiment is the therapeutic combination for use according to the invention, wherein at least two of the first, second and, when present, third therapeutic compositions are co-administered to a patient in need thereof, preferably all three of said first, second and third therapeutic compositions are co-administered.

An embodiment is the therapeutic combination for use according to the invention, wherein the first therapeutic composition is administered to the patient in need thereof orally at a dose of 0.1 mg to 1000 mg, preferably 1 mg to 500 mg, most preferably 5 mg to 250 mg; and/or wherein the second therapeutic composition is administered to the patient in need thereof orally at a dose of 0.1 mg to 1000 mg, preferably 1 mg to 500 mg, most preferably 5 mg to 250 mg; and/or, wherein, when present, the third therapeutic composition is administered to the patient in need thereof orally at a dose of 0.1 mg to 1000 mg, preferably 1 mg to 500 mg, most preferably 5 mg to 250 mg; preferably, the first therapeutic composition, the second therapeutic composition and, when present, the third therapeutic composition are administered to the patient in need thereof orally at a dose of 0.1 mg to 1000 mg, preferably 1 mg to 500 mg, most preferably 5 mg to 250 mg.

An embodiment is the therapeutic combination for use according to the invention, wherein the first therapeutic composition is administered to the patient in need thereof parentally at a dose of 0.01 mg/ml to 10 mg/ml; and/or wherein the second therapeutic composition is administered to the patient in need thereof parentally at a dose of 0.01 mg/ml to 10 mg/ml; and/or, wherein, when present, the third therapeutic composition is administered to the patient in need thereof parentally at a dose of 0.01 mg/ml to 10 mg/ml; preferably, the first therapeutic composition, the second therapeutic composition and, when present, the third therapeutic composition are administered to the patient in need thereof parentally at a dose of 0.01 mg/ml to 10 mg/ml.

An aspect of the invention relates to a kit for use as a medicament comprising: the therapeutic combination according to the invention; and optionally instructions for use,.

An embodiment is the kit according to the invention, comprising:
the first therapeutic composition provided as one or more unit doses for oral administration, each unit dose comprising 0.1 mg to 1000 mg, preferably 1 mg to 500 mg, most preferably 5 mg to 250 mg of the first therapeutic composition;
the second therapeutic composition provided as one or more unit doses for oral administration, each unit dose comprising 0.1 mg to 1000 mg, preferably 1 mg to 500 mg, most preferably 5 mg to 250 mg of the second therapeutic composition;
the third therapeutic composition, when present in the therapeutic combination, provided as one or more unit doses for oral administration, each unit dose comprising 0.1 mg to 1000 mg, preferably 1 mg to 500 mg, most preferably 5 mg to 250 mg of the third therapeutic composition.

An aspect of the invention relates to a pharmaceutical composition for use as a medicament, comprising:
two or three of: at least one NADPH oxidase inhibitor, at least one nitric oxide synthase inhibitor and at least one soluble guanylate cyclase agonist or a pharmaceutically acceptable salt thereof wherein, the NADPH oxidase inhibitor is selected from setanaxib, GKT136901, GKT137831, GLX7013114, VAS2870, perphenazine, fluphenazine, perazine and thioridazine, and wherein the nitric oxide synthase inhibitor is selected NG-nitro-L-arginine methyl ester, L-NAME, NG-monomethyl-L-arginine, 2-iminobiotin, ronopterin, S-methyl-1-thiocitrulline and propylthiouracil; and optionally
a pharmaceutically acceptable diluent and optionally a pharmaceutically acceptable excipient.

Preferred is the pharmaceutical composition of the invention comprising:
at least one NADPH oxidase inhibitor or at least one nitric oxide synthase inhibitor and at least one soluble guanylate cyclase agonist or a pharmaceutically acceptable salt thereof; or
at least one NADPH oxidase inhibitor, at least one nitric oxide synthase inhibitor and at least one soluble guanylate cyclase agonist or a pharmaceutically acceptable salt thereof;
and optionally
   a pharmaceutically acceptable diluent and optionally a pharmaceutically acceptable excipient.

An embodiment is the pharmaceutical composition according to the invention, wherein the sole active pharmaceutical ingredients in the pharmaceutical composition are at least one NADPH oxidase inhibitor and at least one nitric oxide synthase inhibitor, preferably a single NADPH oxidase inhibitor and a single nitric oxide synthase inhibitor.

An embodiment is the pharmaceutical composition according to the invention, wherein the sole active pharmaceutical ingredients in the pharmaceutical composition are at least one NADPH oxidase inhibitor and at least one soluble guanylate cyclase agonist, preferably a single NADPH oxidase inhibitor and a single soluble guanylate cyclase agonist.

An embodiment is the pharmaceutical composition according to the invention, wherein the sole active pharmaceutical ingredients in the pharmaceutical composition are at least one nitric oxide synthase inhibitor and at least one soluble guanylate cyclase agonist, preferably a single nitric oxide synthase inhibitor and a single soluble guanylate cyclase agonist.

An embodiment is the pharmaceutical composition according to the invention, wherein the pharmaceutical composition comprises the combination of at least one NADPH oxidase inhibitor, at least one nitric oxide synthase inhibitor and at least one soluble guanylate cyclase agonist, preferably the combination of a single NADPH oxidase inhibitor, a single nitric oxide synthase inhibitor and a single soluble guanylate cyclase agonist, or wherein the sole active pharmaceutical ingredients in the composition are the combination of at least one NADPH oxidase inhibitor, at least one nitric oxide synthase inhibitor and at least one soluble guanylate cyclase agonist, preferably the combination of a single NADPH oxidase inhibitor, a single nitric oxide synthase inhibitor and a single soluble guanylate cyclase agonist.

An embodiment is the pharmaceutical composition according to the invention, wherein the pharmaceutical composition comprises any one or more of the NADPH oxidase inhibitors selected from setanaxib, GKT136901, GKT137831, GLX7013114, VAS2870, perphenazine, fluphenazine, perazine and thioridazine.

An embodiment is the pharmaceutical composition according to the invention, wherein the pharmaceutical composition comprises any one or more of the NADPH oxidase inhibitors GKT137831, GKT136901 and perphenazine, or wherein the NADPH oxidase inhibitor is GKT137831, GKT136901 and perphenazine.

An embodiment is the pharmaceutical composition according to the invention, wherein the pharmaceutical composition comprises any one or more of the nitric oxide synthase inhibitors selected from NG-nitro-L-arginine methyl ester, L-NAME, NG-monomethyl-L-arginine, 2-iminobiotin, ronopterin, S-methyl-1-thiocitrulline and propylthiouracil.

An embodiment is the pharmaceutical composition according to the invention, wherein the pharmaceutical composition comprises any one or more of the nitric oxide synthase inhibitors L-NAME, S-methyl-1-thiocitrulline and propylthiouracil, or wherein the nitric oxide synthase inhibitor is one of L-NAME, S-methyl-1-thiocitrulline and propylthiouracil.

An embodiment is the pharmaceutical composition according to the invention, wherein the soluble guanylate cyclase agonist is a soluble guanylate cyclase activator or a soluble guanylate cyclase stimulator.

An embodiment is the pharmaceutical composition according to the invention, wherein the pharmaceutical composition comprises any one or more of soluble guanylate cyclase activators selected from cinaciguat, BAY60-2770, BAY41-2272, ataciguat, BI 703704, BI 684067, S-3448, BR-11257, MGV-354, and TY-55002 and/or any one or more of soluble guanylate cyclase stimulators selected from riociguat, vericiguat, nelociguat, olinciguat, BAY41-2772, BAY60-4552, BAY63-2521, IWP-953, A-350619, CF-1571, CFM-1571, lificiguat, etriciguat and praliciguat.

An embodiment is the pharmaceutical composition according to the invention, wherein the pharmaceutical composition comprises any one or more of the soluble guanylate cyclase agonists selected from cinaciguat, BAY60-2770 and riociguat, or wherein the soluble guanylate cyclase agonist is any one of cinaciguat, BAY60-2770 and riociguat.

An embodiment is the pharmaceutical composition according to the invention, wherein the NADPH oxidase inhibitor is GKT137831, GKT136901 or perphenazine, and wherein the nitric oxide synthase inhibitor is L-NAME or propylthiouracil, preferably, the NADPH oxidase inhibitor is GKT136901 and the nitric oxide synthase inhibitor is L-NAME.

An embodiment is the pharmaceutical composition according to the invention, wherein the NADPH oxidase inhibitor is perphenazine, the nitric oxide synthase inhibitor is propylthiouracil.

An embodiment is the pharmaceutical composition according to the invention, wherein the NADPH oxidase inhibitor is GKT136901 and the soluble guanylate cyclase agonist is BAY60-2770.

An embodiment is the pharmaceutical composition according to the invention, wherein the nitric oxide synthase inhibitor is L-NAME and the soluble guanylate cyclase agonist is BAY60-2770.

An embodiment is the pharmaceutical composition according to the invention, wherein the nitric oxide synthase inhibitor is propylthiouracil and the soluble guanylate cyclase agonist is riociguat.

An embodiment is the pharmaceutical composition according to the invention, wherein the NADPH oxidase inhibitor is one of GKT137831, GKT136901 and perphenazine, wherein the nitric oxide synthase inhibitor is one of L-NAME, propylthiouracil and S-methyl-1-thiocitrulline, and wherein the soluble guanylate cyclase agonist is one of BAY60-2770, riociguat and BAY58-2667 (cinaciguat); preferably, the NADPH oxidase inhibitor is GKT136901, the nitric oxide synthase inhibitor is L-NAME and the soluble guanylate cyclase agonist is BAY60-2770, or preferably the NADPH oxidase inhibitor is perphenazine, the nitric oxide synthase inhibitor is propylthiouracil and the soluble guanylate cyclase agonist is riociguat, or preferably the NADPH oxidase inhibitor is GKT136901, the nitric oxide synthase inhibitor is S-methyl-1-thiocitrulline and the soluble guanylate cyclase agonist is BAY58-2667 (cinaciguat).

An embodiment is the pharmaceutical composition according to the invention, wherein the pharmaceutical composition is for oral administration to a patient in need thereof.

An embodiment is the pharmaceutical composition according to the invention, wherein the pharmaceutical composition is a capsule, tablet, powder, granule, solution, syrup or suspension.

An embodiment is the pharmaceutical composition according to the invention, wherein the pharmaceutical composition is formulated for oral administration to the patient in need thereof, and preferably formulated as a unit dose comprising a dose of 0.1 mg to 1000 mg of the active pharmaceutical ingredients, preferably 1 mg to 500 mg, most preferably 5 mg to 250 mg.

An embodiment is the pharmaceutical composition according to the invention, wherein the pharmaceutical composition is suitable for parenteral administration to a patient in need thereof.

An embodiment is the pharmaceutical composition according to the invention, wherein the pharmaceutical composition is a subcutaneous injection, an intramuscular injection, an intravenous injection or an intravascular infusion.

An embodiment is the pharmaceutical composition according to the invention, wherein the pharmaceutical composition is for parental administration to the patient in need thereof, and wherein the pharmaceutical composition comprises the active pharmaceutical ingredients at a concentration of 0.01 mg/ml to 10 mg/ml.

An aspect of the invention relates to a pharmaceutical composition according to the invention for use as a medicament.

An aspect of the invention relates to a pharmaceutical composition according to the invention for use in the prevention or treatment of brain ischemia.

An aspect of the invention relates to a pharmaceutical composition according to the invention for use in the prevention or treatment of cerebral infarction.

An aspect of the invention relates to a pharmaceutical composition according to the invention for use in the prevention or treatment of ischemic stroke.

An aspect of the invention relates to a pharmaceutical composition according to the invention for use in the prevention or treatment of ischemia-reperfusion injury.

An aspect of the invention relates to a pharmaceutical composition according to the invention for use in the prevention or treatment of any one or more of brain ischemia, cerebral infarction, ischemic stroke and ischemia-reperfusion injury.

An embodiment is the pharmaceutical composition for use according to the invention, wherein the patient in need thereof suffers from an obstructed blood vessel in an ischemic brain region of the patient, and wherein the pharmaceutical composition is administered to said patient in need thereof prior to initiation of recanalization of the obstructed blood vessel in the ischemic brain region or at the start of the initiation of recanalization of the obstructed blood vessel in the ischemic brain region, preferably an effective dose of the pharmaceutical composition is administered to said patient in need thereof.

An embodiment is the pharmaceutical composition for use according to the invention, wherein the pharmaceutical composition is administered orally to the patient in need thereof, preferably at a dose of 0.1 mg to 1000 mg, preferably 1 mg to 500 mg, most preferably 5 mg to 250 mg.

An embodiment is the pharmaceutical composition for use according to the invention, wherein the pharmaceutical composition is administered parentally to the patient in need thereof, preferably wherein the pharmaceutical composition comprises the active pharmaceutical ingredients at a concentration of 0.01 mg/ml to 10 mg/ml.

An aspect of the invention relates to a composition comprising two or three of: at least one NADPH oxidase inhibitor, at least one nitric oxide synthase inhibitor and at least one soluble guanylate cyclase agonist.

An embodiment is the composition according to the invention, wherein the composition comprises at least one NADPH oxidase inhibitor and at least one nitric oxide synthase inhibitor, or wherein the sole active pharmaceutical ingredients in the composition are at least one NADPH oxidase inhibitor and at least one nitric oxide synthase inhibitor, preferably a single NADPH oxidase inhibitor and a single nitric oxide synthase inhibitor.

An embodiment is the composition according to the invention, wherein the composition comprises at least one nitric oxide synthase inhibitor and at least one soluble guanylate cyclase agonist, or wherein the sole active pharmaceutical ingredients in the composition are at least one nitric oxide synthase inhibitor and at least one soluble guanylate cyclase agonist, preferably a single nitric oxide synthase inhibitor and a single soluble guanylate cyclase agonist.

An embodiment is the composition according to the invention, wherein the composition comprises at least one NADPH oxidase inhibitor and at least one soluble guanylate cyclase agonist, or wherein the sole active pharmaceutical ingredients in the composition are at least one NADPH oxidase inhibitor and at least one soluble guanylate cyclase agonist, preferably a single NADPH oxidase inhibitor and a single soluble guanylate cyclase agonist.

An embodiment is the composition according to the invention, wherein the composition comprises the combination of at least one NADPH oxidase inhibitor, at least one nitric oxide synthase inhibitor and at least one soluble guanylate cyclase agonist, preferably the combination of a single NADPH oxidase inhibitor, a single nitric oxide synthase inhibitor and a single soluble guanylate cyclase agonist, or wherein the sole active pharmaceutical ingredients in the composition are the combination of at least one NADPH oxidase inhibitor, at least one nitric oxide synthase inhibitor and at least one soluble guanylate cyclase agonist, preferably the combination of a single NADPH oxidase inhibitor, a single nitric oxide synthase inhibitor and a single soluble guanylate cyclase agonist.

An embodiment is the composition according to the invention, wherein the composition comprises any one or more of the NADPH oxidase inhibitors selected from setanaxib, GKT136901, GKT137831, GLX7013114, VAS2870, perphenazine, fluphenazine, perazine and thioridazine, or wherein the NADPH oxidase inhibitor is any one of setanaxib, GKT136901, GKT137831, GLX7013114, VAS2870, perphenazine, fluphenazine, perazine and thioridazine.

An embodiment is the composition according to the invention, wherein the composition comprises any one or more of the NADPH oxidase inhibitors GKT137831, GKT136901 and perphenazine, or wherein the NADPH oxidase inhibitor is GKT137831, GKT136901 or perphenazine.

An embodiment is the composition according to the invention, wherein the composition comprises any one or more of the nitric oxide synthase inhibitors selected from NG-nitro-L-arginine methyl ester, L-NAME, NG-monomethyl-L-arginine, 2-iminobiotin, ronopterin, S-methyl-1-thiocitrulline and propylthiouracil, or wherein the nitric oxide synthase inhibitor is any one of NG-nitro-L-arginine methyl ester, L-NAME, NG-monomethyl-L-arginine, 2-iminobiotin, ronopterin, S-methyl-1-thiocitrulline and propylthiouracil.

An embodiment is the composition according to the invention, wherein the composition comprises any one or more of the nitric oxide synthase inhibitors is L-NAME, S-methyl-1-thiocitrulline and propylthiouracil, or wherein the nitric oxide synthase inhibitor is one of L-NAME, S-methyl-1-thiocitrulline and propylthiouracil.

An embodiment is the composition according to the invention, wherein the soluble guanylate cyclase agonist is a soluble guanylate cyclase activator.

An embodiment is the composition according to the invention, wherein the composition comprises any one or more of soluble guanylate cyclase activators selected from cinaciguat, BAY60-2770, BAY41-2272, ataciguat, BI 703704, BI 684067, S-3448, BR-11257, MGV-354, and TY-55002.

An embodiment is the composition according to the invention, wherein the soluble guanylate cyclase agonist is a soluble guanylate cyclase stimulator.

An embodiment is the composition according to the invention, wherein the composition comprises any one or more of soluble guanylate cyclase stimulators selected from riociguat, vericiguat, nelociguat, olinciguat, BAY41-2772, BAY60-4552, BAY63-2521, IWP-953, A-350619, CF-1571, CFM-1571, lificiguat, etriciguat and praliciguat.

An embodiment is the composition according to the invention, wherein the composition comprises any one or more of the soluble guanylate cyclase agonists selected from cinaciguat, BAY60-2770 and riociguat, or wherein the soluble guanylate cyclase agonist is any one of cinaciguat, BAY60-2770 and riociguat.

An embodiment is the composition according to the invention, wherein the NADPH oxidase inhibitor is GKT136901, and wherein the nitric oxide synthase inhibitor is L-NAME.

An embodiment is the composition according to the invention, wherein the NADPH oxidase inhibitor is perphenazine, the nitric oxide synthase inhibitor is propylthiouracil.

An embodiment is the composition according to the invention, wherein the nitric oxide synthase inhibitor is L-NAME and the soluble guanylate cyclase agonist is BAY60-2770.

An embodiment is the composition according to the invention, wherein the nitric oxide synthase inhibitor is propylthiouracil and the soluble guanylate cyclase agonist is riociguat.

An embodiment is the composition according to the invention, wherein the soluble guanylate cyclase agonist is BAY60-2770 and the NADPH oxidase inhibitor is GKT136901

An embodiment is the composition according to the invention, wherein the NADPH oxidase inhibitor is GKT136901, the nitric oxide synthase inhibitor is L-NAME and the soluble guanylate cyclase agonist is BAY60-2770, or the NADPH oxidase inhibitor is perphenazine, the nitric oxide synthase inhibitor is propylthiouracil and the soluble guanylate cyclase agonist is riociguat.

An embodiment is the composition according to the invention, wherein the NADPH oxidase inhibitor is GKT136901, the nitric oxide synthase inhibitor is S-methyl-1-thiocitrulline and the soluble guanylate cyclase agonist is cinaciguat.

An embodiment of the invention relates to the composition according to the invention for use in the prevention or treatment of brain ischemia.

An embodiment of the invention relates to the composition according to the invention for use in the prevention or treatment of cerebral infarction.

An embodiment of the invention relates to the composition according to the invention for use in the prevention or treatment of ischemic stroke.

An embodiment of the invention relates to the composition according to the invention for use in the prevention or treatment of ischemia-reperfusion injury.

An embodiment is the composition for use according to the invention, wherein the composition is administered parentally.

An embodiment is the composition for use according to the invention, wherein the composition is administered perorally.

An embodiment is the composition for use according to the invention, wherein the composition is administered prior to the initiation of recanalization of the obstructed blood vessel in the ischemic brain region.

An embodiment is the composition for use according to the invention, wherein the composition is administered at the start of the initiation of recanalization of the obstructed blood vessel in the ischemic brain region.

Compositions of the invention and pharmaceutical compositions of the invention and the therapeutic compositions in the therapeutic combinations of the invention may include NADPH oxidase inhibitors, nitric oxidase synthase inhibitors, and sGC stimulators and/or activators, individually or as combination products, in the form of pharmaceutically acceptable salts such as are generally known in the art, and in the case of the present invention, include relatively non-toxic, organic or inorganic salts of the compounds of the present invention. Examples of such salts include, but are not limited to, acid addition salts; basic salts such as alkali metal salts, alkaline earth salts, and ammonium salts; or organic salts may also be used including, e.g., salts of lysine, choline, diethanolamine, ethylenediamine, meglumine (N-methylglucamine), and organic pH buffer compounds.

Compounds as used in this invention or salts thereof may exist in the form of solvates. As used herein, the term "solvate" refers to a complex of variable stoichiometry formed by a solute or a salt thereof) and a solvent. Such solvents for the purpose of the invention may not interfere with the biological activity of the solute. Examples of suitable solvents include water, methanol, ethanol and acetic acid. If the solvent used is water, the solvate may be referred to as a hydrate.

Compositions of the invention and pharmaceutical compositions of the invention and the therapeutic compositions in the therapeutic combinations of the invention may contain stabilizers, preservatives, wetting and emulsifying agents, consistency-improving agents, flavor-improving agents, solubilizers, colorants and masking agents and antioxidants as pharmaceutical adjuvants.

In some embodiments, one or more compounds selected from the NADPH oxidase inhibitors, nitric oxidase synthase inhibitors, and sGC stimulators and/or activators are provided as a prodrug that is inactive or minimally active towards its respective enzyme target and will, after administration, be metabolized or otherwise converted to a biologically active or more active compound with respect to its target enzyme. A prodrug may have, relative to the active drug, altered metabolic stability or transport characteristics, fewer side effects or lower toxicity, or improved flavor.

Pharmaceutical compositions adapted for oral administration may be presented as discrete units such as capsules or tablets; as powders or granules; as solutions, syrups or suspensions (in aqueous or non-aqueous liquids; or as edible foams or whips; or as emulsions). Such pharmaceutical compositions can be solid, semi-solid, or liquid and will comprise, in addition to the active ingredient or ingredients, at least one pharmaceutically acceptable solvent or excipient; for example, a pharmaceutically acceptable carrier and/or a pharmaceutically acceptable diluent. Suitable carriers and/or diluents are well known in the art and include pharmaceutical grade starch, mannitol, lactose, magnesium stearate, sodium saccharin, talcum, cellulose, glucose, sucrose (or other sugar), magnesium carbonate, gelatin oil, alcohol, detergents, emulsifiers or water (preferably sterile). A single discrete unit may comprise a unit dose, such as a daily dose, or multiple discrete units may together contain an amount of two or three of the active pharmaceutical ingredients together that adds up to a daily dose, such as for example two or three discrete units that together comprise a daily dose of the pharmaceutical composition of the invention or the composition of the invention. Preferably, a single discrete unit such as a single tablet contains a daily dose of the pharmaceutical composition of the invention or the composition of the invention. For the therapeutic combinations of the invention, preferably each of the therapeutic compositions is provided as a discrete unit comprising a daily dose of the (combination of) active pharmaceutical ingredient(s) in the first, second and, when present, the third therapeutic composition.

Pharmaceutical compositions or therapeutic compositions in the therapeutic combination of the invention adapted for parenteral administration will be administered by injection (subcutaneously, intramuscularly or intravenously), or by intravascular infusion. Such compositions will include aqueous and non-aqueous sterile injection solution which may contain anti-oxidants, buffers, bacteriostats and solutes which render the formulation substantially isotonic with the blood of the intended recipient; and aqueous and non-aqueous sterile suspensions which may include suspending agents and thickening agents.

Pernasal compositions that are solid will be powders having a particle size for example in the range 20 - 500 µM which is administered into the nasal passage from a container of the powder held close up to the nostrils, or has an outlet that will be inserted into the nostrils for inhalation. Suitable compositions wherein the carrier is a liquid, for administration as a nasal spray or as nasal drops, include aqueous or oil solutions of the active ingredient.

The (pharmaceutical or therapeutic) compositions of the invention can further comprise one or more additional therapeutic compounds that are known to be effective in the context of the therapeutic indication. Preferred are therapeutic compositions in the therapeutic combination, each comprising a single active pharmaceutical ingredient selected from an NADPH oxidase inhibitor a nitric oxide synthase inhibitor and a soluble guanylate cyclase agonist, for the first, second and third therapeutic compositions.

A (pharmaceutical or therapeutic) composition of the invention may be provided in unit dosage form, will generally be provided in a sealed container and may be provided as part of a kit. Such a kit would normally (although not necessarily) include instructions for use. It may include a plurality of said unit dosage forms.

The appropriate amount and frequency of administration of the compounds and compositions of the invention will be determined according to the judgment of the attending clinician considering such factors as the type and severity of the disease and the characteristics of the individual, such as general health, age, sex, body weight and tolerance to drugs. The skilled artisan will be able to determine appropriate dosages depending on these and other factors.

Oral (pharmaceutical or therapeutic) compositions will contain the active ingredients of the invention at doses of 0.1 mg to 1000 mg, preferably at doses of 1 - 500 mg, most preferably at doses of 5-250 mg. Parenteral compositions will contain the active ingredients of the invention at doses of 0.01 mg/ml to 10 mg/ml.

### EMBODIMENTS

Stroke is the leading cause of disability and represents one of the largest unmet medical needs as only one drug is available for treatment. This drug is limited to the acute phase of stroke and dissolves clots that reduce blood flow to the brain. It is, however, only marginally effective, bears a high risk of fatal bleeding and has over 30 contraindications, which is why most stroke patients are not treated with it. There is a strong need for a stroke drug that is broadly applicable, and/or has few or no contraindications, and/or bears no bleeding risk, and/or reduces brain damage and/or improves brain function, preferably a stroke drug that fulfils all of these aspects beneficial to the patient to be treated. The inventors found a therapeutic approach that, surprisingly, fulfils all of the above criteria and that is also innovative from a commercial perspective, and that is rapidly applicable in the clinic. The current invention relates to the field of repurposing of drugs that are already registered but for a different indication than stroke. By combining two or three selected optimal compounds, the risk of the frequent failure of single compounds in drug development is reduced. Without wishing to be bound by any theory, all two or three chosen compounds according to the invention are strongly neuroprotective on their own; all compounds target the same disease mechanism, yet at different positions and thereby potentiate each other according to embodiments of the invention, which increases the chance of therapeutic success such as therapeutic success in clinical studies. The inventors were, to their surprise, also able to lower the dose of each compound, which lowers the risk of possible side effects. In preparation of a clinical trial, which for regulatory requirements has to have safety as primary outcome, the inventors extended the conducted small-animal validation data by conducting a successful large animal safety study with two of the compounds that were suitable for administration in sheep. Moreover, through plasma biomarkers measured in biobank samples from stroke patients the inventors narrowed down the ideal time-window up to which the drug combination is highly likely to be effective, according to the invention.

The present disclosure also relates to a method for the treatment of any one or more of brain ischemia, cerebral infarction, ischemic stroke and ischemia-reperfusion injury, the method comprising the step of administering an effective dose of a therapeutic combination of the invention or a pharmaceutical composition of the invention or a composition of the invention to a patient in need thereof, wherein the patient is preferably a human patient, e.g. a human patient suffering from brain ischemia, cerebral infarction, ischemic stroke or ischemia-reperfusion injury.

### EXAMPLES

The following Examples are meant to illustrate certain embodiments of the invention.

### EXAMPLE 1

*Rat Hippocampal Brain Slice Experiments.* -2-3 months old adult male Sprague-Dawley rats were used. For hippocampal slices preparation and induction of oxygen deprivation and glucose deprivation (OGD), animals were decapitated and the brains were removed and transferred into ice-cold Krebs bicarbonate dissection buffer containing glucose and sucrose. Thereafter, hippocampi were dissected and sectioned in transverse slices of 250 µm. To stabilize tissue after slicing, slices were transferred to sucrose-free dissection buffer during 45 minutes at 34°C. Then, control group slices were incubated 15 minutes in a Krebs solution. Slices subjected to OGD were incubated in a Krebs buffer pre-bubbled with 95% N₂/5% CO₂, where 2-deoxyglucose replaced glucose. OGD period was followed by 2 h of re-oxygenation at 37°C where slices were returned back to an oxygenated Krebs solution containing glucose⁸. During the re-oxygenation period, slices were treated with 0,01 µM BAY60-2770 (BAY; sGCa), 0,1 µM GKT136901 (GKT; NOXi) and 0,3 µM L-NAME (LN; NOSi) separately and in combination at identical respective doses of: NOXi and NOSi, NOSi and sGCa, NOXi and sGCa, and NOXi, NOSi and sGCa. Cellular viability was quantified by their ability to reduce MTT. After re-oxygenation, slices were incubated with MTT (0.5 mg/ml) in Krebs bicarbonate solution for 40 minutes at 37°C. The formazan produced was solubilized by adding 200 µl dimethyl sulfoxide, giving a colored compound whose optical density was measured in an ELISA microplate reader at 540 nm⁹. The results of these measurements are depicted in Figure 2, Figure 4, Figure 6 and Figure 7.

Referring to Figure 2, the NOXi (GKT) and the NOSi (LN) separately, were able to restore the cell viability of the hippocampal cells to some extent when compared to the untreated cells after OGD. Treatment of the cells with the combination of the NOXi and the NOSi showed a(n) (significant) improvement in cell viability when compared to the untreated cells (OGD), as well as when compared to cells treated with the two compounds, separately. This shows that at least an additive effect, moreover a synergistic effect is present when the NOXi and NOSi are combined into one treatment.

Referring to Figure 4, treatment of the cells with the separate NOSi (L-NAME) or with the separate sGCa (BAY) did restore cell viability to a limited degree if at all. That is to say, after treatment the cells were less viable when compared with the untreated control cells after OGD. The combination of the NOSi and the sGCa improves the cell viability when compared with the untreated cells and when compared with cells treated with either the NOSi, or the sGCa, *i.e*. the two compounds separately. In fact, apparently the effect achieved by the combination of the NOSi and sGCa is greater than the effects of the two separate compounds combined: synergistic effect is present when these two compounds are combined into one treatment.

Figure 6 shows that the NOXi (GKT) alone was able to improve cell viability when compared to the untreated cells after OGD. The cells treated with the sGCa (BAY) alone did not show improved cell viability when compared to untreated cells (OGD). The combination of the NOXi and the sGCa showed a greater improvement in cell viability than the improvement achieved for the cells treated with the separate compounds and for the untreated cells. This shows that there is at least an additive effect of combining these two compounds into one treatment.

Referring now to Figure 7, treatment of the cells with the NOXi (GKT) alone was able to improve cell viability compared to cell viability of untreated control cells after OGD. Neither the NOSi (L-NAME), nor the sGCa (BAY60) alone was able to improve cell viability compared to cell viability of untreated cells (OGD). Treatment with these two separate compounds decreases the extent of the restoration of cell viability when compared to untreated control cells. The combination of the three compounds (GKT + L-NAME + BAY60) showed a(n) (significant) improvement in cell viability when compared to the cell viability of untreated cells or to the cell viability of cells treated with the separate compounds. At least an additive effect is apparent of combining these three compounds into one treatment: synergistic effect.

This data shows that the combination of two or all three of an NOSi, an NOXi and an sGCa has a positive effect on the restorative ability of hippocampal cells after going through a situation that mimics brain ischemia. The combination of an NOXi and an NOSi, the combination of an NOSi and an sGCa, and the combination of an NOXi, an NOSi and an sGCa all have an effect on cell viability that is greater than the effect on cell viability achieved by the separate compounds alone. A synergistic effect for the combinations of these compounds is apparent, and at least an additive effect is apparent.

Thus, the combinations of these two or three compounds provide for an improved treatment of brain ischemia and/or ischemic stroke by preventing and/or reversing damage caused by the oxygen deficiencies and glucose deficiencies in an ischemic brain.

### EXAMPLE 2

*In vivo MCAO Ischemia Model.* - C57BL/6J mice were subjected to middle cerebral artery occlusion (MCAO) during 1 h followed by 24 h of reperfusion. Mice were anesthetized with 1.5% isoflurane (Abbott) in a 70% N₂O/30% O₂ mixture. Core body temperature was maintained at 37°C. The external carotid artery was ligated and a rubber-coated 6.0 nylon monofilament (6021; Doccol) was inserted to occlude the origin of the right MCA for 60 minutes. Then, animals were re-anesthetized and the occluding monofilament was withdrawn to allow for reperfusion¹⁰. L-NAME (NOSi, 3 mg/kg) or propylthiouracil (NOSi, 9 mg/kg), GKT136901 (NOXi, 10 mg/kg) or perphenazine (NOXi, 3 mg/kg) and BAY60-2770 (sGCa, 0,5 µg/kg) or riociguat (sGCs, 0.1 mg/kg), as well as combinations of these compounds were dissolved and vehicle or compound(s) were injected intraperitoneally (i.p.) 1 h after reperfusion. After 23 h of reperfusion animals were sacrificed and brains were quickly removed and cut in 3 2-mm thick coronal sections. The slices were stained for 10 minutes at 37°C with 2% 2,3,5-triphenyltetrazolium chloride (TTC, Sigma-Aldrich) in PBS to visualize the infarct volume. The results of these measurements are shown in Figure 1, Figure 3, Figure 5 and Figure 9.

Figure 1 shows that the infarct volume in the brain of the mice treated with either the sGCa, or the NOXi, or the NOSi, was reduced compared to the mice treated with only a vehicle (control). The pictures above the graph visualize that the infarct (indicated by the arrows pointing to the light grey area in the left sides of the brains in the photographs, in the otherwise perfused brains depicted in dark grey in the photographs for each compound) before treatment (left photographs for each compound) has decreased after treatment with these compounds (right photographs for each compound). The light grey area (arrow), representing the infarct, is reduced by treatment with the sGCa, NOXi or NOSi, which proves that these compounds separately have a positive effect on an ischemic brain.

Figure 3A shows the reduction in infarct size was highest when the NOSi (LN) was combined with the NOXi (GKT) compared to when the infarct was treated with the separate compounds or left untreated (control). The beneficial effect obtained with the combination of the NOSi and NOXi was more than the total effect achieved by the two compounds separately: synergy. This shows that a synergistic effect on infarct size is present in the combination of the NOXi and NOSi.

Figure 3B shows the effect of a combination of an alternative NOSi and NOXi, namely propylthiouracil (PTU) and perphenazine (PPZ), which also shows to induce a significantly reduced infarct volume when compared to the untreated infarct (Control). This shows that with alternative NOSi and NOXi compounds the same desired effect in an infarct is achieved as the NOSi L-NAME and NOXi GKT136901.

Referring now to Figure 5A, the NOSi (LN) and the sGCa (BAY60) alone reduced infarct volume when compared to an untreated infarct (Control). However, the largest reduction in infarct volume was observed when the NOSi and the sGCa were combined into one treatment. The combination of the NOSi and the sGCa obtains an effect on the infarct volume that is larger than the effects of the separate NOSi and sGCa combined: synergy. Thus, a synergistic effect in the use of the NOSi and the sGCa combination in one treatment is apparent, and at least an additive effect is apparent.

Fig 5B shows that the combination of an alternative NOSi and sGCs, namely propylthiouracil (PTU) and riociguat (rio), were also able to significantly reduce the infarct volume when compared to an untreated infarct (Control). This shows that alternative NOSi and sGCs compounds induce the same desired effect in an infarct as the NOSi L-NAME and the sGCa BAY60-2770.

Figure 9 shows that the NOSi (PTU), the NOXi (PPZ) and the sGCs (Riociguat/Rio) alone were not able to reduce infarct volume. However, treatment of the animals with the combination of all three of these compounds together showed a significant reduction in infarct volume compared to an untreated infarct (Control). The reduction of the infarct size when treated with all three compounds as a combination in one treatment is larger than the reductions achieved by the three separate compounds combined: synergy. Thus, a synergistic effect is apparently present when the NOSi, NOXi and sGCa are combined in one treatment, and least an additive effect is achieved with the combination of the three compounds.

These data show that a combination of two or all three of an NOSi, an NOXi and an sGCa has a positive effect on the size and extent of a cerebral infarction and potential on ischemia-reperfusion injury. These combinations of two or three of these compounds are thus suitable and useful in an improved treatment of a cerebral infarction and/or ischemia-reperfusion injury in a patient in need thereof.

### EXAMPLE 3

*Human Brain Microvascular Endothelial Cells (HBMEC) Subjected to Hypoxia.* - HBMEC (Cell systems, USA) between passage 3 and 9 were cultured using specialized cell medium enriched with 5% fetal bovine serum. HBMECs were seeded (6x10⁴ cells/ml) in 12 wells-plates and incubated during 24 h at 37°C. Later, cell medium was replaced for non-FBS containing medium followed by 6 h of hypoxia (94,8% N₂, 0.2% O₂ and 5% CO₂) at 37°C using hypoxia workstations. The hypoxia period was followed by 24 h of reperfusion in presence or absence of GKT136901 (GKT, 0.1 µM; NOXi ), BAY58-2667 (BAY58, 0.01 µM; sGCa) or S-methyl-1-thiocitrulline (STMC, 0.3 µM; NOSi) or a combination of the three. Control cells were exposed to normoxia (75% N₂, 20% O₂ and 5% CO₂), normal glucose concentration and enriched medium during the hypoxia period (Basal). After 24 h reperfusion, cell viability was assessed using the colorimetric MTT assay. MTT solution (5 mg/ml) was added to each well (100 µl/ml) and incubated for 2 h at 37°C. The formazan salt formed was solubilized by adding 250 ml DMSO and measured spectrophotometrically at 540 nm. The results of these measurements are shown in Figure 8.

Figure 8 shows that the cells that were untreated and subjected to hypoxia (OGD) and the cells treated with the sGCa (BAY58) alone showed a lower cell viability than the control cells (Basal). Cells treated with the NOXi (GKT) or the NOSi (STMC) alone showed only a relatively small improvement in cell viability when compared to the untreated cells subjected to OGD. The combination of the NOXi, NOSi and sGCa (Combi) showed significant cell viability improvement when compared to the untreated cells. In fact, the improvement in cell viability of the three compounds combined in one treatment was higher than the improvement in cell viabilities of the three separate compounds combined: synergy. Thus, a synergistic effect is apparent when the NOXi, NOSi and sGCa are combined in one treatment, and at least an additive effect is apparent.

These data show that the use of these three NOXi, NOSi and sGCa compounds in combination in the treatment of *e.g*. brain ischemia, cerebral infarction, ischemic stroke and/or ischemia-reperfusion injury, provides an improved treatment and/or even reverses the damage of *e.g*. ischemia-reperfusion injury.

### EXAMPLE 4

*Clinical Study.* - A 90-day prospective clinical study with adaptive design will be conducted in patients with acute ischemic stroke using double and triple combinations of perphenazine (a marketed antipsychotic and also an NADPH oxidase inhibitor), riociguat (an sGc stimulator marketed for pulmonary hypertension) and propylthiouracil (a drug marketed for hyperthyroidism and also a nitric oxide synthase inhibitor). Patients will be stratified for randomization based on age, gender, time of stroke onset, and reperfusion. Patients randomized to the four investigational study arms will receive, in addition to standard of care, either perphenazine plus riociguat; perphenazine plus propylthiouracil; riociguat plus propylthiouracil; or (in a second stage, following a safety review) the triple combination of these compounds will be administered. Patients in the control study arm will receive only standard of care.

### EXAMPLE 5

### ROS-cGMP disease module-based network pharmacology prevents hemorrhagic transformation in real-word stroke-diabetes comorbidity, prevents post-stroke diabetic hemorrhagic transformation, prevents post-stroke diabetic hemorrhagic transformation, and prevents diabetic hemorrhagic transformation.

### Interactome-based disease module identification for ischemic stroke.

To identify the first mechanism-based disease module for stroke therapy, the inventors implemented an *in silico* multi-target approach extended to all known NO-cGMP and ROS clinically translational drug targets, followed by first-neighbor network analysis. A protein-protein interaction (PPI) network was built using the Integrative Interactive Database (IID) *(19)* and a list of clinically validated seed proteins. This list included:
(i) the three human nitric oxide synthase (NOS) isoforms, NOS1, NOS2, and NOS3;
(ii) the soluble guanylate cyclase (sGC) subunits GCYA1, GCYA2, GCYB1, and GCYB2;
(iii) all NADPH oxidase (NOX) isoforms, NOX1, NOX2, NOX3, NOX4, and NOX5;
(iv) both human monoamine oxidase (MAO) isoforms, MAO-A and MAO-B;
(v) xanthine oxidase (XO); and
(vi) the ROS toxifier, myeloperoxidase (MPO).

To correct for non-disease relevant but highly connected proteins (hub nodes), a subnet participation-degree (SPD) score was calculated by normalizing the total PPIs in the subnetwork to the total PPIs within the interactome. The final SPD-pruned network yields to a relevant disease module including NOX5, NOS1, NOS3, and sGC. Moreover, the inventors identified a separate subnetwork composed by NOS2 and further connected to NOX1 and NOX2. GCYB2 does not have any protein interactions (according to IID) and consequently does not appear in the final network. The remaining enzymatic ROS sources appear individually, i.e. MPO, MAO-A, MAO-B, XAO, NOX3, and NOX4 as independent modules. Nevertheless, this method is limited by PPI without considering formed metabolites, i.e. H₂O₂, O₂. Once including generated metabolites, NOX4 turned to be directly connected to NOS based on a previously described guilt-by-association analysis, finally resulting in a protein-metabolic ROS-cGMP disease module formed by NOX4, NOX5, NOS1, NOS2, NOS3 and sGC; and to a certain extent, NOX1 and NOX2.

### Target engagement and ROS biomarker assessment.

Having identified NOX, NOS, and sGC as mechanistically-related targets through an *in silico* disease module construction, the inventors subsequently designed a network pharmacology based therapeutic approach in line with the *in silico* findings. This strategy aims to treat diseases by co-targeting multiple components of a common underline mechanism, altogether towards potential synergistic effects, dose reduction, and decreased side-effects. However, this therapeutic approach should not be confused with a classic combination therapy where multiple mechanistically unrelated drugs are co-prescribed, often targeting a symptom rather than causal mechanisms, and therefore not aiming for a synergistic effect (Fig. 10A). Conversely, the inventors applied a combination of mechanism-based drugs to restore the physiological ROS-cGMP signaling towards neuroprotection. Therefore, first, the inventors examined the link of their therapeutic approach to the enzymatic activity, *i.e*. ROS formation and nitration pattern. Diabetic 12-to-24 week old mice were subjected to 45min of transient middle cerebral occlusion (tMCAO) followed by 23h of reperfusion in absence or presence of the network pharmacology-based triple therapy (3Rx), *i.e*. GKT137831 (10 mg/kg), SMTC (1 mg/kg) and BAY58-2776 (0.03 mg/kg). ROS generation was assessed through dihydroethidium staining of stroked brain cryo-sections while the biomarker N-Tyr revealed nitration levels (Fig. 10B). Both ROS and N-Tyr generation were dramatically reduced in treated mice after 24 h (Fig. 10C), demonstrating a direct link in ROS/RNS reduction and the pharmacological targeting of mechanism-related enzymes.

### Network pharmacology based triple therapy validation in a stroke-diabetes comorbidity model.

Hyperglycemia leads to endothelial dysfunction, systemic inflammation, and thickening of capillary membranes. Thus, early endothelial aging linked to diabetes makes these patients 2-6 times more prone to develop a stroke event, therefore resulting in a dramatically worsen prognosis. However, stroke preclinical research frequently ignores this comorbidity risk overlooking real-world clinical scenarios. Thus, to first validate the inventors' network pharmacology therapeutic strategy in an *in vivo* model, non-diabetic male and female mice were subjected to 45 min tMCAO in the presence or absence of the triple. Single subthreshold treatments showed no neuroprotective effect while the triple therapy reduced infarct volume 24h post-reperfusion in a synergistic manner (Fig. 11A). In fact, the proposed triple therapy approach showed an improved therapeutic effect compared with the dual therapy suggesting a supra-additive synergistic therapy and further re-confirming the network pharmacology strategy (Fig. 16). Moreover, a significant reduction of therapeutic doses aims to maximally reduce possible side-effects towards a fully safe drug profile.

Similar to a clinical scenario, 45 min tMCAO in diabetic mice increased infarct volume in comparison with non-diabetic animals, while the triple therapy completely prevented this diabetic-dependent worsen outcome (Fig. 11A). Although infarct volume remains the standard read-out, neuro-motor dysfunction and life expectancy post-stroke are currently consider the major challenge of stroke survivors. Hence, the inventors additionally assessed two neuro-motor functioning tests in diabetic and non-diabetic comorbid mice, (i) the elevated body swing test (Fig. 11B), and (ii) Bederson score (Fig. 11C), which both showed significant neuro-motor impairment in diabetic animals compared to non-comorbid mice. Surprisingly, 3Rx therapy improved the outcome post-stroke avoiding any worsening due to diabetes. Therefore, the network pharmacology-based therapy prevents neuro-motor dysfunction and increased infarct volume not exclusively on ischemic mice but also on diabetes-stroke comorbid animals.

Blood-brain barrier (BBB) disruption takes place in early ischemic stages being associated with endothelial dysfunction and inflammatory processes, subsequently damaging the neurovascular unit. Additionally, vascular dysfunction post-stroke leads to inflammation of the endothelium, affecting tight-junction and extracellular matrix proteins. High level of matrix metalloproteinase-9 (MMP-9) is currently considered a BBB marker directly correlated to larger infarct volumes, stroke severity, and impaired functional outcome. To test whether the triple therapy modulates blood-brain barrier damage after ischemia, the inventors assessed MMP-9 levels post-stroke in diabetic animals subjected to stroke. In line with previous findings, the 3Rx therapy significantly reduced MMP-9 levels and subsequent blood-brain barrier leakage upon stroke compared to non-treated animals (Fig. 11D).

To link the neuroprotective effect of 3Rx therapy to a possible improvement of life expectancy, the inventors assessed acute mortality over the first 24 h post-stroke. 1-day post-reperfusion, 95% of treated animals survived, while only 62% of non-treated diabetic animals remained alive 24h post-surgery (Fig. 11E). Thus, pharmacological targeting of NOX, NOS, and sGC as part of the same disease module leads to the first potent, mechanistic-based, neuroprotective, and synergistic therapy for currently not treatable patients suffering from diabetes-related poor outcome after a stroke event.

### Identification of NOX5 as the direct cause of diabetes-associated hemorrhagic transformation.

Ca²⁺-dependent *NOX5* has been identified as the mechanistic link between post-reperfusion calcium overload and early BBB opening, playing a key role in stroke patho-mechanism *(21)*. However, this NOX isoform is missing from the mouse genome *(22)*. Thus, to examine the role of NOX5 in a clinical scenario, *i.e.* as part of a diabetes-stroke comorbidity model, hyperglycemia was induced in humanized NOX5 mice later subjected to 40 min tMCAO followed by 24 h reperfusion. Surprisingly, NOX5KI diabetic mice developed hemorrhagic transformation (HT) upon stroke (Fig. 20), and no clear infarct volume was therefore detectable (Fig. 12A). Mechanical validation of the MCAO surgery in diabetic NOX5KI mice was conducted to discard any possible technical bias (Fig. 21).

HT risk has been directly associated with diabetes patients excluding them from any available therapeutic option. In fact, these patients suffer from the worst outcome post-stroke, increased recurrence rate, and terrible prognosis. However, in the humanized *in vivo* model presented here, HT was prevented by the triple therapy (Fig. 12A). Moreover, no HT events occurred in NOX5WT mice, while 6 out of 7 humanized animals developed HT post-reperfusion (Fig. 12B). NOX5KI diabetic mice treated post-stroke showed either no hemorrhage or a small petechial hemorrhagic infarction (Fig. 12C). Of clinical relevance, acute survival upon stroke was assessed both in NOX5WT and NOX5KI diabetic mice. Indeed, the 3Rx combination therapy significantly increased survival both in NOX5WT (Fig. 12D) and NOX5KI (Fig. 12E) diabetic mice post-reperfusion. Hence, NOX5 was identified as the main cause of diabetes-associated hemorrhagic transformation which could be directly prevented by the triple therapy while increasing acute survival.

### In vitro human validation for clinical translation.

Hyperglycemia is strongly associated with the alteration of BBB transporting functions, tight junctions disruption, and thickening of the capillary walls followed by ROS/RNS induction. Brain ischemia dramatically aggravates this pathological scenario. Therefore, to test the translatability of these findings into a future clinical setting, the inventors used a primary culture of human brain microvascular endothelial cells (HBMECs) as an *in vitro* blood-brain barrier model. First, HBMECs expressing NOX5 were subjected endogenously to 6 hours of hypoxia under normal glucose conditions, followed by 24 hours of re-oxygenation. Cell viability was increased in the presence or absence of the triple therapy compared to non-treated cultures while subthreshold concentrations of single-drug therapies remained non-effective (Fig. 13A). Similarly, 3Rx therapy prevented the increase in permeability after hypoxia under normal glucose conditions (Fig. 13B). Importantly, hyperglycemia (25 mM glucose) significantly reduced cell viability post-re-oxygenation in comparison with normal glycemic conditions, which could be prevented by the 3Rx (Fig. 13C). In line with the role of glucose in BBB stability, cell permeability was severely reduced upon hypoxia-hyperglycemia, a phenotype completely prevented by the triple therapy (Fig. 13D). Thus, 3Rx therapy was validated in a human *in vitro* translational model where the hyperglycemia-dependent aggravation of hypoxia could be directly treated. Based on these results it is proposed the first mechanism-based, synergic and neuroprotective therapy for a currently non-treatable disease condition.

Subthreshold doses (ST) of Rio, PPZ, and PTU showed no reduction of infarct volume in mice after 1-hour tMCAO when administered 1 hour post-ischemia (FIG. 14). PTU = NOSi (propylthiouracil, 3 mg/kg); PPZ = NOXi (perphenazine, 1 mg/kg); Rio = sGC stimulator (riociguat, 0.004 mg/kg).

### Materials and Methods

### In silico disease module prediction

A protein-protein interaction subnetwork with 320 proteins as nodes and 3229 protein-protein interactions was extracted from the Integrative Interaction Database (IID) *(12) i.e.* interactome. First, proteins were selected depending on whether they are enzymatic sources of oxidative stress i.e. NOX1, NOX2, NOX3, NOX4, NOX5, MAO, MPO, and XAO or they belong to the NO-cGMP signaling pathway *i.e.* NOS1, NOS2, NOS3, and sGC. From the starting list of seed proteins, all first neighbors' protein interactions were added. In order to emerge weighted disease modules, a subnet participation degree (SPD) score was calculated by normalizing the degree of the protein nodes in the subnetwork to the degree of the nodes in the interactome. Nodes with an SPD score below or equal to 0.20 are excluded. This score cutoff corresponds to 80% of the cumulative sum of the percentage of the nodes, removing non-specific interactions while including most module-specific interactions. The extracted subnetworks include 74 nodes and 305 protein-protein interactions.

### Study design

All animal experiments were performed according to the EU Directive 2010/63/EU for animal experiments and approved by the Dutch law on animal experiments and the Institutional Ethics Committee of Universidad Autónoma de Madrid, Madrid, Spain. Animals were socially housed under controlled conditions (22°C, 55- 65% humidity, 12 h light-dark cycle) and had free access to water and standard laboratory chow. All efforts were made to minimize animal suffering and to reduce the number of animals used in the experiments. 8-20 weeks C57/BlJ, NOX5WT, and NOX5KI adult male and female mice were used for the *in vivo* animal study. NOX5KI animals were compared to their respective matched WT line. Animals were excluded from end-point analyses if death occurred within 24 h after tMCAO. Details regarding the study design and animal exclusion could be found in Fig. 17 (Table S1).

### Generation of a diabetic mouse model

Diabetes was induced in mice (6-9 weeks old) by i.p. injections of streptozotocin (STZ) (Merck Millipore, The Netherlands) dissolved in 0.5M sodium citrate buffer with a final dose of 55 mg/kg during 5 consecutive days. Blood glucose was measured daily for 7 days after the last STZ injection using a glucometer (Contor XT, Ascensia Diabetes Care, The Netherlands). Blood glucose levels higher than 12mM 7 days after STZ injections were considered diabetic and therefore included in the study. Diabetic mice were monitored daily for any signs of welfare discomfort, and blood glucose levels were followed-up weekly (Fig. 18 (Table S2), Fig. 19 (Table S3)).

### In vivo stroke model: Transient occlusion of the middle cerebral artery (tMCAO)

C57BI6/J mice were anesthetized with isoflurane (0.6% in oxygen). The animal was placed on a heating pad, and rectal temperature was maintained at 37.0°C using a homeo-thermic monitoring system (Harvard Apparatus, Spain). The model was conducted as previously described in *(11)*. Transient cerebral ischemia was induced using the intraluminal filament technique. A midline neck incision was made, and the right common and external carotid arteries were isolated and permanently ligated, altogether using a surgical microscope (Tecnoscopio OPMI pico, Carl Zeiss, Meditec Iberia SA, Spain). A temporary microvascular ligature was placed on the internal carotid artery to stop the blood flow temporarily. A silicon rubber-coated monofilament (6023910PK10, Doccol Corporation, Sharon, MA, USA) was inserted through a small incision into the common carotid artery and advanced into the internal carotid artery until the tip of the monofilament is then precisely located at the origin of the right middle cerebral artery and thus interrupting the blood flow completely. The filament was held in place by a tourniquet suture on the common carotid artery to prevent filament displacement during the ischemic period. Animals were maintained under anesthesia during 45 min followed by 23 h reperfusion period starting when the monofilament is removed. After the surgery, wounds were carefully sutured, and animals could recover from surgery. Operation time per animal did not exceed 15 minutes.

### In vivo network-pharmacology based combination therapy

Three different compounds were simultaneously used, *i.e*. S-Methyl-L-thiocitrulline (SMTC, SanBio bv, The Netherlands), GKT137831 (GKT, Genkyotech, Switzerland) and BAY 58-2667 (Bayer Pharmaceuticals, Germany). SMTC was directly dissolved in sterile saline, while GKT and BAY 58-2667 were dissolved in a mixture of DMSO/saline in a ratio of 1/99. SMTC (1 mg/kg), GKT (10 mg/kg) and BAY (0.03 mg/kg), or vehicle (DMSO/saline in a ratio of 1/99) were injected i.p. 30 min after filament removal, i.e. reperfusion. Infarct size after mono-therapy was only tested in non-diabetic animals due to ethical limitations. Indeed, the inventors' ethical approval was focused on go/no-go decisions to further reduce unnecessary animal experimentation. Therefore, testing an already validated, non-effective therapeutic strategy in diabetic animals would result in ethical concerns. Therefore, only the combination therapy was assessed.

### Oxidative stress measurement: DHE staining

Assessing ROS production ex vivo in stroked brain tissue was determined using the fluorescence dye dihydroethidium (DHE, Thermo Fisher Scientific, The Netherlands). Frozen brain cryo-sections (10 µm) were fixated using 4% paraformaldehyde in PBS and then incubated under 2 µM DHE (2mM stock solution) for 30 minutes at 37°C. After three washing steps with PBS, slices were incubated with Hoechst 2 ng/ml (Hoechst 33342, Sigma-Aldrich, The Netherlands) for 10 min at 37°C. The relative pixel intensity was measured in identical regions using a Leica DMI3000 B fluorescence microscope (The Netherlands) and later analyzed with the ImageJ software (National Institutes of Health, USA).

### Assessment of nitrated proteins

Stroked brain tissue cryo-sections (10 µm) were fixed with 4% paraformaldehyde in PBS. After fixation, sections were incubated for 1 h at room temperature using a rabbit polyclonal anti-nitrotyrosine antibody (1:100); (A-21285, ThermoFisher Scientific, The Netherlands) in blocking buffer. After washing in PBS (3x), sections were incubated with the secondary antibody, Alexa Fluor 488 donkey anti-rabbit (1:100); (A-21206, ThermoFisher Scientific, The Netherlands) for 45 min at room temperature. The fluorescent Hoechst33342 dye (ThermoFisher Scientific, The Netherlands) was added (2 ng/ml) for 10 min at room temperature. Sections were washed in PBS and then mounted using a Dako Fluorescence Mounting Medium (S3023, Agilent Technologies, The Netherlands). Immunofluorescent signal was assessed using a Leica DMI3000 B microscope (The Netherlands), and a quantitative analysis of nitro-tyrosine fluorescence was performed with the ImageJ software (National Institute of Health, USA).

### Infarct size determination

After animal sacrificing, brains were quickly removed and cut into four 2mm thick coronal sections using a mouse brain slicer matrix (Zivic Instruments, The Netherlands). Brain slices were kept at 4°C during 5 min and later stained using 2% 2,3,5-triphenyltetrazolium (TTC; Sigma-Aldrich, The Netherlands) for 15 min at room temperature in PBS to visualize the infarctions. Indirect infarct volumes were calculated by volumetry (ImageJ software, National Institutes of Health, USA) according to the following equation:$Vindirect \left({mm}^{3}\right) = Vinfarct \times \left(1-\left(Vih - Vch\right)/Vch\right)$ where the term (Vih - Vch) represents the volume difference between the ischemic hemisphere and the control hemisphere and (Vih - Vch)/Vch expresses this difference as a percentage of the control hemisphere.

### Neuro-motor functioning

Two different neuro-motor functional tests were assessed in both comorbid and non-comorbid animals in presence and absence of the combinatory therapy 24 h post-reperfusion. Test 1. The Bederson Score (23) categorizes the animals based on: Score 0, no apparent neurological deficits; 1, body torsion and forelimb flexion; 2, right side weakness and thus decreased resistance to lateral push; 3, unidirectional circling behavior; 4, longitudinal spinning; 5, no movement. Test 2. During the elevated body swing test, the mice are held ~1 cm from the base of its tail and then elevated above the surface in the vertical axis around 20 cm. A swing was considered whenever the animal moved its head out of the vertical axis to either the left or the right side (more than 10 degrees). The ratio of right/left swings was subsequently analyzed.

### Determination of blood-brain barrier stability

Cryo-sections (10 µm) from stroked mice were fixed in 4% paraformaldehyde (Sigma-Aldrich, The Netherlands) for 10 min, followed by 1h blocking period in 1% bovine serum albumin. Brain sections were incubated overnight at 4°C with a rabbit polyclonal MMP9 antibody (1:100); (ThermoFisher Scientific, The Netherlands). Subsequently, they were incubated for 45 min at room temperature with the secondary antibody, Alexa fluor 488 donkey anti-rabbit (1:200 in 1% BSA in PBS); (ThermoFisher Scientific, The Netherlands). Brain sections were then incubated with 2µg/ml 4',6-diamidino-2-phenylindole (DAPI) fluorescent dye (ThermoFisher, The Netherlands) for 10 min and finally mounted using DAKO mounting medium (Agilent, US). Signal was assessed using a fluorescence microscope Leica DMI3000 B microscope (The Netherlands), and quantitative analysis was performed with the ImageJ software (National Institute of Health, USA).

### Generation of the humanized NOX5KI mouse

Since both mice and rats genome naturally lacks the *NADPH oxidase* 5 gene, the inventors created a new mouse line expressing the human *NOX5* gene under the control of the *Tie2* promoter. Details concerning the generation process could be found in (21).

### Hemorrhagic transformation: Events and assessment

A macroscopic score for HT based on human clinical studies classified brain hemorrhage into five types: (0) no hemorrhage; (1) small petechial hemorrhagic infarction; (2) confluent petechial hemorrhagic infarction; (3) parenchymal hematoma type-1 (<30% of infarct); (4) parenchymal hematoma type-2 (>30% of infarct).

### HEK culture and NOX5 transfections

Human embryonic kidney 293 (HEK293) cells cultured in DMEM medium containing 5% FBS were transfected with pcDNA control plasmid (vector control) or NOX5 plasmid using FuGENE6 transfection reagent (Promega) followed by ROS measurement after 48h.

### ROS measurement by luminol assay

To measure NOX5 activity, superoxide from vector-transfected or NOX5-transfected HEK293 cells was measured by luminol-enhanced chemi-luminescence in white plates as follows. HEK293 cells were cultured in DMEM medium containing 5% FBS and then transfected with pcDNA control or NOX5 plasmids using FuGENE^{®}6 transfection reagent. After 48 h, cells were detached by adding trypsin and then re-suspended in HBSS buffer. Each 50 µl of cell suspension consisted of 100,000 cells was added to each well (in triplicate) in a 96-well plate and incubated at 37°C for 10 min with vehicle, superoxide dismutase (SOD), diphenyleneiodonium chloride (DPI) or GKT137831. After 10 min of incubation, 50 µl reaction buffer (containing 6.4 U/ml HRP and 0.4 mM luminol in KRPG buffer) was added to the 50 µl cell suspension so that the total assay volume was 100 µl. Cells were then stimulated by the addition of 1 µM phorbol 12-myristate 13-acetate (PMA; PKC activator) and the Ca²⁺ ionophore ionomycin (40 µM). Superoxide generation was detected by monitoring relative light units (RLU) with a Wallac luminometer Victor2 at 37°C for 20 min. Cell-free experiments were similarly performed using xanthine 50µM/xanthine oxidase 1mU/ml as the ROS source.

### Human brain microvascular endothelial cells (HBMEC) under hypoxic conditions

HBME cells (Cell systems, USA) between passage 3 and 9 were cultured to ~95% confluence using a specialized cell medium and grow factors (EGM-2 MV BulletKit, Lonza, The Netherlands) enriched with 5% fetal bovine serum (FBS; Sigma-Aldrich, The Netherlands). Before being subjected to hypoxia, HBMECs were seeded at 6x10⁴ cells/ml in 12 wells-plate and incubated for 24 h at 37°C. Then, the cell medium was replaced with non-FBS containing medium (2 ml/well) followed by 6h of hypoxia (94,8% N₂, 0.2% O₂ and 5% CO₂) at 37°C using the hypoxia workstations (Ruskin Invivo2 400 station, The Netherlands). The hypoxia period was followed by 24 h of reperfusion in the presence or absence of the combinatory therapy 0.3 µM GKT137831, 1 µM SMTC, and 0,03 µM BAY 58-2667 under normal glucose conditions or hyperglycemia (25mM glucose). Control cells were exposed to normoxia (75% N₂, 20% O₂, and 5% CO₂) and enriched medium during the hypoxia period.

### Assessment of cell viability in HBMEC

24 h post-re-oxygenation, cell viability was assessed using the colorimetric MTT (3-(4,5-dimethylthiazol-2-yl)-2,5-diphenyltetrazolium bromide) assay *(24)*. MTT solution (5 mg/ml) was added (100 µl/ml) and incubated for 2 h at 37°C. The formazan salt formed was solubilized by adding DMSO (250 µl/well) for later measuring spectrophotometrically its optical density at 540 nm.

### Assessment of cell permeability in HBMEC

### 2x10⁴ HBME cells were seeded and grown to confluence on membranes of Transwell inserts

(12 mm Transwell^{®}-COL Collagen-Coated 3.0 µm Pore PTFE Membrane Insert, Corning, The Netherlands) 24 h before inducing hypoxia. 6 h of ischemic conditions were followed by a 24 h re-oxygenation period in the presence or absence of the combinatory therapy 0.3 µM GKT137831, 1 µM SMTC, and 0,03 µM BAY 58-2667. Cell permeability was assessed using the Evans Blue dye (Sigma-Aldrich, The Netherlands). Before the diffusion experiment, the assay buffer (4% bovine serum albumin in PBS, 1.5 ml) was added to the abluminal side of the insert. The permeability buffer (0.5 ml) containing 4% bovine serum albumin and 0.67 mg/ml Evans blue dye was loaded on the luminal side of the insert followed by 10 min incubation at 37°C. The concentration of Evans Blue in the abluminal chamber was measured by determining the absorbance of 150 µl buffer at 630 nm using a microplate reader.

### Statistical analysis

All results obtained from the *in vitro* (HBMECs), ex vivo (staining and immunohistochemistry), and *in vivo* (tMCAO) experimentation were analyzed using Prism 8.0 software. Data were expressed as the means ± SEM of separate experiments. Statistical comparisons between groups were performed using one-way ANOVA, followed by a Newman-Keuls multiple-comparison test. Differences between the two groups were considered significant at *P*<0.05. In each case, when only two groups were compared, the unpaired two-tailed Student's t-test was applied, where significance was considered at *P*<0.05. For comparison of survival curves, the log-rank (Mantel-Cox) test was used. P<0.05 was considered statistically significant. Numbers of animals necessary to detect a standardized effect size on infarct volumes ≥0.2 (vehicle-treated control mice vs. treated mice) were determined via a priori sample size calculation with the following assumptions: α=0.05; β=0.2; 20% SD of the mean.

### EXAMPLE 6

### Synergistic network-pharmacology intervention: NOXi, NOSi and sGCs

Single target therapeutic approaches resulted in a complete failure for the stroke field. Therefore, the inventors suggest the new so-called strategy, network pharmacology which suggests that complex diseases, i.e. brain ischemia, should be modulated at several mechanistically-related nodes -targets- at the same time. Thus, the inventors first assessed 3 different double therapies *in vivo,*
(i) NOSi + sGCs,
(ii) NOXi + sGCs,
(iii) NOXi + NOSi; and finally,
(iv) the triple combination, NOSi + sGCs + NOXi.

Based on a repurposing strategy, already marketed compounds validated as NOX inhibitors and NOS inhibitors were selected for further pre-clinical experimentation. Similarly, an already approved sGC stimulator was included repurposed for a non-cardiovascular indication. Wildtype mice were subjected to 1 h tMCAO and subsequently treated with each compound separately, in double combination or triple for later assessment of infarct volume 24 h post-stroke (Figure 15A-G).

### REFERENCES

1. Sun et al. Free Radical Damage in Ischemia-Reperfusion Injury: An Obstacle in Acute Ischemic Stroke after Revascularization Therapy. Oxid Med Cell Longev. 2018; 2018:3804979. doi: 10.1155/2018/3804979.
2. Nour et al. Ischemia-Reperfusion Injury in Stroke. Interv Neurol. 2013; 1(3-4): 185-199. doi: 10.1159/000353125.
3. González-Moreno et al. Glycemic Variability and Acute Ischemic Stroke: The Missing Link?. Transl Stroke Res. 2014; 5(6):638-46. doi: 10.1007/s12975-014-0365-7.
4. Casas et al. Reactive Oxygen-Related Disease: Therapeutic Targets and Emerging Clinical Indications. Antioxid Redox Signal. 2015; 23(14):1171-85. doi: 10.1089/ars.2015.6433.
5. Kleinschnitz et al. Post-Stroke Inhibition of Induced NADPH Oxidase Type 4 Prevents Oxidative Stress and Neurodegeneration. PLoS Biol. 2010; 8(9). pii: e1000479. doi: 10.1371/journal.pbio.1000479.
6. Kleinschnitz et al. NOS knockout or inhibition but not disrupting PSD-95-NOS interaction protect against ischemic brain damage. J Cereb Blood Flow Metab. 2016; 36(9):1508-12. doi: 10.1177/0271678X16657094.
7. Atochin et al. Soluble Guanylate Cyclase α1β1 Limits Stroke Size and Attenuates Neurological Injury. Stroke. 2010;41(8):1815-9. doi: 10.1161/STROKEAHA.109.577635.
8. Buendia et al. Neuroprotective mechanism of the novel melatonin derivative Neu-P11 in brain ischemia related models. Neuropharmacology 2015; 99:187-195.
9. Denizot & Lang. Rapid colorimetric assay for cell growth and survival: Modifications to the tetrazolium dye procedure giving improved sensitivity and reliability. J Immunol Methods 1986; 89(2):271-277.
10. Göb et al. Blocking of Plasma Kallikrein Ameliorates Stroke by Reducing Thromboinflammation. Ann Neurol 2015; 77(5):784-803.
11. A. I. Casas, A. A. Hassan, S. J. Larsen, V. Gomez-Rangel, M. Elbatreek, P. W. M. Kleikers, E. Guney, J. Egea, M. G. López, J. Baumbach, H. H. H. W. Schmidt, From single drug targets to synergistic network pharmacology in ischemic stroke, Proc. Natl. Acad. Sci. 116, 7129-7136 (2019).
12. J. Menche, A. Sharma, M. Kitsak, S. D. Ghiassian, M. Vidal, J. Loscalzo, A.-L. Barabasi, Uncovering disease-disease relationships through the incomplete interactome, Science (80-. ). 347, 1257601-1257601 (2015).
13. R. Chen, B. Ovbiagele, W. Feng, Diabetes and Stroke: Epidemiology, Pathophysiology, Pharmaceuticals and Outcomes, Am. J. Med. Sci. 351, 380-386 (2016).
14. D. B. Carter, M. J. Kennett, C. L. Franklin, Use of perphenazine to control cannibalism in DBA/1 mice. Comp. Med. (2002*).*
15. C. Dees, et al., Stimulators of soluble guanylate cyclase (sGC) inhibit experimental skin fibrosis of different aetiologies. Ann. Rheum. Dis. (2015) doi.org/10.1136/annrheumdis-2014-206809.
16. C. Reiss, et al., The sGC stimulator riociguat inhibits platelet function in washed platelets but not in whole blood. Br. J. Pharmacol. (2015) doi.org/10.1111/bph.13286.
17. N. Rai, et al., Effect of Riociguat and Sildenafil on Right Heart Remodeling and Function in Pressure Overload Induced Model of Pulmonary Arterial Banding. Biomed Res. Int. (2018) doi.org/10.1155/2018/3293584.
18. M. K. Mallela, et al., Evaluation of developmental toxicity of propylthiouracil and methimazole. Birth Defects Res. Part B - Dev. Reprod. Toxicol. (2014) doi.org/10.1002/bdrb.21113.
19. M. Kotlyar, C. Pastrello, Z. Malik, I. Jurisica, IID 2018 update: context-specific physical protein-protein interactions in human, model organisms and domesticated species, Nucleic Acids Res. 47, D581-D589 (2019).
20. G. Bagnato, et al., Propylthiouracil prevents cutaneous and pulmonary fibrosis in the reactive oxygen species murine model of systemic sclerosis. Arthritis Res. Ther. (2013) doi.org/10.1186/ar4300.
21. A. I. Casas, P. W. M. Kleikers, E. Geuss, F. Langhauser, T. Adler, D. H. Busch, V. Gailus-Durner, M. H. de Angelis, J. Egea, M. G. Lopez, C. Kleinschnitz, H. H. H. W. Schmidt, Calcium-dependent blood-brain barrier breakdown by NOX5 limits postreperfusion benefit in stroke, J. Clin. Invest. 129, 1772-1778 (2019).
22. C. E. Holterman, J.-F. Thibodeau, C. Towaij, A. Gutsol, A. C. Montezano, R. J. Parks, M. E. Cooper, R. M. Touyz, C. R. J. Kennedy, Nephropathy and Elevated BP in Mice with Podocyte-Specific NADPH Oxidase 5 Expression, J. Am. Soc. Nephrol. 25, 784-797 (2014).
23. J. B. Bederson, L. H. Pitts, S. M. Germano, M. C. Nishimura, R. L. Davis, H. M. Bartkowski, Evaluation of 2,3,5-triphenyltetrazolium chloride as a stain for detection and quantification of experimental cerebral infarction in rats., Stroke 17, 1304-1308 (1986).
24. F. Denizot, R. Lang, Rapid colorimetric assay for cell growth and survival, J. Immunol. Methods 89, 271-277 (1986).

## Claims

1. Therapeutic combination comprising:
- a first therapeutic composition comprising at least one of: at least one NADPH oxidase inhibitor, at least one nitric oxide synthase inhibitor and at least one soluble guanylate cyclase agonist;
- a second therapeutic composition comprising at least one of: at least one NADPH oxidase inhibitor, at least one nitric oxide synthase inhibitor and at least one soluble guanylate cyclase agonist, wherein the second therapeutic composition is different from the first therapeutic composition;
wherein, the NADPH oxidase inhibitor is selected from setanaxib, GKT136901, GKT137831, GLX7013114, VAS2870, perphenazine, fluphenazine, perazine and thioridazine and wherein the nitric oxide synthase inhibitor is selected from NG-nitro-L-arginine methyl ester, L-NAME, NG-monomethyl-L-arginine, 2-iminobiotin, ronopterin, S-methyl-1-thiocitrulline and propylthiouracil, for use as a medicament.

2. Therapeutic combination for use according to claim 1, comprising a third therapeutic composition comprising at least one of: at least one NADPH oxidase inhibitor, at least one nitric oxide synthase inhibitor and at least one soluble guanylate cyclase agonist, wherein the third therapeutic composition is different from the first therapeutic composition and the second therapeutic composition, wherein, the NADPH oxidase inhibitor is selected from setanaxib, GKT136901, GKT137831, GLX7013114, VAS2870, perphenazine, fluphenazine, perazine and thioridazine and wherein the nitric oxide synthase inhibitor is selected from NG-nitro-L-arginine methyl ester, L-NAME, NG-monomethyl-L-arginine, 2-iminobiotin, ronopterin, S-methyl-1-thiocitrulline and propylthiouracil.

3. Therapeutic combination for use according to claim 1 or 2, wherein the NADPH oxidase inhibitor is selected from GKT137831, GKT136901 and perphenazine, wherein the nitric oxide synthase inhibitor is selected from L-NAME, S-methyl-1-thiocitrulline and propylthiouracil, and/or wherein the soluble guanylate cyclase agonist is a soluble guanylate cyclase activator or a soluble guanylate cyclase stimulator, preferably a soluble guanylate cyclase agonist selected from cinaciguat, BAY60-2770, BAY41-2272, ataciguat, BI 703704, BI 684067, S-3448, BR-11257, MGV-354, TY-55002, riociguat, vericiguat, nelociguat, olinciguat, BAY41-2772, BAY60-4552, BAY63-2521, IWP-953, A-350619, CF-1571, CFM-1571, lificiguat, etriciguat and praliciguat, more preferably a soluble guanylate cyclase agonist selected from cinaciguat, BAY60-2770 and riociguat.

4. Therapeutic combination for use according to claim 2 or 3, wherein the NADPH oxidase inhibitor is GKT137831 or GKT136901 or perphenazine, and wherein the nitric oxide synthase inhibitor is L-NAME or propylthiouracil; preferably, the NADPH oxidase inhibitor is GKT136901 and the nitric oxide synthase inhibitor is L-NAME.

5. Therapeutic combination for use according to any one of claims 1-4, wherein:
- the NADPH oxidase inhibitor is perphenazine, and the nitric oxide synthase inhibitor is propylthiouracil; - the NADPH oxidase inhibitor is GKT136901, and the soluble guanylate cyclase agonist is BAY60-2770; - the nitric oxide synthase inhibitor is L-NAME or propylthiouracil, and the soluble guanylate cyclase agonist is BAY60-2770 or riociguat; - the nitric oxide synthase inhibitor is L-NAME and the soluble guanylate cyclase agonist is BAY60-2770; or- the nitric oxide synthase inhibitor is propylthiouracil, and the soluble guanylate cyclase agonist is riociguat.

6. Therapeutic combination for use according to any one of claims 1-5, for use in the prevention or treatment of:
- brain ischemia;- cerebral infarction; - ischemic stroke; or
- ischemia-reperfusion injury.

7. Therapeutic combination for use according to any one of claims 1-6, wherein at least one, preferably at least two, more preferably all of the first, second and, when present, third therapeutic compositions is administered orally or parentally.

8. Therapeutic combination for use according to any one of claims 1-7, wherein the therapeutic combination is administered to a patient suffering from an obstructed blood vessel in an ischemic brain region, and wherein the therapeutic combination is administered to said patient prior to initiation of recanalization of the obstructed blood vessel in the ischemic brain region or at the start of the initiation of recanalization of the obstructed blood vessel in the ischemic brain region.

9. Kit comprising the therapeutic combination according to any one of claims 1-8 for use as a medicament.

10. Pharmaceutical composition comprising two or three of: at least one NADPH oxidase inhibitor, at least one nitric oxide synthase inhibitor and at least one soluble guanylate cyclase agonist or a pharmaceutically acceptable salt thereof, wherein, the NADPH oxidase inhibitor is selected from any one or more of NADPH oxidase inhibitors setanaxib, GKT136901, GKT137831, GLX7013114, VAS2870, perphenazine, fluphenazine, perazine and thioridazine and wherein the nitric oxide synthase inhibitor is selected from NG-nitro-L-arginine methyl ester, L-NAME, NG-monomethyl-L-arginine, 2-iminobiotin, ronopterin, S-methyl-1-thiocitrulline and propylthiouracil, wherein the pharmaceutical composition preferably comprises the combination of at least one NADPH oxidase inhibitor, at least one nitric oxide synthase inhibitor and at least one soluble guanylate cyclase agonist, for use as a medicament.

11. Pharmaceutical composition for use according to claim 10 for use in the prevention or treatment of brain ischemia, cerebral infarction, ischemic stroke and/or of ischemia-reperfusion injury.

12. Pharmaceutical composition comprising at least one soluble guanylate cyclase agonist, for use in the prevention or treatment of brain ischemia cerebral infarction, ischemic stroke and/or of ischemia-reperfusion injury, wherein the method further comprises the administration of at least one NADPH oxidase inhibitor and/or at least one nitric oxide synthase inhibitor, wherein, the NADPH oxidase inhibitor is selected from setanaxib, GKT136901, GKT137831, GLX7013114, VAS2870, perphenazine, fluphenazine, perazine and thioridazine, and wherein the nitric oxide synthase inhibitor is selected from NG-nitro-L-arginine methyl ester, L-NAME, NG-monomethyl-L-arginine, 2-iminobiotin, ronopterin, S-methyl-1-thiocitrulline and propylthiouracil.

13. Pharmaceutical composition comprising at least one NADPH oxidase inhibitor, for use in the prevention or treatment of brain ischemia, cerebral infarction, ischemic stroke and/or of ischemia-reperfusion injury, wherein the method further comprises the administration of at least one nitric oxide synthase inhibitor and/or at least one soluble guanylate cyclase agonist, wherein, the NADPH oxidase inhibitor is selected from setanaxib, GKT136901, GKT137831, GLX7013114, VAS2870, perphenazine, fluphenazine, perazine and thioridazine, and wherein the nitric oxide synthase inhibitor is selected from NG-nitro-L-arginine methyl ester, L-NAME, NG-monomethyl-L-arginine, 2-iminobiotin, ronopterin, S-methyl-1-thiocitrulline and propylthiouracil.

14. Pharmaceutical composition comprising at least one nitric oxide synthase inhibitor, for use in the prevention or treatment of brain ischemia, cerebral infarction, ischemic stroke and/or of ischemia-reperfusion injury, wherein the method further comprises the administration of at least one NADPH oxidase inhibitor and/or at least one soluble guanylate cyclase agonist, wherein, the NADPH oxidase inhibitor is selected from setanaxib, GKT136901, GKT137831, GLX7013114, VAS2870, perphenazine, fluphenazine, perazine and thioridazine, and wherein the nitric oxide synthase inhibitor is selected from NG-nitro-L-arginine methyl ester, L-NAME, NG-monomethyl-L-arginine, 2-iminobiotin, ronopterin, S-methyl-1-thiocitrulline and propylthiouracil.

15. Pharmaceutical composition for use according to claim 12, 13 or 14, wherein the method comprises the administration of at least one soluble guanylate cyclase agonist, at least one NADPH oxidase inhibitor and at least one nitric oxide synthase inhibitor.

## Patentansprüche

1. Therapeutische Kombination, umfassend:
- eine erste therapeutische Zusammensetzung, umfassend mindestens eines von: mindestens einem NADPH-Oxidase-Inhibitor, mindestens einem Stickstoffmonoxid-Synthase-Inhibitor und mindestens einem löslichen Guanylatcyclase-Agonisten;
- eine zweite therapeutische Zusammensetzung, umfassend mindestens eines von: mindestens einem NADPH-Oxidase-Inhibitor, mindestens einem Stickstoffmonoxid-Synthase-Inhibitor und mindestens einem löslichen Guanylatcyclase-Agonisten, wobei die zweite therapeutische Zusammensetzung verschieden von der ersten therapeutischen Zusammensetzung ist;
wobei der NADPH-Oxidase-Inhibitor ausgewählt ist aus Setanaxib, GKT136901, GKT137831, GLX7013114, VAS2870, Perphenazin, Fluphenazin, Perazin und Thioridazin und wobei der Stickstoffmonoxid-Synthase-Inhibitor ausgewählt ist aus NG-Nitro-L-arginin-methylester, L-NAME, NG-Monomethyl-L-arginin, 2-Iminobiotin, Ronopterin, S-Methyl-1-thiocitrullin und Propylthiouracil, zur Verwendung als Medikament.

2. Therapeutische Kombination zur Verwendung nach Anspruch 1, umfassend eine dritte therapeutische Zusammensetzung, umfassend mindestens eines von: mindestens einem NADPH-Oxidase-Inhibitor, mindestens einem Stickstoffmonoxid-Synthase-Inhibitor und mindestens einem löslichen Guanylatcyclase-Agonisten, wobei die dritte therapeutische Zusammensetzung verschieden von der ersten therapeutischen Zusammensetzung und der zweiten therapeutischen Zusammensetzung ist, wobei der NADPH-Oxidase-Inhibitor ausgewählt ist aus Setanaxib, GKT136901, GKT137831, GLX7013114, VAS2870, Perphenazin, Fluphenazin, Perazin und Thioridazin und wobei der Stickstoffmonoxid-Synthase-Inhibitor ausgewählt ist aus NG-Nitro-L-arginin-methylester, L-NAME, NG-Monomethyl-L-arginin, 2-Iminobiotin, Ronopterin, S-Methyl-1-thiocitrullin und Propylthiouracil.

3. Therapeutische Kombination zur Verwendung nach Anspruch 1 oder 2, wobei der NADPH-Oxidase-Inhibitor ausgewählt ist aus GKT137831, GKT136901 und Perphenazin, wobei der Stickstoffmonoxid-Synthase-Inhibitor ausgewählt ist aus L-NAME, S-Methyl-1-thiocitrullin und Propylthiouracil, und/oder wobei der lösliche Guanylatcyclase-Agonist ein löslicher Guanylatcyclase-Aktivator oder ein löslicher Guanylatcyclase-Stimulator ist, vorzugsweise ein löslicher Guanylatcyclase-Agonist, ausgewählt aus Cinaciguat, BAY60-2770, BAY41-2272, Ataciguat, BI 703704, BI 684067, S-3448, BR-11257, MGV-354, TY-55002, Riociguat, Vericiguat, Nelociguat, Olinciguat, BAY41-2772, BAY60-4552, BAY63-2521, IWP-953, A-350619, CF-1571, CFM-1571, Lificiguat, Etriciguat und Praliciguat, mehr vorzugsweise ein löslicher Guanylatcyclase-Agonist, ausgewählt aus Cinaciguat, BAY60-2770 und Riociguat.

4. Therapeutische Kombination zur Verwendung nach Anspruch 2 oder 3, wobei der NADPH-Oxidase-Inhibitor GKT137831 oder GKT136901 oder Perphenazin ist, und wobei der Stickstoffmonoxid-Synthase-Inhibitor L-NAME oder Propylthiouracil ist; vorzugsweise ist der NADPH-Oxidase-Inhibitor GKT136901 und der Stickstoffmonoxid-Synthase-Inhibitor ist L-NAME.

5. Therapeutische Kombination zur Verwendung nach einem der Ansprüche 1 bis 4, wobei:
- der NADPH-Oxidase-Inhibitor Perphenazin ist, und der Stickstoffmonoxid-Synthase-Inhibitor Propylthiouracil ist; - der NADPH-Oxidase-Inhibitor GKT136901 ist, und der lösliche Guanylatcyclase-Agonist BAY60-2770 ist; - der Stickstoffmonoxid-Synthase-Inhibitor L-NAME oder Propylthiouracil ist, und der lösliche Guanylatcyclase-Agonist BAY60-2770 oder Riociguat ist; - der Stickstoffmonoxid-Synthase-Inhibitor L-NAME ist und der lösliche Guanylatcyclase-Agonist BAY60-2770 ist; oder - der Stickstoffmonoxid-Synthase-Inhibitor Propylthiouracil ist, und der lösliche Guanylatcyclase-Agonist Riociguat ist.

6. Therapeutische Kombination zur Verwendung nach einem der Ansprüche 1 bis 5, zur Verwendung in der Prävention oder Behandlung von:
- Gehirnischämie; - Hirninfarkt; - ischämischem Schlaganfall; oder
- Ischämie-Reperfusionsschädigung.

7. Therapeutische Kombination zur Verwendung nach einem der Ansprüche 1 bis 6, wobei mindestens eine, vorzugsweise mindestens zwei, mehr vorzugsweise alle der ersten, zweiten und, wenn vorhanden, dritten therapeutischen Zusammensetzungen oral oder parenteral verabreicht wird.

8. Therapeutische Kombination zur Verwendung nach einem der Ansprüche 1 bis 7, wobei die therapeutische Kombination an einen Patienten, der an einem verschlossenen Blutgefäß in einer ischämischen Gehirnregion leidet, verabreicht wird, und wobei die therapeutische Kombination an den Patienten vor Einleitung der Rekanalisierung des verschlossenen Blutgefäßes in der ischämischen Gehirnregion oder zu Beginn der Einleitung der Rekanalisierung des verschlossenen Blutgefäßes in der ischämischen Gehirnregion verabreicht wird.

9. Kit, umfassend die therapeutische Kombination nach einem der Ansprüche 1 bis 8, zur Verwendung als Medikament.

10. Pharmazeutische Zusammensetzung, umfassend zwei oder drei von: mindestens einem NADPH-Oxidase-Inhibitor, mindestens einem Stickstoffmonoxid-Synthase-Inhibitor und mindestens einem löslichen Guanylatcyclase-Agonisten oder einem pharmazeutisch verträglichen Salz davon, wobei der NADPH-Oxidase-Inhibitor ausgewählt ist aus einem oder mehreren der NADPH-Oxidase-Inhibitoren Setanaxib, GKT136901, GKT137831, GLX7013114, VAS2870, Perphenazin, Fluphenazin, Perazin und Thioridazin und wobei der Stickstoffmonoxid-Synthase-Inhibitor ausgewählt ist aus NG-Nitro-L-arginin-methylester, L-NAME, NG-Monomethyl-L-arginin, 2-Iminobiotin, Ronopterin, S-Methyl-1-thiocitrullin und Propylthiouracil, wobei die pharmazeutische Zusammensetzung vorzugsweise die Kombination von mindestens einem NADPH-Oxidase-Inhibitor, mindestens einem Stickstoffmonoxid-Synthase-Inhibitor und mindestens einem löslichen Guanylatcyclase-Agonisten umfasst, zur Verwendung als Medikament.

11. Pharmazeutische Zusammensetzung zur Verwendung nach Anspruch 10 zur Verwendung in der Prävention oder Behandlung von Gehirnischämie, Hirninfarkt, ischämischem Schlaganfall und/oder von Ischämie-Reperfusionsschädigung.

12. Pharmazeutische Zusammensetzung, umfassend mindestens einen löslichen Guanylatcyclase-Agonisten, zur Verwendung in der Prävention oder Behandlung von Gehirnischämie, Hirninfarkt, ischämischem Schlaganfall und/oder von Ischämie-Reperfusionsschädigung, wobei das Verfahren ferner die Verabreichung von mindestens einem NADPH-Oxidase-Inhibitor und/oder mindestens einem Stickstoffmonoxid-Synthase-Inhibitor umfasst, wobei der NADPH-Oxidase-Inhibitor ausgewählt ist aus Setanaxib, GKT136901, GKT137831, GLX7013114, VAS2870, Perphenazin, Fluphenazin, Perazin und Thioridazin, und wobei der Stickstoffmonoxid-Synthase-Inhibitor ausgewählt ist aus NG-Nitro-L-arginin-methylester, L-NAME, NG-Monomethyl-L-arginin, 2-Iminobiotin, Ronopterin, S-Methyl-1-thiocitrullin und Propylthiouracil.

13. Pharmazeutische Zusammensetzung, umfassend mindestens einen NADPH-Oxidase-Inhibitor, zur Verwendung in der Prävention oder Behandlung von Gehirnischämie, Hirninfarkt, ischämischem Schlaganfall und/oder von Ischämie-Reperfusionsschädigung, wobei das Verfahren ferner die Verabreichung von mindestens einem Stickstoffmonoxid-Synthase-Inhibitor und/oder mindestens einem löslichen Guanylatcyclase-Agonisten umfasst, wobei der NADPH-Oxidase-Inhibitor ausgewählt ist aus Setanaxib, GKT136901, GKT137831, GLX7013114, VAS2870, Perphenazin, Fluphenazin, Perazin und Thioridazin, und wobei der Stickstoffmonoxid-Synthase-Inhibitor ausgewählt ist aus NG-Nitro-L-arginin-methylester, L-NAME, NG-Monomethyl-L-arginin, 2-Iminobiotin, Ronopterin, S-Methyl-1-thiocitrullin und Propylthiouracil.

14. Pharmazeutische Zusammensetzung, umfassend mindestens einen Stickstoffmonoxid-Synthase-Inhibitor, zur Verwendung in der Prävention oder Behandlung von Gehirnischämie, Hirninfarkt, ischämischem Schlaganfall und/oder von Ischämie-Reperfusionsschädigung, wobei das Verfahren ferner die Verabreichung von mindestens einem NADPH-Oxidase-Inhibitor und/oder mindestens einem löslichen Guanylatcyclase-Agonisten umfasst, wobei der NADPH-Oxidase-Inhibitor ausgewählt ist aus Setanaxib, GKT136901, GKT137831, GLX7013114, VAS2870, Perphenazin, Fluphenazin, Perazin und Thioridazin, und wobei der Stickstoffmonoxid-Synthase-Inhibitor ausgewählt ist aus NG-Nitro-L-arginin-methylester, L-NAME, NG-Monomethyl-L-arginin, 2-Iminobiotin, Ronopterin, S-Methyl-1-thiocitrullin und Propylthiouracil.

15. Pharmazeutische Zusammensetzung zur Verwendung nach Anspruch 12, 13 oder 14, wobei das Verfahren die Verabreichung von mindestens einem löslichen Guanylatcyclase-Agonisten, mindestens einem NADPH-Oxidase-Inhibitor und mindestens einem Stickstoffmonoxid-Synthase-Inhibitor umfasst.

## Revendications

1. Combinaison thérapeutique comprenant :
- une première composition thérapeutique comprenant au moins un élément parmi : au moins un inhibiteur de la NADPH oxydase, au moins un inhibiteur de la synthase d'oxyde nitrique et au moins un agoniste de la guanylate cyclase soluble ;
- une deuxième composition thérapeutique comprenant au moins un élément parmi : au moins un inhibiteur de la NADPH oxydase, au moins un inhibiteur de la synthase d'oxyde nitrique et au moins un agoniste de la guanylate cyclase soluble, dans laquelle la deuxième composition thérapeutique est différente de la première composition thérapeutique ;
dans laquelle l'inhibiteur de la NADPH oxydase est choisi parmi le sétanaxib, le GKT136901, le GKT137831, le GLX7013114, le VAS2870, la perphénazine, la fluphénazine, la pérazine et la thioridazine et dans laquelle l'inhibiteur de la synthase d'oxyde nitrique est choisi parmi l'ester méthylique de NG-nitro-L-arginine, le L-NAME, la NG-monométhyl-L-arginine, la 2-iminobiotine, la ronoptérine, la S-méthyl-1-thiocitrulline et le propylthiouracile, pour utilisation en tant que médicament.

2. Combinaison thérapeutique pour utilisation selon la revendication 1, comprenant une troisième composition thérapeutique comprenant au moins un élément parmi : au moins un inhibiteur de la NADPH oxydase, au moins un inhibiteur de la synthase d'oxyde nitrique et au moins un agoniste de la guanylate cyclase soluble, dans laquelle la troisième composition thérapeutique est différente de la première composition thérapeutique et de la deuxième composition thérapeutique, dans laquelle l'inhibiteur de la NADPH oxydase est choisi parmi le sétanaxib, le GKT136901, le GKT137831, le GLX7013114, le VAS2870, la perphénazine, la fluphénazine, la pérazine et la thioridazine et dans laquelle l'inhibiteur de la synthase d'oxyde nitrique est choisi parmi l'ester méthylique de NG-nitro-L-arginine, le L-NAME, la NG-monométhyl-L-arginine, la 2-iminobiotine, la ronoptérine, la S-méthyl-1-thiocitrulline et le propylthiouracile.

3. Combinaison thérapeutique pour utilisation selon la revendication 1 ou 2, dans laquelle l'inhibiteur de la NADPH oxydase est choisi parmi le GKT137831, le GKT136901 et la perphénazine, dans laquelle l'inhibiteur de la synthase d'oxyde nitrique est choisi parmi le L-NAME, la S-méthyl-1-thiocitrulline et le propylthiouracile, et/ou dans laquelle l'agoniste de la guanylate cyclase soluble est un activateur de la guanylate cyclase soluble ou un stimulateur de la guanylate cyclase soluble, de préférence un agoniste de la guanylate cyclase soluble choisi parmi le cinaciguat, le BAY60-2770, le BAY41-2272, l'ataciguat, le BI 703704, le BI 684067, le S-3448, le BR-11257, le MGV-354, le TY-55002, le riociguat, le vericiguat, le nélociguat, l'olinciguat, le BAY41-2772, le BAY60-4552, le BAY63-2521, l'IWP-953, l'A-350619, le CF-1571, le CFM-1571, le lificiguat, l'etriciguat et le praliciguat, plus préférablement un agoniste de la guanylate cyclase soluble choisi parmi le cinaciguat, le BAY60-2770 et le riociguat.

4. Combinaison thérapeutique pour utilisation selon la revendication 2 ou 3, dans laquelle l'inhibiteur de la NADPH oxydase est le GKT137831 ou le GKT136901 ou la perphénazine, et dans laquelle l'inhibiteur de la synthase d'oxyde nitrique est le L-NAME ou le propylthiouracile ; de préférence, l'inhibiteur de la NADPH oxydase est le GKT136901 et l'inhibiteur de la synthase d'oxyde nitrique est le L-NAME.

5. Combinaison thérapeutique pour utilisation selon l'une quelconque des revendications 1 à 4, dans laquelle :
- l'inhibiteur de la NADPH oxydase est la perphénazine, et l'inhibiteur de la synthase d'oxyde nitrique est le propylthiouracile ; - l'inhibiteur de la NADPH oxydase est le GKT136901, et l'agoniste de la guanylate cyclase soluble est le BAY60-2770 ; - l'inhibiteur de la synthase d'oxyde nitrique est le L-NAME ou le propylthiouracile, et l'agoniste de la guanylate cyclase soluble est le BAY60-2770 ou le riociguat ; - l'inhibiteur de la synthase d'oxyde nitrique est le L-NAME et l'agoniste de la guanylate cyclase soluble est le BAY60-2770 ; ou - l'inhibiteur de la synthase d'oxyde nitrique est le propylthiouracile, et l'agoniste de la guanylate cyclase soluble est le riociguat.

6. Combinaison thérapeutique pour utilisation selon l'une quelconque des revendications 1 à 5, pour utilisation dans la prévention ou le traitement de :
- l'ischémie cérébrale ; - l'infarctus cérébral ; - l'accident vasculaire cérébral ischémique ; ou
- la lésion d'ischémie-reperfusion.

7. Combinaison thérapeutique pour utilisation selon l'une quelconque des revendications 1 à 6, dans laquelle au moins une, de préférence au moins deux, plus préférablement toutes les première, deuxième et, lorsqu'elle est présente, troisième compositions thérapeutiques sont administrées par voie orale ou par voie parentérale.

8. Combinaison thérapeutique pour utilisation selon l'une quelconque des revendications 1 à 7, dans laquelle la combinaison thérapeutique est administrée à un patient qui souffre d'un vaisseau sanguin obstrué dans une région cérébrale ischémique, et dans laquelle la combinaison thérapeutique est administrée audit patient avant l'initiation de la recanalisation du vaisseau sanguin obstrué dans la région cérébrale ischémique ou au début de l'initiation de la recanalisation du vaisseau sanguin obstrué dans la région cérébrale ischémique.

9. Kit comprenant la combinaison thérapeutique selon l'une quelconque des revendications 1 à 8 pour utilisation en tant que médicament.

10. Composition pharmaceutique comprenant deux ou trois éléments parmi : au moins un inhibiteur de la NADPH oxydase, au moins un inhibiteur de la synthase d'oxyde nitrique et au moins un agoniste de la guanylate cyclase soluble ou un sel pharmaceutiquement acceptable de celui-ci, dans laquelle l'inhibiteur de la NADPH oxydase est choisi parmi un ou plusieurs quelconques des inhibiteurs de la NADPH oxydase sétanaxib, GKT136901, GKT137831, GLX7013114, VAS2870, perphénazine, fluphénazine, pérazine et thioridazine et dans laquelle l'inhibiteur de la synthase d'oxyde nitrique est choisi parmi l'ester méthylique de NG-nitro-L-arginine, le L-NAME, la NG-monométhyl-L-arginine, la 2-iminobiotine, la ronoptérine, la S-méthyl-1-thiocitrulline et le propylthiouracile, dans laquelle la composition pharmaceutique comprend de préférence la combinaison d'au moins un inhibiteur de la NADPH oxydase, d'au moins un inhibiteur de la synthase d'oxyde nitrique et d'au moins un agoniste de la guanylate cyclase soluble, pour utilisation en tant que médicament.

11. Composition pharmaceutique pour utilisation selon la revendication 10 pour utilisation dans la prévention ou le traitement de l'ischémie cérébrale, de l'infarctus cérébral, de l'accident vasculaire cérébral ischémique et/ou de la lésion d'ischémie-reperfusion.

12. Composition pharmaceutique comprenant au moins un agoniste de la guanylate cyclase soluble, pour utilisation dans la prévention ou le traitement de l'ischémie cérébrale, de l'infarctus cérébral, de l'accident vasculaire cérébral ischémique et/ou de la lésion d'ischémie-reperfusion, dans laquelle le procédé comprend en outre l'administration d'au moins un inhibiteur de la NADPH oxydase et/ou d'au moins un inhibiteur de la synthase d'oxyde nitrique, dans laquelle l'inhibiteur de la NADPH oxydase est choisi parmi le sétanaxib, le GKT136901, le GKT137831, le GLX7013114, le VAS2870, la perphénazine, la fluphénazine, la pérazine et la thioridazine, et dans laquelle l'inhibiteur de la synthase d'oxyde nitrique est choisi parmi l'ester méthylique de NG-nitro-L-arginine, le L-NAME, la NG-monométhyl-L-arginine, la 2-iminobiotine, la ronoptérine, la S-méthyl-1-thiocitrulline et le propylthiouracile.

13. Composition pharmaceutique comprenant au moins un inhibiteur de la NADPH oxydase, pour utilisation dans la prévention ou le traitement de l'ischémie cérébrale, de l'infarctus cérébral, de l'accident vasculaire cérébral ischémique et/ou de la lésion d'ischémie-reperfusion, dans laquelle le procédé comprend en outre l'administration d'au moins un inhibiteur de la synthase d'oxyde nitrique et/ou d'au moins un agoniste de la guanylate cyclase soluble, dans laquelle l'inhibiteur de la NADPH oxydase est choisi parmi le sétanaxib, le GKT136901, le GKT137831, le GLX7013114, le VAS2870, la perphénazine, la fluphénazine, la pérazine et la thioridazine, et dans laquelle l'inhibiteur de la synthase d'oxyde nitrique est choisi parmi l'ester méthylique de NG-nitro-L-arginine, le L-NAME, la NG-monométhyl-L-arginine, la 2-iminobiotine, la ronoptérine, la S-méthyl-1-thiocitrulline et le propylthiouracile.

14. Composition pharmaceutique comprenant au moins un inhibiteur de la synthase d'oxyde nitrique, pour utilisation dans la prévention ou le traitement de l'ischémie cérébrale, de l'infarctus cérébral, de l'accident vasculaire cérébral ischémique et/ou de la lésion d'ischémie-reperfusion, dans laquelle le procédé comprend en outre l'administration d'au moins un inhibiteur de la NADPH oxydase et/ou d'au moins un agoniste de la guanylate cyclase soluble, dans laquelle l'inhibiteur de la NADPH oxydase est choisi parmi le sétanaxib, le GKT136901, le GKT137831, le GLX7013114, le VAS2870, la perphénazine, la fluphénazine, la pérazine et la thioridazine, et dans laquelle l'inhibiteur de la synthase d'oxyde nitrique est choisi parmi l'ester méthylique de NG-nitro-L-arginine, le L-NAME, la NG-monométhyl-L-arginine, la 2-iminobiotine, la ronoptérine, la S-méthyl-1-thiocitrulline et le propylthiouracile.

15. Composition pharmaceutique pour utilisation selon la revendication 12, 13 ou 14, dans laquelle le procédé comprend l'administration d'au moins un agoniste de la guanylate cyclase soluble, d'au moins un inhibiteur de la NADPH oxydase et d'au moins un inhibiteur de la synthase d'oxyde nitrique.
